# EUROPÄISCHE PATENTSCHRIFT

(11) **EP 1 435 205 B1**
(45) Veröffentlichungstag und Bekanntmachung des Hinweises auf die Patenterteilung: **11.11.2009**
(21) Anmeldenummer: 04002725.2
(22) Anmeldetag: 19.09.1996
(51) Int. Cl.: A23L 1/0522, C12N 15/82

(54) **Verfahren zur Herstellung einer modifizierten Stärke**
Process for the production of a modified starch
Procédé de production d'amidon modifié

(30) Priorität: 19.09.1995 DE 19534759; 20.12.1995 DE 19547733
(43) Veröffentlichungstag der Anmeldung: 07.07.2004
(62) Teilanmeldung aus: 96932575.2
(73) Patentinhaber: Bayer BioScience GmbH, 14473 Potsdam (DE)
(72) Erfinder: Kossmann, Jens, Somerset West 7130 (ZA); Lorberth, Ruth, Brookline, MA 02467 (US)
(74) Vertreter: Vossius & Partner

(56) Entgegenhaltungen:
- WO-A-92/11375
- WO-A-94/09144
- WO-A-95/07355
- WO-A-95/26407

### SEQUENZPROTOKOLL

(1) ALLGEMEINE ANGABEN:
   (i) ANMELDER:
      (A) NAME: Jens Koßmann
      (B) STRASSE: Golmer Fichten 9.
      (C) ORT: Golm
      (E) LAND: DE
      (F) POSTLEITZAHL: 14476
   (ii) BEZEICHNUNG DER ERFINDUNG: Pflanzen, die eine modifizierte Staerke synthetisieren, Verfahren zu ihrer Herstellung, sowie modifizierte Stärke
   (iii) ANZAHL DER SEQUENZEN: 4
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 4856 Basenpaare
      (B) ART: Nucleotid
      (C) STRANGFORM: Einzelstrang
      (D) TOPOLOGIE: linear
   (ii) ART DES MOLEKÜLS: cDNA zu mRNA
   (vi) URSPRÜNLICHE HERKUNFT:
      (A) ORGANISMUS: Solanum tuberosum
      (B) STAMM: C.V. Berolina
   (ix) MERKMAL:
      (A) NAME/SCHLÜSSEL: CDS
      (B) LAGE:105..4497
   (xi) SEQUENZBESCHREIBUNG: SEQ ID NO: 1:
   CATCTTCATC GAATTTCTCG AAGCTTCTTC GCTAATTTCC TGGTTTCTTC ACTCAAAATC       60
   GACGTTTCTA GCTGAACTTG AGTGAATTAA GCCAGTGGGA GGAT ATG AGT AAT TCC      116
                                                    Met Ser Asn Ser
                                                     1
   TTA GGG AAT AAC TTG CTG TAC CAG GGA TTC CTA ACC TCA ACA GTG TTG      164
   Leu Gly Asn Asn Leu Leu Tyr Gln Gly Phe Leu Thr Ser Thr Val Leu
     5                  10                  15                  20
   GAA CAT AAA AGT AGA ATC AGT CCT CCT TGT GTT GGA GGC AAT TCT TTG      212
   Glu His Lys Ser Arg Ile Ser Pro Pro Cys Val Gly Gly Asn Ser Leu
                    25                  30                  35
   TTT CAA CAA CAA GTG ATC TCG AAA TCA CCT TTA TCA ACT GAG TTT CGA      260
   Phe Gln Gln Gln Val Ile Ser Lys Ser Pro Leu Ser Thr Glu Phe Arg
               40                  45                  50
   GGT AAC AGG TTA AAG GTG CAG AAA AAG AAA ATA CCT ATG GAA AAG AAG      308
   Gly Asn Arg Leu Lys Val Gln Lys Lys Lys Ile Pro Met Glu Lys Lys
           55                  60                  65
   CGT GCT TTT TCT AGT TCT CCT CAT GCT GTA CTT ACC ACT GAT ACC TCT      356
   Arg Ala Phe Ser Ser Ser Pro His Ala Val Leu Thr Thr Asp Thr Ser
       70                  75                  80
   TCT GAG CTA GCA GAA AAG TTC AGT CTA GGG GGG AAT ATT GAG CTA CAG      404
   Ser Glu Leu Ala Glu Lys Phe Ser Leu Gly Gly Asn Ile Glu Leu Gln
   85                  90                  95                  100
   GTT GAT GTT AGG CCT CCC ACT TCA GGT GAT GTG TCC TTT GTG GAT TTT      452
   Val Asp Val Arg Pro Pro Thr Ser Gly Asp Val Ser Phe Val Asp Phe
                   105                 110                 115
   CAA GTA ACA AAT GGT AGT GAT AAA CTG TTT TTG CAC TGG GGG GCA GTA      500
   Gln Val Thr Asn Gly Ser Asp Lys Leu Phe Leu His Trp Gly Ala Val
               120                 125                 130
   AAA TTC GGG AAA GAA ACA TGG TCT CTT CCG AAT GAT CGT CCA GAT GGG      548
   Lys Phe Gly Lys Glu Thr Trp Ser Leu Pro Asn Asp Arg Pro Asp Gly
           135                 140                 145
   ACC AAA GTG TAC AAG AAC AAA GCA CTT AGA ACT CCA TTT GTT AAA TCT      596
   Thr Lys Val Tyr Lys Asn Lys Ala Leu Arg Thr Pro Phe Val Lys Ser
       150                 155                 160
   GGC TCT AAC TCC ATC CTG AGA CTG GAG ATA CGA GAC ACT GCT ATC GAA      644
   Gly Ser Asn Ser Ile Leu Arg Leu Glu Ile Arg Asp Thr Ala Ile Glu
   165                 170                 175                 180
   GCT ATT GAG TTT CTC ATA TAC GAT GAA GCC CAC GAT AAA TGG ATA AAG      692
   Ala Ile Glu Phe Leu Ile Tyr Asp Glu Ala His Asp Lys Trp Ile Lys
                   185                 190                 195
   AAT AAT GGT GGT AAT TTT CGT GTC AAA TTG TCA AGA AAA GAG ATA CGA      740
   Asn Asn Gly Gly Asn Phe Arg Val Lys Leu Ser Arg Lys Glu Ile Arg
               200                 205                 210
   GGC CCA GAT GTT TCT GTT CCT GAG GAG CTT GTA CAG ATC CAA TCA TAT      788
   Gly Pro Asp Val Ser Val Pro Glu Glu Leu Val Gln Ile Gln Ser Tyr
           215                 220                 225
   TTG AGG TGG GAG AGG AAG GGA AAA CAG AAT TAC CCC CCT GAG AAA GAG      836
   Leu Arg Trp Glu Arg Lys Gly Lys Gln Asn Tyr Pro Pro Glu Lys Glu
       230                 235                 240
   AAG GAG GAA TAT GAG GCT GCT CGA ACT GTG CTA CAG GAG GAA ATA GCT      884
   Lys Glu Glu Tyr Glu Ala Ala Arg Thr Val Leu Gln Glu Glu Ile Ala
   245                 250                 255                 260
   CGT GGT GCT TCC ATA CAG GAC ATT CGA GCA AGG CTA ACA AAA ACT AAT      932
   Arg Gly Ala Ser Ile Gln Asp Ile Arg Ala Arg Leu Thr Lys Thr Asn
                   265                 270                 275
   GAT AAA AGT CAA AGC AAA GAA GAG CCT CTT CAT GTA ACA AAG AGT GAT      980
   Asp Lys Ser Gln Ser Lys Glu Glu Pro Leu His Val Thr Lys Ser Asp
               280                 285                 290
   ATA CCT GAT GAC CTT GCC CAA GCA CAA GCT TAC ATT AGG TGG GAG AAA     1028
   Ile Pro Asp Asp Leu Ala Gln Ala Gln Ala Tyr Ile Arg Trp Glu Lys
           295                 300                 305
   GCA GGA AAG CCG AAC TAT CCT CCA GAA AAG CAA ATT GAA GAA CTC GAA     1076
   Ala Gly Lys Pro Asn Tyr Pro Pro Glu Lys Gln Ile Glu Glu Leu Glu
       310                 315                 320
   GAA GCA AGA AGA GAA TTG CAA CTT GAG CTT GAG AAA GGC ATT ACC CTT     1124
   Glu Ala Arg Arg Glu Leu Gln Leu Glu Leu Glu Lys Gly Ile Thr Leu
   325                 330                 335                 340
   GAT GAG TTG CGG AAA ACG ATT ACA AAA GGG GAG ATA AAA ACT AAG GTG     1172
   Asp Glu Leu Arg Lys Thr Ile Thr Lys Gly Glu Ile Lys Thr Lys Val
                   345                 350                 355
   GAA AAG CAC CTG AAA AGA AGT TCT TTT GCC GTT GAA AGA ATC CAA AGA     1220
   Glu Lys His Leu Lys Arg Ser Ser Phe Ala Val Glu Arg Ile Gln Arg
               360                 365                 370
   AAG AAG AGA GAC TTT GGG CAT CTT ATT AAT AAG TAT ACT TCC AGT CCT     1268
   Lys Lys Arg Asp Phe Gly His Leu Ile Asn Lys Tyr Thr Ser Ser Pro
           375                 380                 385
   GCA GTA CAA GTA CAA AAG GTC TTG GAA GAA CCA CCA GCC TTA TCT AAA     1316
   Ala Val Gln Val Gln Lys Val Leu Glu Glu Pro Pro Ala Leu Ser Lys
       390                 395                 400
   ATT AAG CTG TAT GCC AAG GAG AAG GAG GAG CAG ATT GAT GAT CCG ATC     1364
   Ile Lys Leu Tyr Ala Lys Glu Lys Glu Glu Gln Ile Asp Asp Pro Ile
   405                 410                 415                 420
   CTA AAT AAA AAG ATC TTT AAG GTC GAT GAT GGG GAG CTA CTG GTA CTG     1412
   Leu Asn Lys Lys Ile Phe Lys Val Asp Asp Gly Glu Leu Leu Val Leu
                   425                 430                 435
   GTA GCA AAG TCC TCT GGG AAG ACA AAA GTA CAT CTA GCT ACA GAT CTG     1460
   Val Ala Lys Ser Ser Gly Lys Thr Lys Val His Leu Ala Thr Asp Leu
               440                 445                 450
   AAT CAG CCA ATT ACT CTT CAC TGG GCA TTA TCC AAA AGT CCT GGA GAG     1508
   Asn Gln Pro Ile Thr Leu His Trp Ala Leu Ser Lys Ser Pro Gly Glu
           455                 460                 465
   TGG ATG GTA CCA CCT TCA AGC ATA TTG CCT CCT GGG TCA ATT ATT TTA     1556
   Trp Met Val Pro Pro Ser Ser Ile Leu Pro Pro Gly Ser Ile Ile Leu
       470                 475                 480
   GAC AAG GCT GCC GAA ACA CCT TTT TCA GCC AGT TCT TCT GAT GGT CTA     1604
   Asp Lys Ala Ala Glu Thr Pro Phe Ser Ala Ser Ser Ser Asp Gly Leu
   485                 490                 495                 500
   ACT TCT AAG GTA CAA TCT TTG GAT ATA GTA ATT GAA GAT GGC AAT TTT     1652
   Thr Ser Lys Val Gln Ser Leu Asp Ile Val Ile Glu Asp Gly Asn Phe
                   505                 510                 515
   GTG GGG ATG CCA TTT GTT CTT TTG TCT GGT GAA AAA TGG ATT AAG AAC      1700
   Val Gly Met Pro Phe Val Leu Leu Ser Gly Glu Lys Trp Ile Lys Asn
               520                 525                 530
   CAA GGG TCG GAT TTC TAT GTT GGC TTC AGT GCT GCA TCC AAA TTA GCA      1748
   Gln Gly Ser Asp Phe Tyr Val Gly Phe Ser Ala Ala Ser Lys Leu Ala
           535                 540                 545
   CTC AAG GCT GCT GGG GAT GGC AGT GGA ACT GCA AAG TCT TTA CTG GAT      1796
   Leu Lys Ala Ala Gly Asp Gly Ser Gly Thr Ala Lys Ser Leu Leu Asp
       550                 555                 560
   AAA ATA GCA GAT ATG GAA AGT GAG GCT CAG AAG TCA TTT ATG CAC CGG      1844
   Lys Ile Ala Asp Met Glu Ser Glu Ala Gln Lys Ser Phe Met His Arg
   565                 570                 575                 580
   TTT AAT ATT GCA GCT GAC TTG ATA GAA GAT GCC ACT AGT GCT GGT GAA      1892
   Phe Asn Ile Ala Ala Asp Leu Ile Glu Asp Ala Thr Ser Ala Gly Glu
                   585                 590                 595
   CTT GGT TTT GCT GGA ATT CTT GTA TGG ATG AGG TTC ATG GCT ACA AGG      1940
   Leu Gly Phe Ala Gly Ile Leu Val Trp Met Arg Phe Met Ala Thr Arg
               600                 605                 610
   CAA CTG ATA TGG AAC AAA AAC TAT AAC GTA AAA CCA CGT GAA ATA AGC      1988
   Gln Leu Ile Trp Asn Lys Asn Tyr Asn Val Lys Pro Arg Glu Ile Ser
           615                 620                 625
   AAG GCT CAG GAC AGA CTT ACA GAC TTG TTG CAG AAT GCT TTC ACC AGT      2036
   Lys Ala Gln Asp Arg Leu Thr Asp Leu Leu Gln Asn Ala Phe Thr Ser
       630                 635                 640
   CAC CCT CAG TAC CGT GAA ATT TTG CGG ATG ATT ATG TCA ACT GTT GGA      2084
   His Pro Gln Tyr Arg Glu Ile Leu Arg Met Ile Met Ser Thr Val Gly
   645                 650                 655                 660
   CGT GGA GGT GAA GGG GAT GTA GGA CAG CGA ATT AGG GAT GAA ATT TTG      2132
   Arg Gly Gly Glu Gly Asp Val Gly Gln Arg Ile Arg Asp Glu Ile Leu
                   665                 670                 675
   GTC ATC CAG AGG AAC AAT GAC TGC AAG GGT GGT ATG ATG CAA GAA TGG      2180
   Val Ile Gln Arg Asn Asn Asp Cys Lys Gly Gly Met Met Gln Glu Trp
               680                 685                 690
   CAT CAG AAA TTG CAT AAT AAT ACT AGT CCT GAT GAT GTT GTG ATC TGT      2228
   His Gln Lys Leu His Asn Asn Thr Ser Pro Asp Asp Val Val Ile Cys
           695                 700                 705
   CAG GCA TTA ATT GAC TAC ATC AAG AGT GAT TTT GAT CTT GGT GTT TAT      2276
   Gln Ala Leu Ile Asp Tyr Ile Lys Ser Asp Phe Asp Leu Gly Val Tyr
       710                 715                 720
   TGG AAA ACC CTG AAT GAG AAC GGA ATA ACA AAA GAG CGT CTT TTG AGT      2324
   Trp Lys Thr Leu Asn Glu Asn Gly Ile Thr Lys Glu Arg Leu Leu Ser
   725                 730                 735                 740
   TAT GAC CGT GCT ATC CAT TCT GAA CCA AAT TTT AGA GGA GAT CAA AAG      2372
   Tyr Asp Arg Ala Ile His Ser Glu Pro Asn Phe Arg Gly Asp Gln Lys
                   745                 750                 755
   GGT GGT CTT TTG CGT GAT TTA GGT CAC TAT ATG AGA ACA TTG AAG GCA      2420
   Gly Gly Leu Leu Arg Asp Leu Gly His Tyr Met Arg Thr Leu Lys Ala
               760                 765                 770
   GTT CAT TCA GGT GCA GAT CTT GAG TCT GCT ATT GCA AAC TGC ATG GGC      2468
   Val His Ser Gly Ala Asp Leu Glu Ser Ala Ile Ala Asn Cys Met Gly
           775                 780                 785
   TAC AAA ACT GAG GGA GAA GGC TTT ATG GTT GGA GTC CAG ATA AAT CCT      2516
   Tyr Lys Thr Glu Gly Glu Gly Phe Met Val Gly Val Gln Ile Asn Pro
       790                 795                 800
   GTA TCA GGC TTG CCA TCT GGC TTT CAG GAC CTC CTC CAT TTT GTC TTA      2564
   Val Ser Gly Leu Pro Ser Gly Phe Gln Asp Leu Leu His Phe Val Leu
   805                 810                 815                 820
   GAC CAT GTG GAA GAT AAA AAT GTG GAA ACT CTT CTT GAG AGA TTG CTA      2612
   Asp His Val Glu Asp Lys Asn Val Glu Thr Leu Leu Glu Arg Leu Leu
                   825                 830                 835
   GAG GCT CGT GAG GAG CTT AGG CCC TTG CTT CTC AAA CCA AAC AAC CGT      2660
   Glu Ala Arg Glu Glu Leu Arg Pro Leu Leu Leu Lys Pro Asn Asn Arg
               840                 845                 850
   CTA AAG GAT CTG CTG TTT TTG GAC ATA GCA CTT GAT TCT ACA GTT AGA      2708
   Leu Lys Asp Leu Leu Phe Leu Asp Ile Ala Leu Asp Ser Thr Val Arg
           855                 860                 865
   ACA GCA GTA GAA AGG GGA TAT GAA GAA TTG AAC AAC GCT AAT CCT GAG      2756
   Thr Ala Val Glu Arg Gly Tyr Glu Glu Leu Asn Asn Ala Asn Pro Glu
       870                 875                 880
   AAA ATC ATG TAC TTC ATC TCC CTC GTT CTT GAA AAT CTC GCA CTC TCT      2804
   Lys Ile Met Tyr Phe Ile Ser Leu Val Leu Glu Asn Leu Ala Leu Ser
   885                 890                 895                 900
   GTG GAC GAT AAT GAA GAT CTT GTT TAT TGC TTG AAG GGA TGG AAT CAA      2852
   Val Asp Asp Asn Glu Asp Leu Val Tyr Cys Leu Lys Gly Trp Asn Gln
                   905                 910                 915
   GCT CTT TCA ATG TCC AAT GGT GGG GAC AAC CAT TGG GCT TTA TTT GCA      2900
   Ala Leu Ser Met Ser Asn Gly Gly Asp Asn His Trp Ala Leu Phe Ala
               920                 925                 930
   AAA GCT GTG CTT GAC AGA ACC CGT CTT GCA CTT GCA AGC AAG GCA GAG      2948
   Lys Ala Val Leu Asp Arg Thr Arg Leu Ala Leu Ala Ser Lys Ala Glu
           935                 940                 945
   TGG TAC CAT CAC TTA TTG CAG CCA TCT GCC GAA TAT CTA GGA TCA ATA      2996
   Trp Tyr His His Leu Leu Gln Pro Ser Ala Glu Tyr Leu Gly Ser Ile
       950                 955                 960
   CTT GGG GTG GAC CAA TGG GCT TTG AAC ATA TTT ACT GAA GAA ATT ATA      3044
   Leu Gly Val Asp Gln Trp Ala Leu Asn Ile Phe Thr Glu Glu Ile Ile
   965                 970                 975                 980
   CGT GCT GGA TCA GCA GCT TCA TTA TCC TCT CTT CTT AAT AGA CTC GAT      3092
   Arg Ala Gly Ser Ala Ala Ser Leu Ser Ser Leu Leu Asn Arg Leu Asp
                   985                 990                 995
   CCC GTG CTT CGG AAA ACT GCA AAT CTA GGA AGT TGG CAG ATT ATC AGT        3140
   Pro Val Leu Arg Lys Thr Ala Asn Leu Gly Ser Trp Gln Ile Ile Ser
               1000                1005                1010
   CCA GTT GAA GCC GTT GGA TAT GTT GTC GTT GTG GAT GAG TTG CTT TCA        3188
   Pro Val Glu Ala Val Gly Tyr Val Val Val Val Asp Glu Leu Leu Ser
           1015                1020                1025
   GTT CAG AAT GAA ATC TAC GAG AAG CCC ACG ATC TTA GTA GCA AAA TCT        3236
   Val Gln Asn Glu Ile Tyr Glu Lys Pro Thr Ile Leu Val Ala Lys Ser
       1030                1035                1040
   GTT AAA GGA GAG GAG GAA ATT CCT GAT GGT GCT GTT GCC CTG ATA ACA        3284
   Val Lys Gly Glu Glu Glu Ile Pro Asp Gly Ala Val Ala Leu Ile Thr
   1045                1050                1055                1060
   CCA GAC ATG CCA GAT GTT CTT TCA CAT GTT TCT GTT CGA GCT AGA AAT        3332
   Pro Asp Met Pro Asp Val Leu Ser His Val Ser Val Arg Ala Arg Asn
                   1065                1070                1075
   GGG AAG GTT TGC TTT GCT ACA TGC TTT GAT CCC AAT ATA TTG GCT GAC        3380
   Gly Lys Val Cys Phe Ala Thr Cys Phe Asp Pro Asn Ile Leu Ala Asp
               1080                1085                1090
   CTC CAA GCA AAG GAA GGA AGG ATT TTG CTC TTA AAG CCT ACA CCT TCA        3428
   Leu Gln Ala Lys Glu Gly Arg Ile Leu Leu Leu Lys Pro Thr Pro Ser
           1095                1100                1105
   GAC ATA ATC TAT AGT GAG GTG AAT GAG ATT GAG CTC CAA AGT TCA AGT        3476
   Asp Ile Ile Tyr Ser Glu Val Asn Glu Ile Glu Leu Gln Ser Ser Ser
       1110                1115                1120
   AAC TTG GTA GAA GCT GAA ACT TCA GCA ACA CTT AGA TTG GTG AAA AAG        3524
   Asn Leu Val Glu Ala Glu Thr Ser Ala Thr Leu Arg Leu Val Lys Lys
   1125                1130                1135                1140
   CAA TTT GGT GGT TGT TAC GCA ATA TCA GCA GAT GAA TTC ACA AGT GAA        3572
   Gln Phe Gly Gly Cys Tyr Ala Ile Ser Ala Asp Glu Phe Thr Ser Glu
                   1145                1150                1155
   ATG GTT GGA GCT AAA TCA CGT AAT ATT GCA TAT CTG AAA GGA AAA GTG        3620
   Met Val Gly Ala Lys Ser Arg Asn Ile Ala Tyr Leu Lys Gly Lys Val
               1160                1165                1170
   CCT TCC TCG GTG GGA ATT CCT ACG TCA GTA GCT CTT CCA TTT GGA GTC        3668
   Pro Ser Ser Val Gly Ile Pro Thr Ser Val Ala Leu Pro Phe Gly Val
           1175                1180                1185
   TTT GAG AAA GTA CTT TCA GAC GAC ATA AAT CAG GGA GTG GCA AAA GAG        3716
   Phe Glu Lys Val Leu Ser Asp Asp Ile Asn Gln Gly Val Ala Lys Glu
       1190                1195                1200
   TTG CAA ATT CTG ATG AAA AAA CTA TCT GAA GGA GAC TTC AGC GCT CTT        3764
   Leu Gln Ile Leu Met Lys Lys Leu Ser Glu Gly Asp Phe Ser Ala Leu
   1205                1210                1215                1220
   GGT GAA ATT CGC ACA ACG GTT TTA GAT CTT TCA GCA CCA GCT CAA TTG        3812
   Gly Glu Ile Arg Thr Thr Val Leu Asp Leu Ser Ala Pro Ala Gln Leu
                   1225                1230                1235
   GTC AAA GAG CTG AAG GAG AAG ATG CAG GGT TCT GGC ATG CCT TGG CCT      3860
   Val Lys Glu Leu Lys Glu Lys Met Gln Gly Ser Gly Met Pro Trp Pro
               1240                1245                1250
   GGT GAT GAA GGT CCA AAG CGG TGG GAA CAA GCA TGG ATG GCC ATA AAA      3908
   Gly Asp Glu Gly Pro Lys Arg Trp Glu Gln Ala Trp Met Ala Ile Lys
           1255                1260                1265
   AAG GTG TGG GCT TCA AAA TGG AAT GAG AGA GCA TAC TTC AGC ACA AGG      3956
   Lys Val Trp Ala Ser Lys Trp Asn Glu Arg Ala Tyr Phe Ser Thr Arg
       1270                1275                1280
   AAG GTG AAA CTG GAT CAT GAC TAT CTG TGC ATG GCT GTC CTT GTT CAA      4004
   Lys Val Lys Leu Asp His Asp Tyr Leu Cys Met Ala Val Leu Val Gln
   1285                1290                1295                1300
   GAA ATA ATA AAT GCT GAT TAT GCA TTT GTC ATT CAC ACA ACC AAC CCA      4052
   Glu Ile Ile Asn Ala Asp Tyr Ala Phe Val Ile His Thr Thr Asn Pro
                   1305                1310                1315
   TCT TCC GGA GAC GAC TCA GAA ATA TAT GCC GAG GTG GTC AGG GGC CTT      4100
   Ser Ser Gly Asp Asp Ser Glu Ile Tyr Ala Glu Val Val Arg Gly Leu
               1320                1325                1330
   GGG GAA ACA CTT GTT GGA GCT TAT CCA GGA CGT GCT TTG AGT TTT ATC      4148
   Gly Glu Thr Leu Val Gly Ala Tyr Pro Gly Arg Ala Leu Ser Phe Ile
           1335                1340                1345
   TGC AAG AAA AAG GAT CTC AAC TCT CCT CAA GTG TTA GGT TAC CCA AGC      4196
   Cys Lys Lys Lys Asp Leu Asn Ser Pro Gln Val Leu Gly Tyr Pro Ser
       1350                1355                1360
   AAA CCG ATC GGC CTT TTC ATA AAA AGA TCT ATC ATC TTC CGA TCT GAT      4244
   Lys Pro Ile Gly Leu Phe Ile Lys Arg Ser Ile Ile Phe Arg Ser Asp
   1365                1370                1375                1380
   TCC AAT GGG GAA GAT TTG GAA GGT TAT GCC GGT GCT GGC CTC TAC GAC      4292
   Ser Asn Gly Glu Asp Leu Glu Gly Tyr Ala Gly Ala Gly Leu Tyr Asp
                   1385                1390                1395
   AGT GTA CCA ATG GAT GAG GAG GAA AAA GTT GTA ATT GAT TAC TCT TCC      4340
   Ser Val Pro Met Asp Glu Glu Glu Lys Val Val Ile Asp Tyr Ser Ser
               1400                1405                1410
   GAC CCA TTG ATA ACT GAT GGT AAC TTC CGC CAG ACA ATC CTG TCC AAC      4388
   Asp Pro Leu Ile Thr Asp Gly Asn Phe Arg Gln Thr Ile Leu Ser Asn
           1415                1420                1425
   ATT GCT CGT GCT GGA CAT GCT ATC GAG GAG CTA TAT GGC TCT CCT CAA      4436
   Ile Ala Arg Ala Gly His Ala Ile Glu Glu Leu Tyr Gly Ser Pro Gln
       1430                1435                1440
   GAC ATT GAG GGT GTA GTG AGG GAT GGA AAG ATT TAT GTC GTT CAG ACA      4484
   Asp Ile Glu Gly Val Val Arg Asp Gly Lys Ile Tyr Val Val Gln Thr
   1445                1450                1455                1460
   AGA CCA CAG ATG T GATTATATTC TCGTTGTATG TTGTTCAGAG AAGACCACAG        4537
   Arg Pro Gln Met
   ATGTGATCAT ATTCTCATTG TATCAGATCT GTGACCACTT ACCTGATACC TCCCATGAAG   4597
   TTACCTGTAT GATTATACGT GATCCAAAGC CATCACATCA TGTTCACCTT CAGCTATTGG   4657
   AGGAGAAGTG AGAAGTAGGA ATTGCAATAT GAGGAATAAT AAGAAAAACT TTGTAAAAGC   4717
   TAAATTAGCT GGGTATGATA TAGGGAGAAA TGTGTAAACA TTGTACTATA TATAGTATAT   4777
   ACACACGCAT TATGTATTGC ATTATGCACT GAATAATATC GCAGCATCAA AGAAGAAATC   4837
   CTTTGGGTGG TTTCAAAAA                                                4856
(2) ANGABEN ZU SEQ ID NO: 2:
   (i) SEQUENZKENNZEICHEN:
      (A) LÄNGE: 1464 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Ser Asn Ser Leu Gly Asn Asn Leu Leu Tyr Gln Gly Phe Leu Thr
    1               5                   10                  15
   Ser Thr Val Leu Glu His Lys Ser Arg Ile Ser Pro Pro Cys Val Gly
               20                  25                  30
   Gly Asn Ser Leu Phe Gln Gln Gln Val Ile Ser Lys Ser Pro Leu Ser
           35                  40                  45
   Thr Glu Phe Arg Gly Asn Arg Leu Lys Val Gln Lys Lys Lys Ile Pro
       50                  55                  60
   Met Glu Lys Lys Arg Ala Phe Ser Ser Ser Pro His Ala Val Leu Thr
   65                  70                  75                  80
   Thr Asp Thr Ser Ser Glu Leu Ala Glu Lys Phe Ser Leu Gly Gly Asn
                   85                  90                  95
   Ile Glu Leu Gln Val Asp Val Arg Pro Pro Thr Ser Gly Asp Val Ser
               100                 105                 110
   Phe Val Asp Phe Gln Val Thr Asn Gly Ser Asp Lys Leu Phe Leu His
           115                 120                 125
   Trp Gly Ala Val Lys Phe Gly Lys Glu Thr Trp Ser Leu Pro Asn Asp
       130                 135                 140
   Arg Pro Asp Gly Thr Lys Val Tyr Lys Asn Lys Ala Leu Arg Thr Pro
   145                 150                 155                 160
   Phe Val Lys Ser Gly Ser Asn Ser Ile Leu Arg Leu Glu Ile Arg Asp
                   165                 170                 175
   Thr Ala Ile Glu Ala Ile Glu Phe Leu Ile Tyr Asp Glu Ala His Asp
               180                 185                 190
   Lys Trp Ile Lys Asn Asn Gly Gly Asn Phe Arg Val Lys Leu Ser Arg
           195                 200                 205
   Lys Glu Ile Arg Gly Pro Asp Val Ser Val Pro Glu Glu Leu Val Gln
       210                 215                 220
   Ile Gln Ser Tyr Leu Arg Trp Glu Arg Lys Gly Lys Gln Asn Tyr Pro
   225                 230                 235                 240
   Pro Glu Lys Glu Lys Glu Glu Tyr Glu Ala Ala Arg Thr Val Leu Gln
                   245                 250                 255
   Glu Glu Ile Ala Arg Gly Ala Ser Ile Gln Asp Ile Arg Ala Arg Leu
               260                 265                 270
   Thr Lys Thr Asn Asp Lys Ser Gln Ser Lys Glu Glu Pro Leu His Val
           275                 280                 285
   Thr Lys Ser Asp Ile Pro Asp Asp Leu Ala Gln Ala Gln Ala Tyr Ile
       290                 295                 300
   Arg Trp Glu Lys Ala Gly Lys Pro Asn Tyr Pro Pro Glu Lys Gln Ile
   305                 310                 315                 320
   Glu Glu Leu Glu Glu Ala Arg Arg Glu Leu Gln Leu Glu Leu Glu Lys
                   325                 330                 335
   Gly Ile Thr Leu Asp Glu Leu Arg Lys Thr Ile Thr Lys Gly Glu Ile
               340                 345                 350
   Lys Thr Lys Val Glu Lys His Leu Lys Arg Ser Ser Phe Ala Val Glu
           355                 360                 365
   Arg Ile Gln Arg Lys Lys Arg Asp Phe Gly His Leu Ile Asn Lys Tyr
       370                 375                 380
   Thr Ser Ser Pro Ala Val Gln Val Gln Lys Val Leu Glu Glu Pro Pro
   385                 390                 395                 400
   Ala Leu Ser Lys Ile Lys Leu Tyr Ala Lys Glu Lys Glu Glu Gln Ile
                   405                 410                 415
   Asp Asp Pro Ile Leu Asn Lys Lys Ile Phe Lys Val Asp Asp Gly Glu
               420                 425                 430
   Leu Leu Val Leu Val Ala Lys Ser Ser Gly Lys Thr Lys Val His Leu
           435                 440                 445
   Ala Thr Asp Leu Asn Gln Pro Ile Thr Leu His Trp Ala Leu Ser Lys
       450                 455                 460
   Ser Pro Gly Glu Trp Met Val Pro Pro Ser Ser Ile Leu Pro Pro Gly
   465                 470                 475                 480
   Ser Ile Ile Leu Asp Lys Ala Ala Glu Thr Pro Phe Ser Ala Ser Ser
                   485                 490                 495
   Ser Asp Gly Leu Thr Ser Lys Val Gln Ser Leu Asp Ile Val Ile Glu
               500                 505                 510
   Asp Gly Asn Phe Val Gly Met Pro Phe Val Leu Leu Ser Gly Glu Lys
           515                 520                 525
   Trp Ile Lys Asn Gln Gly Ser Asp Phe Tyr Val Gly Phe Ser Ala Ala
       530                 535                 540
   Ser Lys Leu Ala Leu Lys Ala Ala Gly Asp Gly Ser Gly Thr Ala Lys
   545                 550                 555                 560
   Ser Leu Leu Asp Lys Ile Ala Asp Met Glu Ser Glu Ala Gln Lys Ser
                   565                 570                 575
   Phe Met His Arg Phe Asn Ile Ala Ala Asp Leu Ile Glu Asp Ala Thr
               580                 585                 590
   Ser Ala Gly Glu Leu Gly Phe Ala Gly Ile Leu Val Trp Met Arg Phe
           595                 600                 605
   Met Ala Thr Arg Gln Leu Ile Trp Asn Lys Asn Tyr Asn Val Lys Pro
       610                 615                 620
   Arg Glu Ile Ser Lys Ala Gln Asp Arg Leu Thr Asp Leu Leu Gln Asn
   625                 630                 635                 640
   Ala Phe Thr Ser His Pro Gln Tyr Arg Glu Ile Leu Arg Met Ile Met
                   645                 650                 655
   Ser Thr Val Gly Arg Gly Gly Glu Gly Asp Val Gly Gln Arg Ile Arg
               660                 665                 670
   Asp Glu Ile Leu Val Ile Gln Arg Asn Asn Asp Cys Lys Gly Gly Met
           675                 680                 685
   Met Gln Glu Trp His Gln Lys Leu His Asn Asn Thr Ser Pro Asp Asp
       690                 695                 700
   Val Val Ile Cys Gln Ala Leu Ile Asp Tyr Ile Lys Ser Asp Phe Asp
   705                 710                 715                 720
   Leu Gly Val Tyr Trp Lys Thr Leu Asn Glu Asn Gly Ile Thr Lys Glu
                   725                 730                 735
   Arg Leu Leu Ser Tyr Asp Arg Ala Ile His Ser Glu Pro Asn Phe Arg
               740                 745                 750
   Gly Asp Gln Lys Gly Gly Leu Leu Arg Asp Leu Gly His Tyr Met Arg
           755                 760                 765
   Thr Leu Lys Ala Val His Ser Gly Ala Asp Leu Glu Ser Ala Ile Ala
       770                 775                 780
   Asn Cys Met Gly Tyr Lys Thr Glu Gly Glu Gly Phe Met Val Gly Val
   785                 790                 795                 800
   Gln Ile Asn Pro Val Ser Gly Leu Pro Ser Gly Phe Gln Asp Leu Leu
                   805                 810                 815
   His Phe Val Leu Asp His Val Glu Asp Lys Asn Val Glu Thr Leu Leu
               820                 825                 830
   Glu Arg Leu Leu Glu Ala Arg Glu Glu Leu Arg Pro Leu Leu Leu Lys
           835                 840                 845
   Pro Asn Asn Arg Leu Lys Asp Leu Leu Phe Leu Asp Ile Ala Leu Asp
       850                 855                 860
   Ser Thr Val Arg Thr Ala Val Glu Arg Gly Tyr Glu Glu Leu Asn Asn
   865                 870                 875                 880
   Ala Asn Pro Glu Lys Ile Met Tyr Phe Ile Ser Leu Val Leu Glu Asn
                   885                 890                 895
   Leu Ala Leu Ser Val Asp Asp Asn Glu Asp Leu Val Tyr Cys Leu Lys
               900                 905                 910
   Gly Trp Asn Gln Ala Leu Ser Met Ser Asn Gly Gly Asp Asn His Trp
           915                 920                 925
   Ala Leu Phe Ala Lys Ala Val Leu Asp Arg Thr Arg Leu Ala Leu Ala
       930                 935                 940
   Ser Lys Ala Glu Trp Tyr His His Leu Leu Gln Pro Ser Ala Glu Tyr
   945                 950                 955                 960
   Leu Gly Ser Ile Leu Gly Val Asp Gln Trp Ala Leu Asn Ile Phe Thr
                   965                 970                 975
   Glu Glu Ile Ile Arg Ala Gly Ser Ala Ala Ser Leu Ser Ser Leu Leu
               980                 985                 990
   Asn Arg Leu Asp Pro Val Leu Arg Lys Thr Ala Asn Leu Gly Ser Trp
           995                 1000                1005
   Gln Ile Ile Ser Pro Val Glu Ala Val Gly Tyr Val Val Val Val Asp
       1010                1015                1020
   Glu Leu Leu Ser Val Gln Asn Glu Ile Tyr Glu Lys Pro Thr Ile Leu
   1025                1030                1035                1040
   Val Ala Lys Ser Val Lys Gly Glu Glu Glu Ile Pro Asp Gly Ala Val
                   1045                1050                1055
   Ala Leu Ile Thr Pro Asp Met Pro Asp Val Leu Ser His Val Ser Val
               1060                1065                1070
   Arg Ala Arg Asn Gly Lys Val Cys Phe Ala Thr Cys Phe Asp Pro Asn
           1075                1080                1085
   Ile Leu Ala Asp Leu Gln Ala Lys Glu Gly Arg Ile Leu Leu Leu Lys
       1090                1095                1100
   Pro Thr Pro Ser Asp Ile Ile Tyr Ser Glu Val Asn Glu Ile Glu Leu
   1105                1110                1115                1120
   Gln Ser Ser Ser Asn Leu Val Glu Ala Glu Thr Ser Ala Thr Leu Arg
                   1125                1130                1135
   Leu Val Lys Lys Gln Phe Gly Gly Cys Tyr Ala Ile Ser Ala Asp Glu
               1140                1145                1150
   Phe Thr Ser Glu Met Val Gly Ala Lys Ser Arg Asn Ile Ala Tyr Leu
           1155                1160                1165
   Lys Gly Lys Val Pro Ser Ser Val Gly Ile Pro Thr Ser Val Ala Leu
       1170                1175                1180
   Pro Phe Gly Val Phe Glu Lys Val Leu Ser Asp Asp Ile Asn Gln Gly
   1185                1190                1195                1200
   Val Ala Lys Glu Leu Gln Ile Leu Met Lys Lys Leu Ser Glu Gly Asp
                   1205                1210                1215
   Phe Ser Ala Leu Gly Glu Ile Arg Thr Thr Val Leu Asp Leu Ser Ala
               1220                1225                1230
   Pro Ala Gln Leu Val Lys Glu Leu Lys Glu Lys Met Gln Gly Ser Gly
           1235                1240                1245
   Met Pro Trp Pro Gly Asp Glu Gly Pro Lys Arg Trp Glu Gln Ala Trp
       1250                1255                1260
   Met Ala Ile Lys Lys Val Trp Ala Ser Lys Trp Asn Glu Arg Ala Tyr
   1265                1270                1275                1280
   Phe Ser Thr Arg Lys Val Lys Leu Asp His Asp Tyr Leu Cys Met Ala
                   1285                1290                1295
   Val Leu Val Gln Glu Ile Ile Asn Ala Asp Tyr Ala Phe Val Ile His
               1300                1305                1310
   Thr Thr Asn Pro Ser Ser Gly Asp Asp Ser Glu Ile Tyr Ala Glu Val
           1315                1320                1325
   Val Arg Gly Leu Gly Glu Thr Leu Val Gly Ala Tyr Pro Gly Arg Ala
       1330                1335                1340
   Leu Ser Phe Ile Cys Lys Lys Lys Asp Leu Asn Ser Pro Gln Val Leu
   1345                1350                1355                1360
   Gly Tyr Pro Ser Lys Pro Ile Gly Leu Phe Ile Lys Arg Ser Ile Ile
                   1365                1370                1375
   Phe Arg Ser Asp Ser Asn Gly Glu Asp Leu Glu Gly Tyr Ala Gly Ala
               1380                1385                1390
   Gly Leu Tyr Asp Ser Val Pro Met Asp Glu Glu Glu Lys Val Val Ile
           1395                1400                1405
   Asp Tyr Ser Ser Asp Pro Leu Ile Thr Asp Gly Asn Phe Arg Gln Thr
       1410                1415                1420
   Ile Leu Ser Asn Ile Ala Arg Ala Gly His Ala Ile Glu Glu Leu Tyr
   1425                1430                1435                1440
   Gly Ser Pro Gln Asp Ile Glu Gly Val Val Arg Asp Gly Lys Ile Tyr
                   1445                1450                1455
   Val Val Gln Thr Arg Pro Gln Met
               1460
(2) ANGABEN ZU SEQ ID NO: 3:
   (i) SEQUENZKENNZEICHEN:
      (A) LÄNGE: 1918 Basenpaare
      (B) ART: Nucleotid.
      (C) STRANGFORM: Einzelstrang
      (D) TOPOLOGIE: linear
   (ii) ART DES MOLEKÜLS: cDNA zu mRNA
   (vi) URSPRÜNLICHE HERKUNFT:
      (A) ORGANISMUS: Solanum tuberosum
      (B) STAMM: C.V. Desiree
   (ix) MERKMAL:
      (A) NAME/SCHLÜSSEL: CDS
      (B) LAGE: 1..1555
   (xi) SEQUENZBESCHREIBUNG: SEQ ID NO: 3:
   GCA GAG TGG TAC CAT CAC TTA TTG CAG CCA TCT GCC GAA TAT CTA GGA      48
   Ala Glu Trp Tyr His His Leu Leu Gln Pro Ser Ala Glu Tyr Leu Gly
     1               5                  10                  15
   TCA ATA CTT GGG GTG GAC CAA TGG GCT TTG AAC ATA TTT ACT GAA GAA      96
   Ser Ile Leu Gly Val Asp Gln Trp Ala Leu Asn Ile Phe Thr Glu Glu
                20                  25                  30
   ATT ATA CGT GCT GGA TCA GCA GCT TCA TTA TCC TCT CTT CTT AAT AGA     144
   Ile Ile Arg Ala Gly Ser Ala Ala Ser Leu Ser Ser Leu Leu Asn Arg
            35                  40                  45
   CTC GAT CCC GTG CTT CGG AAA ACT GCA AAT CTA GGA AGT TGG CAG ATT     192
   Leu Asp Pro Val Leu Arg Lys Thr Ala Asn Leu Gly Ser Trp Gln Ile
        50                  55                  60
   ATC AGT CCA GTT GAA GCC GTT GGA TAT GTT GTC GTT GTG GAT GAG TTG     240
   Ile Ser Pro Val Glu Ala Val Gly Tyr Val Val Val Val Asp Glu Leu
    65                  70                  75                  80
   CTT TCA GTT CAG AAT GAA ATC TAC GAG AAG CCC ACG ATC TTA GTA GCA     288
   Leu Ser Val Gln Asn Glu Ile Tyr Glu Lys Pro Thr Ile Leu Val Ala
                    85                  90                  95
   AAA TCT GTT AAA GGA GAG GAG GAA ATT CCT GAT GGT GCT GTT GCC CTG     336
   Lys Ser Val Lys Gly Glu Glu Glu Ile Pro Asp Gly Ala Val Ala Leu
               100                 105                 110
   ATA ACA CCA GAC ATG CCA GAT GTT CTT TCA CAT GTT TCT GTT CGA GCT     384
   Ile Thr Pro Asp Met Pro Asp Val Leu Ser His Val Ser Val Arg Ala
           115                 120                 125
   AGA AAT GGG AAG GTT TGC TTT GCT ACA TGC TTT GAT CCC AAT ATA TTG     432
   Arg Asn Gly Lys Val Cys Phe Ala Thr Cys Phe Asp Pro Asn Ile Leu
       130                 135                 140
   GCT GAC CTC CAA GCA AAG GAA GGA AGG ATT TTG CTC TTA AAG CCT ACA     480
   Ala Asp Leu Gln Ala Lys Glu Gly Arg Ile Leu Leu Leu Lys Pro Thr
   145                 150                 155                 160
   CCT TCA GAC ATA ATC TAT AGT GAG GTG AAT GAG ATT GAG CTC CAA AGT         528
   Pro Ser Asp Ile Ile Tyr Ser Glu Val Asn Glu Ile Glu Leu Gln Ser
                   165                 170                 175
   TCA AGT AAC TTG GTA GAA GCT GAA ACT TCA GCA ACA CTT AGA TTG GTG         576
   Ser Ser Asn Leu Val Glu Ala Glu Thr Ser Ala Thr Leu Arg Leu Val
               180                 185                 190
   AAA AAG CAA TTT GGT GGT TGT TAC GCA ATA TCA GCA GAT GAA TTC ACA         624
   Lys Lys Gln Phe Gly Gly Cys Tyr Ala Ile Ser Ala Asp Glu Phe Thr
           195                 200                 205
   AGT GAA ATG GTT GGA GCT AAA TCA CGT AAT ATT GCA TAT CTG AAA GGA         672
   Ser Glu Met Val Gly Ala Lys Ser Arg Asn Ile Ala Tyr Leu Lys Gly
       210                 215                 220
   AAA GTG CCT TCC TCG GTG GGA ATT CCT ACG TCA GTA GCT CTT CCA TTT         720
   Lys Val Pro Ser Ser Val Gly Ile Pro Thr Ser Val Ala Leu Pro Phe
   225                 230                 235                     240
   GGA GTC TTT GAG AAA GTA CTT TCA GAC GAC ATA AAT CAG GGA GTG GCA         768
   Gly Val Phe Glu Lys Val Leu Ser Asp Asp Ile Asn Gln Gly Val Ala
                   245                 250                 255
   AAA GAG TTG CAA ATT CTG ACA AAA AAA CTA TCT GAA GGA GAC TTT AGC         816
   Lys Glu Leu Gln Ile Leu Thr Lys Lys Leu Ser Glu Gly Asp Phe Ser
               260                 265                 270
   GCT CTT GGT GAA ATT CGC ACA ACG GTT TTA GAT CTT TCG ACA CCA GCT         864
   Ala Leu Gly Glu Ile Arg Thr Thr Val Leu Asp Leu Ser Thr Pro Ala
           275                 280                 285
   CAA TTG GTC AAA GAG CTG AAG GAG AAG ATG CAG GGT TCT GGC ATG CCT         912
   Gln Leu Val Lys Glu Leu Lys Glu Lys Met Gln Gly Ser Gly Met Pro
       290                 295                 300
   TGG CCT GGT GAT GAA GGT CCA AAG CGG TGG GAA CAA GCA TGG ATG GCC         960
   Trp Pro Gly Asp Glu Gly Pro Lys Arg Trp Glu Gln Ala Trp Met Ala
   305                 310                 315                     320
   ATA AAA AAG GTG TGG GCT TCA AAA TGG AAT GAG AGA GCA TAC TTC AGC        1008
   Ile Lys Lys Val Trp Ala Ser Lys Trp Asn Glu Arg Ala Tyr Phe Ser
                   325                 330                 335
   ACA AGG AAG GTG AAA CTG GAT CAT GAC TAT CTG TGC ATG GCT GTC CTT        1056
   Thr Arg Lys Val Lys Leu Asp His Asp Tyr Leu Cys Met Ala Val Leu
               340                 345                 350
   GTT CAA GAA ATA ATA AAT GCT GAT TAT GCA TTT GTC ATT CAC ACA ACC        1104
   Val Gln Glu Ile Ile Asn Ala Asp Tyr Ala Phe Val Ile His Thr Thr
           355                 360                 365
   AAC CCA TCT TCC GGA GAC GAC TCA GAA ATA TAT GCC GAG GTG GTC AGG        1152
   Asn Pro Ser Ser Gly Asp Asp Ser Glu Ile Tyr Ala Glu Val Val Arg
       370                 375                 380
   GGC CTT GGG GAA ACA CTT GTT GGA GCT TAT CCA GGA CGT GCT TTG AGT        1200
   Gly Leu Gly Glu Thr Leu Val Gly Ala Tyr Pro Gly Arg Ala Leu Ser
   385                 390                 395                     400
   TTT ATC TGC AAG AAA AAG GAT CTC AAC TCT CCT CAA GTG TTA GGT TAC     1248
   Phe Ile Cys Lys Lys Lys Asp Leu Asn Ser Pro Gln Val Leu Gly Tyr
                   405                 410                 415
   CCA AGC AAA CCG ATC GGC CTT TTC ATA AAA AGA TCT ATC ATC TTC CGA     1296
   Pro Ser Lys Pro Ile Gly Leu Phe Ile Lys Arg Ser Ile Ile Phe Arg
               420                 425                 430
   TCT GAT TCC AAT GGG GAA GAT TTG GAA GGT TAT GCC GGT GCT GGC CTC     1344
   Ser Asp Ser Asn Gly Glu Asp Leu Glu Gly Tyr Ala Gly Ala Gly Leu
           435                 440                 445
   TAC GAC AGT GTA CCA ATG GAT GAG GAG GAA AAA GTT GTA ATT GAT TAC     1392
   Tyr Asp Ser Val Pro Met Asp Glu Glu Glu Lys Val Val Ile Asp Tyr
       450                 455                 460
   TCT TCC GAC CCA TTG ATA ACT GAT GGT AAC TTC CGC CAG ACA ATC CTG     1440
   Ser Ser Asp Pro Leu Ile Thr Asp Gly Asn Phe Arg Gln Thr Ile Leu
   465                 470                 475                 480
   TCC AAC ATT GCT CGT GCT GGA CAT GCT ATC GAG GAG CTA TAT GGC TCT     1488
   Ser Asn Ile Ala Arg Ala Gly His Ala Ile Glu Glu Leu Tyr Gly Ser
                   485                 490                 495
   CCT CAA GAC ATT GAG GGT GTA GTG AGG GAT GGA AAG ATT TAT GTC GTT     1536
   Pro Gln Asp Ile Glu Gly Val Val Arg Asp Gly Lys Ile Tyr Val Val
               500                 505                 510
   CAG ACA AGA CCA CAG ATG T GATTATATTC TCGTTGTATG TTGTTCAGAG          1585
   Gln Thr Arg Pro Gln Met
           515
   AAGACCACAG ATGTGATCAT ATTCTCATTG TATCAGATCT GTGACCACTT ACCTGATACC   1645
   TCCCATGAAG TTACCTGTAT GATTATACGT GATCCAAAGC CATCACATCA TGTTCACCTT   1705
   CAGCTATTGG AGGAGAAGTG AGAAGTAGGA ATTGCAATAT GAGGAATAAT AAGAAAAACT   1765
   TTGTAAAAGC TAAATTAGCT GGGTATGATA TAGGGAGAAA TGTGTAAACA TTGTACTATA   1825
   TATAGTATAT ACACACGCAT TATGTATTGC ATTATGCACT GAATAATATC GCAGCATCAA   1885
   AGAAGAAATC CTTTGGGTGG TTTCAAAAAA AAA                                1918
(2) ANGABEN ZU SEQ ID NO: 4:
   (i) SEQUENZKENNZEICHEN:
      (A) LÄNGE: 518 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 4:
   Ala Glu Trp Tyr His His Leu Leu Gln Pro Ser Ala Glu Tyr Leu Gly
    1               5                  10                  15
   Ser Ile Leu Gly Val Asp Gln Trp Ala Leu Asn Ile Phe Thr Glu Glu
                20                  25                  30
   Ile Ile Arg Ala Gly Ser Ala Ala Ser Leu Ser Ser Leu Leu Asn Arg
           35                  40                  45
   Leu Asp Pro Val Leu Arg Lys Thr Ala Asn Leu Gly Ser Trp Gln Ile
       50                  55                  60
   Ile Ser Pro Val Glu Ala Val Gly Tyr Val Val Val Val Asp Glu Leu
   65                  70                  75                  80
   Leu Ser Val Gln Asn Glu Ile Tyr Glu Lys Pro Thr Ile Leu Val Ala
                   85                  90                  95
   Lys Ser Val Lys Gly Glu Glu Glu Ile Pro Asp Gly Ala Val Ala Leu
               100                 105                 110
   Ile Thr Pro Asp Met Pro Asp Val Leu Ser His Val Ser Val Arg Ala
           115                 120                 125
   Arg Asn Gly Lys Val Cys Phe Ala Thr Cys Phe Asp Pro Asn Ile Leu
       130                 135                 140
   Ala Asp Leu Gln Ala Lys Glu Gly Arg Ile Leu Leu Leu Lys Pro Thr
   145                 150                 155                 160
   Pro Ser Asp Ile Ile Tyr Ser Glu Val Asn Glu Ile Glu Leu Gln Ser
                   165                 170                 175
   Ser Ser Asn Leu Val Glu Ala Glu Thr Ser Ala Thr Leu Arg Leu Val
               180                 185                 190
   Lys Lys Gln Phe Gly Gly Cys Tyr Ala Ile Ser Ala Asp Glu Phe Thr
           195                 200                 205
   Ser Glu Met Val Gly Ala Lys Ser Arg Asn Ile Ala Tyr Leu Lys Gly
       210                 215                 220
   Lys Val Pro Ser Ser Val Gly Ile Pro Thr Ser Val Ala Leu Pro Phe
   225                 230                 235                 240
   Gly Val Phe Glu Lys Val Leu Ser Asp Asp Ile Asn Gln Gly Val Ala
                   245                 250                 255
   Lys Glu Leu Gln Ile Leu Thr Lys Lys Leu Ser Glu Gly Asp Phe Ser
               260                 265                 270
   Ala Leu Gly Glu Ile Arg Thr Thr Val Leu Asp Leu Ser Thr Pro Ala
           275                 280                 285
   Gln Leu Val Lys Glu Leu Lys Glu Lys Met Gln Gly Ser Gly Met Pro
       290                 295                 300
   Trp Pro Gly Asp Glu Gly Pro Lys Arg Trp Glu Gln Ala Trp Met Ala
   305                 310                 315                 320
   Ile Lys Lys Val Trp Ala Ser Lys Trp Asn Glu Arg Ala Tyr Phe Ser
                   325                 330                 335
   Thr Arg Lys Val Lys Leu Asp His Asp Tyr Leu Cys Met Ala Val Leu
               340                 345                 350
   Val Gln Glu Ile Ile Asn Ala Asp Tyr Ala Phe Val Ile His Thr Thr
   355 360 365
   Asn Pro Ser Ser Gly Asp Asp Ser Glu Ile Tyr Ala Glu Val Val Arg
   370 375 380
   Gly Leu Gly Glu Thr Leu Val Gly Ala Tyr Pro Gly Arg Ala Leu Ser
   385 390 395 400
   Phe Ile Cys Lys Lys Lys Asp Leu Asn Ser Pro Gln Val Leu Gly Tyr
   405 410 415
   Pro Ser Lys Pro Ile Gly Leu Phe Ile Lys Arg Ser Ile Ile Phe Arg
   420 425 430
   Ser Asp Ser Asn Gly Glu Asp Leu Glu Gly Tyr Ala Gly Ala Gly Leu
   435 440 445
   Tyr Asp Ser Val Pro Met Asp Glu Glu Glu Lys Val Val Ile Asp Tyr
   450 455 460
   Ser Ser Asp Pro Leu Ile Thr Asp Gly Asn Phe Arg Gln Thr Ile Leu
   465 470 475 480
   Ser Asn Ile Ala Arg Ala Gly His Ala Ile Glu Glu Leu Tyr Gly Ser
   485 490 495
   Pro Gln Asp Ile Glu Gly Val Val Arg Asp Gly Lys Ile Tyr Val Val
   500 505 510
   Gln Thr Arg Pro Gln Met
   515

## Beschreibung

Die vorliegende Erfindung betrifft Verfahren zur Herstellung einer Stärke, bei denen Stärke aus transgenen Pflanzen oder Pflanzenzellen isoliert wird, welche mittels Nucleinsäuremolekülen, die ein Stärekekorn-gebundenes Protein codieren, modifiziert wurden.

Das Polysaccharid Stärke, das einen der wichtigsten Speicherstoffe im Pflanzenreich darstellt, findet neben der Verwendung im Nahrungsmittelbereich auch eine breite Verwendung als nachwachsender Rohstoff für die Herstellung industrieller Produkte. Um die Anwendung dieses Rohstoffes in möglichst vielen Einsatzgebieten zu ermöglichen, ist es notwendig, eine große Stoffvielfalt und eine Anpassung an die jeweiligen Anforderungen der zu verarbeitenden Industrie zu erreichen.
Obwohl Stärke aus einem chemisch einheitlichen Grundbaustein, der Glucose, aufgebaut ist, stellt Stärke keinen einheitlichen Rohstoff dar. Es handelt sich dabei eher um ein komplexes Gemisch aus unterschiedlichen Molekülformen, die sich hinsichtlich ihres Verzweigungsgrades und des Auftretens von Verzweigungen der Glucoseketten unterscheiden. Man unterscheidet insbesondere die Amylose-Stärke, ein im wesentlichen unverzweigtes Polymer aus α-1,4-verknüpften Glucosemolekülen, von der Amylopektin-Stärke, die ein Gemisch aus unterschiedlich stark verzweigten Glucoseketten darstellt, wobei die Verzweigungen durch das Auftreten von α-1,6-glycosidischen Verknüpfungen zustande kommen.

Die molekulare Struktur der Stärke, die zu einem großen Teil durch den Verzweigungsgrad, das Amylose/Amylopektin-Verhältnis, die durchschnittliche Kettenlänge sowie das Vorhandensein von Phosphatgruppen bestimmt wird, ist ausschlaggebend für wichtige funktionelle Eigenschaften der Stärke bzw. ihrer wäßrigen Lösungen. Als wichtige funktionelle Eigenschaften sind hierbei beispielsweise zu nennen die Löslichkeit, das Retrogradierungsverhalten, die Filmbildungseigenschaften, die Viskosität, die Farbstabilität, die Verkleisterungseigenschaften, d.h. Binde- und Klebeigenschaften, sowie die Kältestabilität. Auch die Stärkekorngröße kann für verschiedene Anwendungen von Bedeutung sein. Von besonderem Interesse ist insbesondere die Erzeugung von hochamylosehaltigen Stärken. Ferner kann eine in Pflanzenzellen enthaltene modifizierte Stärke das Verhalten der Pflanzenzelle unter bestimmten Bedingungen vorteilhaft verändern. Denkbar ist beispielsweise eine Verringerung des Stärkeabbaus während der Lagerung von Stärke-enthaltenden Organen, wie z.B. Samen oder Knollen, vor deren weiterer Verarbeitung, z.B. zur Extraktion der Stärke. Ferner ist es von Interesse, modifizierte Stärken herzustellen, die dazu führen, daß Pflanzenzellen oder pflanzliche Organe, die diese Stärke enthalten, besser zur Weiterverarbeitung geeignet sind, beispielsweise bei der Herstellung von "Popcorn" oder "Corn flakes" aus Mais oder von Pommes frites, Chips oder Kartoffelpulver aus Kartoffeln. Von besonderem Interesse ist hierbei die Verbesserung der Stärken in der Hinsicht, daß sie ein reduziertes "cold sweetening" aufweisen, d.h. eine verringerte Freisetzung von reduzierenden Zuckern (insbesondere Glucose) bei einer längeren Lagerung bei niedrigen Temperaturen. Gerade Kartoffeln werden häufig bei Temperaturen von 4-8 °C gelagert, um den Stärkeabbau während der Lagerung zu minimieren. Die hierbei freigesetzten reduzierenden Zucker, insbesondere Glucose, führen beispielsweise bei der Herstellung von Pommes frites oder Chips zu unerwünschten Bräunungsreaktionen (sogenannte Maillard-Reaktionen).
Die Anpassung der aus Pflanzen isolierbaren Stärke an bestimmte industrielle Verwendungszwecke erfolgt häufig mit Hilfe chemischer Modifikationen, die in der Regel zeit- und kostenintensiv sind. Es erscheint daher wünschenswert, Möglichkeiten zu finden, Pflanzen herzustellen, die eine Stärke synthetisieren, die in ihren Eigenschaften bereits den Anforderungen der verarbeitenden Industrie entspricht.
Herkömmliche Wege zur Herstellung derartiger Pflanzen bestehen in klassischen Züchtungsverfahren und der Erzeugung von Mutanten. So wurde beispielsweise bei Mais eine Mutante erzeugt, die eine Stärke mit veränderten Viskositätseigenschaften synthetisiert (US Patentschrift 5,331,108), sowie eine Maissorte (*waxy maize*), durch Züchtung etabliert, deren Stärke zu nahezu 100% aus Amylopektin besteht (Akasuka und Nelson, J. Biol. Chem. 241 (1966), 2280-2285). Ferner sind bei Mais und Erbse Mutanten beschrieben worden, die Stärken mit hohem Amylosegehalt synthetisieren (70% in Mais bzw. bis zu 50% in Erbse). Diese Mutanten sind bisher nicht auf molekularer Ebene charakterisiert worden und erlauben somit auch nicht die Erzeugung entsprechender Mutanten in anderen stärkespeichernden Pflanzen.
Alternativ können Pflanzen, die eine Stärke mit veränderten Eigenschaften synthetisieren, mit Hilfe gentechnischer Verfahren erzeugt werden. Beschrieben wurde beispielsweise in mehreren Fällen die gentechnische Veränderung von Kartoffelpflanzen, mit dem Ziel der Veränderung der in den Pflanzen synthetisierten Stärke (z.B. WO 92/11376; WO 92/14827). Voraussetzung für die Anwendung gentechnischer Verfahren ist jedoch die Verfügbarkeit von DNA-Sequenzen, deren Genprodukte einen Einfluß auf die Stärkesynthese, die Stärkemodifikation oder den Stärkeabbau haben.

Der vorliegenden Erfindung liegt daher die Aufgabe zugrunde, Nucleinsäuremoleküle und Verfahren zur Verfügung zu stellen, die es ermöglichen, Pflanzen dahingehend zu verändern, daß sie eine Stärke synthetisieren, die sich hinsichtlich ihrer physikalischen und/oder chemischen Eigenschaften von natürlicherweise in den Pflanzen synthetisierter Stärke unterscheidet, insbesondere eine hochamylosehaltige Stärke, und die somit für allgemeine und/oder spezielle Verwendungszwekke besser geeignet ist.

Diese Aufgabe wird durch die Bereitstellung der in den Patentansprüchen bezeichneten Ausführungsformen gelöst.

Die vorliegende Erfindung beschreibt Nucleinsäuremoleküle, die ein Protein mit der unter Seq ID No. 2 angegebenen Aminosäuresequenz codieren. Derartige Proteine liegen in den Plastiden pflanzlicher Zellen sowohl an Stärkekörnern gebunden vor, als auch außerhalb von Stärkekörnern in freier, d.h. löslicher Form. Die Enzymaktivität derartiger Proteine führt bei Expression in E. coli zu einer erhöhten Phosphorylierung des in den Zellen synthetisierten Glycogens. Das Molekulargewicht dieser Proteine liegt im Bereich von 140-160 kd, wenn es mit Hilfe einer SDS-Gelelektrophorese bestimmt wird.

Ferner beschreibtdie vorliegende Erfindung Nucleinsäuremoleküle, die eine Sequenz mit der unter Seq ID No. 1 angegebenen Nucleotidabfolge, insbesondere die in Seq ID No. 1 angegebenen codierenden Region, umfassen.

Ebenfalls beschrieben werden Nucleinsäuremoleküle, die ein Protein codieren, das in den Plastiden pflanzlicher Zellen zum Teil an Stärkekörnern gebunden vorliegt, und die mit den oben genannten erfindungsgemäßen Nucleinsäuremolekülen hybridisieren. Der Begriff "Hybridisierung" bedeutet in diesem Zusammenhang eine Hybridisierung unter konventionellen Hybridisierungsbedingungen, vorzugsweise unter stringenten Bedingungen, wie sie beispielsweise in Sambrook et al. (1989, Molecular Cloning, A Laboratory Manual, 2. Aufl. Cold Spring Harbor Laboratory Press, Cold Spring Harbor, NY) beschrieben sind. Diese Nucleinsäuremoleküle, die mit den oben beschriebenen Nucleinsäuremolekülen hybridisieren, können prinzipiell aus jedem beliebigen Organismus (d.h. Prokaryonten oder Eukaryonten, insbesondere aus Bakterien, Pilzen, Algen, Pflanzen oder tierischen Organismen) stammen, der derartige Nucleinsäuremoleküle besitzt. Sie stammen vorzugsweise aus monokotylen oder dikotylen Pflanzen, insbesondere aus Nutzpflanzen, und besonders bevorzugt aus Stärkespeichernder Pflanzen.
Nucleinsäuremoleküle, die mit den oben beschriebenen Molekülen hybridisieren, können z.B. aus genomischen oder aus cDNA-Bibliotheken verschiedener Organismen isoliert werden. Die Identifizierung und Isolierung derartiger Nucleinsäuremoleküle aus Pflanzen oder anderen Organismen kann dabei unter Verwendung der oben beschriebenen Nucleinsäuremoleküle oder Teile dieser Moleküle bzw. der reversen Komplemente dieser Moleküle erfolgen, z.B. mittels Hybridisierung nach Standardverfahren (siehe z.B. Sambrook et al., 1989, Molecular Cloning, A Laboratory Manual, 2. Aufl. Cold Spring Harbor Laboratory Press, Cold Spring Harbor, NY).
Als Hybridisierungsprobe können z.B. Nucleinsäuremoleküle verwendet werden, die exakt die oder im wesentlichen die unter Seq ID No. 1 angegebene Sequenz oder Teile dieser Sequenz aufweisen. Bei den als Hybridisierungsprobe verwendeten DNA-Fragmenten kann es sich auch um synthetische DNA-Fragmente handeln, die mit Hilfe der gängigen DNA-Synthesetechniken hergestellt wurden und deren Sequenz im wesentlichen mit der der oben beschriebenen Nucleinsäuremolekeule übereinstimmt. Hat man Gene identifiziert und isoliert, die mit den oben beschriebenen Sequenzen hybridisieren, ist eine Bestimmung der Sequenz und eine Analyse der Eigenschaften der von dieser Sequenz codierten Proteine erforderlich.

Weiterhin beschreibt die vorliegende Erfindung Nucleinsäuremoleküle, deren Sequenzen aufgrund des genetischen Codes degeneriert sind im Vergleich zu den Sequenzen der obengenannten Moleküle, und die ein Protein codieren, das in den Plastiden pflanzlicher Zellen teilweise an Stärkekörner gebunden vorliegt.

Beschrieben werden ebenfalls Fragmente, Derivate und allelische Varianten der oben beschriebenen Nucleinsäuremoleküle, die das oben beschriebene Protein codieren. Unter Fragmenten werden dabei Teile der Nucleinsäuremoleküle verstanden, die lang genug sind, um das beschriebene Protein zu codieren. Der Ausdruck Derivat bedeutet in diesem Zusammenhang, daß die Sequenzen dieser Moleküle sich von den Sequenzen der oben beschriebenen Nucleinsäuremoleküle an einer oder mehreren Positionen unterscheiden und einen hohen Grad an Homologie zu den Sequenzen dieser Moleküle aufweisen. Homologie bedeutet dabei eine Sequenzidentität von mindestens 40%, insbesondere eine Identität von mindestens 60%, vorzugsweise über 80% und besonders bevorzugt über 90%. Die Abweichungen zu den oben beschriebenen Nucleinsäuremolekülen können dabei durch Deletion, Substitution, Insertion oder Rekombination entstanden sein.
Homologie bedeutet ferner, daß funktionelle und/oder strukturelle Äquivalenz zwischen den betreffenden Nucleinsäuremolekülen oder den durch sie codierten Proteinen, besteht. Bei den Nucleinsäuremolekülen, die homolog zu den oben beschriebenen Nucleinsäuremolekülen sind und Derivate dieser Moleküle darstellen, handelt es sich in der Regel um Variationen dieser Nucleinsäuremoleküle, die Modifikationen darstellen, die dieselbe biologische Funktion ausüben. Es kann sich dabei sowohl um natürlicherweise auftretende Variationen handeln, beispielsweise um Sequenzen aus anderen Organismen, oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln.
Bei den allelischen Varianten kann es sich sowohl um natürlich auftretende Varianten handeln, als auch um synthetisch hergestellte oder durch rekombinante DNA-Techniken erzeugte Varianten.
Die von den verschiedenen Varianten der erfindungsgemäßen Nucleinsäuremoleküle codierten Proteine weisen bestimmte gemeinsame Charakteristika auf. Dazu können z.B. Enzymaktivität, Molekulargewicht, immunologische Reaktivität, Konformation etc. gehören, sowie physikalische Eigenschaften wie z.B. das Laufverhalten in Gelelektrophoresen, chromatographisches Verhalten, Sedimentationskoeffizienten, Löslichkeit, spektroskopische Eigenschaften, Stabilität, pH-Optimum, Temperatur-Optimum etc.

Die beschriebenen Nucleinsäuremoleküle können prinzipiell aus jedem Organismus stammen, der die beschriebenen Proteine exprimiert, vorzugsweise aus Pflanzen, insbesondere aus stärkesynthetisierenden bzw. stärkespeichernden Pflanzen. Besonders bevorzugt sind dabei z.B. Getreidearten (wie Gerste, Roggen, Hafer, Weizen etc.), Mais, Reis, Erbse, Maniok, Kartoffel usw. Ferner können sie durch dem Fachmann geläufige Synthesetechniken hergestellt werden.

Bei den beschriebenen Nucleinsäuremolekülen kann es sich sowohl um DNA-, beispielsweise um cDNA oder genomische DNA, als auch um RNA-Moleküle handeln.

Ferner 1 werden Vektoren, insbesondere Plasmide, Cösmide, Viren, Bacteriophagen und andere in der Gentechnik gängige Vektoren beschrieben, die die oben beschriebenen Nucleinsäuremoleküle enthalten.

Bevorzugt sind die in den Vektoren enthaltenen Nucleinsäuremoleküle verknüpft mit regulatorischen Elementen, die die Transkription in prokaryontischen oder eukaryontischen Zellen gewährleisten.

Auch beschrieben werden Wirtszellen, insbesondere prokaryontische oder eukaryontische Zellen, die mit einem oben beschriebenen Nucleinsäuremolekül oder Vektor transformiert und/oder genetisch manipuliert sind, sowie Zellen, die von solchen Zellen abstammen und ein beschriebenes Nucleinsäuremolekül oder einen Vektor enthalten. Dabei handelt es sich vorzugsweise um bakterielle Zellen oder um Pflanzenzellen.

Es wurde nun gefunden, daß das durch die beschriebenen Nucleinsäuremoleküle codierte Protein einen Einfluß auf die Stärkesynthese bzw. -modifikation hat, und eine Veränderung der Menge des Proteins in pflanzlichen Zellen zu Veränderungen im Stärkemetabolismus der Pflanzen führt, insbesondere zur Synthese von Stärken mit veränderten physikalischen und chemischen Eigenschaften.
Durch die Bereitstellung der beschriebenen Nucleinsäuremoleküle ist es somit möglich, mit Hilfe gentechnischer Verfahren Pflanzen herzustellen, die eine modifizierte Stärke synthetisieren, die sich in ihrer Struktur und ihren physikalischen und chemischen Eigenschaften von in Wildtyp-Pflanzen synthetisierter Stärke unterscheidet. Hierzu werden die beschriebenen Nucleinsäuremoleküle mit regulatorischen Elementen verknüpft, die die Transkription und Translation in Pflanzenzellen gewährleisten, und in pflanzliche Zellen eingebracht.
Somit werden auch transgene Pflanzenzellen, die ein oben beschriebenes Nucleinsäuremolekül enthalten, wobei dieses mit regulatorischen Elementen verknüpft ist, die die Transkription in pflanzlichen Zellen gewährleisten. Die regulatorischen Elemente sind vorzugsweise heterolog in Bezug auf das Nucleinsäuremolekül. Die transgenen Pflanzenzellen können nach dem Fachmann bekannten Techniken zu ganzen Pflanzen regeneriert werden. Die durch Regeneration der beschriebenen transgenen Pflanzenzellen erhältlichen Pflanzen enthalten die obenbeschriebenen transgenen Pflanzenzellen enthalten. Bei den transgenen Pflanzen kann es sich prinzipiell um Pflanzen jeder beliebigen Pflanzenspezies handeln, d.h. sowohl monokotyle als auch dikotyle Pflanzen. Bevorzugt handelt es sich um Nutzpflanzen, insbesondere stärkespeichernde Nutzpflanzen, wie z.B. Getreidearten (Roggen, Gerste, Hafer, Weizen etc.), Reis, Mais, Erbse, Maniok und Kartoffel.

Die beschriebenen transgenen Pflanzenzellen und Pflanzen synthetisieren aufgrund der Expression bzw. zusätzlichen Expression eines erfindungsgemäßen Nucleinsäuremoleküls eine Stärke, die im Vergleich zu Stärke aus Wildtyp-Pflanzen, d.h. nicht-transformierten Pflanzen, modifiziert ist, insbesondere im Hinblick auf die Viskosität wäßriger Lösungen dieser Stärke und/oder den Phosphatgehalt. Dieser ist in der Regel bei der Stärke aus transgenen Pflanzenzellen bzw. Pflanzen erhöht, wodurch die physikalische Eigenschaften der Stärke verändert werden.

Beschrieben wird auch die aus den erfindungsgemäßen transgenen Pflanzenzellen und Pflanzen erhältliche Stärke.

Es wird ferner ein Verfahren beschrieben zur Herstellung eines Proteins, das in pflanzlichen Zellen sowohl an Stärkekörner gebunden als auch in löslicher Form vorliegt, bei dem oben beschriebene Wirtszellen unter Bedingungen kultiviert werden, die die Expression des Proteins erlauben, und das Protein aus den Zellen und/oder dem Kulturmedium isoliert wird.

Beschrieben werden auch die durch die beschriebenen Nucleinsäuremoleküle codierten Proteine sowie Proteine, die durch das oben beschriebene Verfahren erhältlich sind. Es handelt sich dabei vorzugsweise um pflanzliche, kerncodierte Proteine, die in den Plastiden lokalisiert sind. In den Plastiden liegen diese Enzyme sowohl an den Stärkekörnern gebunden vor als auch frei. Die entsprechenden Proteine aus *Solanum tuberosum* weisen in einer SDS-Gelelektrophorese ein Molekulargewicht von 140-160 kd auf und führen bei Expression in E. coli zu einer erhöhten Phosphorylierung des in den Zellen synthetisierten Glycogens.

Es wurde gefunden, daß es möglich ist, die Eigenschaften der in Pflanzenzellen synthetisierten Stärke dadurch zu beeinflussen, daß die Menge an Proteinen, die durch die beschriebenen Nucleinsäuremoleküle codiert werden, in den Zellen verringert wird. Diese Verringerung kann beispielsweise durch antisense-Expression der erfindungsgemäßen Nucleinsäuremoleküle, durch Expression geeigneter Ribozyme oder mittels Cosuppression erfolgen.

Die vorliegende Erfindung offenbart daher auch DNA-Moleküle, die eine antisense-RNA codieren, die komplementär ist zu Transkripten eines oben beschriebenen DNA-Moleküls. Komplementär bedeutet dabei, daß die codierte RNA nicht 100 % komplementär sein muß, sondern auch ein geringerer Grad an Komplementarität ausreicht, solange sie genügend hoch ist, um bei Expression in pflanzlichen Zellen die Expression eines, oben beschriebenen Proteins zu inhibieren. Die transkribierte RNA ist vorzugsweise zumindest 90 % und besonders bevorzugt mindestens 95 % komplementär zu einem Transkript eines oben beschriebenen Nucleinsäuremoleküls. Um bei Transkription in pflanzlichen Zellen einen antisense-Effekt zu bewirken, sind derartige DNA-Moleküle mindestens 15 bp lang, vorzugsweise länger als 100 bp und besonders bevorzugt länger als 500 bp, jedoch in der Regel kürzer als 5000 bp, vorzugsweise kürzer als 2500 bp.

Ferner offenbart die Anmeldung auch DNA-Moleküle, die bei Expresssion in pflanzlichen Zellen zur Synthese einer RNA führen, die in den Pflanzenzellen aufgrund eines Cosuppressions-Effektes eine Verringerung der Expression oben beschriebener Nucleinsäuremoleküle hervorruft, die das beschriebene Protein codieren. Das Prinzip der Cosuppression sowie die Herstellung entsprechender DNA-Sequenzen ist beispielsweise ausführlich beschrieben in der WO 90/12084. Derartige DNA-Moleküle codieren vorzugsweise eine RNA, die einen hohen Grad an Homologie zu Transkripten der oben beschriebenen Nucleinsäuremoleküle hat. Es ist dabei allerdings nicht unbedingt erforderlich, daß die codierte RNA in ein Protein translatierbar ist.

In einem weiteren Aspekt offenbart die vorliegende Erfindung DNA-Moleküle, die ein RNA-Molekül mit Ribozymaktivität codieren, das spezifisch Transkripte eines oben beschriebenen DNA-Moleküls spaltet.
Ribozyme sind katalytisch aktive RNA-Moleküle, die in der Lage sind, RNA-Moleküle und spezifische Zielsequenzen zu spalten. Mit Hilfe gentechnologischer Methoden ist es möglich, die Spezifität von Ribozymen zu verändern. Es existieren verschiedene Klassen von Ribozymen. Für die praktische Anwendung mit dem Ziel, das Transkript eines bestimmten Gens gezielt zu spalten, werden bevorzugt Vertreter zweier verschiedener Gruppen von Ribozymen verwendet. Die eine Gruppe wird gebildet von Ribozymen, die dem Typ der GruppeI-Intron-Ribozymen zuzuordnen sind. Die zweite Gruppe wird von Ribozymen gebildet, die als charakteristisches Strukturmerkmal ein sogenanntes "hammerhead"-Motiv aufweisen. Die spezifische Erkennung des Ziel-RNA-Moleküls kann modifiziert werden durch Änderung der Sequenzen, die dieses Motiv flankieren Diese Sequenzen bestimmen über Basenpaarung mit Sequenzen im Zielmolekül die Stelle, an der die katalytische Reaktion und somit die Spaltung des Zielmoleküls erfolgt. Da die Sequenzanforderungen für eine effiziente Spaltung äußerst gering sind, ist es im Prinzip möglich, spezifische Ribozyme für praktisch jedes beliebige RNA-Molekül zu entwickeln.
Um DNA-Moleküle herzustellen, die ein Ribozym codieren, das spezifisch Transkripte eines oben beschriebenen DNA-Moleküls spaltet, wird beispielsweise eine DNA-Sequenz, die eine katalytische Domäne eines Ribozyms codiert, beiderseits mit DNA-Sequenzen verknüpft, die homolog sind zu Sequenzen des Zielenzyms. Als Sequenzen, die die katalytische Domänen codieren, kommen beispielsweise in Frage die katalytische Domäne der Satelliten-DNA des SCMo-Virus (Davies et al., Virology 177 (1990), 216-224) oder die der Satelliten-DNA des TobR-Virus (Steinecke et al., EMBO J. 11 (1992), 1525-1530; Haseloff und Gerlach, Nature 334 (1988), 585-591). Die die katalytische Domäne flankierenden DNA-Sequenzen stammen vorzugsweise aus den oben beschriebenen DNA-Molekülen.

In einem weiteren Aspekt offenbart die vorliegende Erfindung Vektoren, die die oben beschriebenen DNA-Moleküle enthalten, insbesondere solche, bei denen die beschriebenen DNA-Moleküle verknüpft sind mit regulatorischen Elementen, die die Transkription in pflanzlichen Zellen gewährleisten.

Ferner werden auch Wirtszelle beschrieben, die die beschriebenen DNA-Moleküle oder Vektoren enthalten. Die Wirtszelle kann eine prokaryontische, beispielsweise bakterielle, oder eukaryontische Zelle sein. Bei den eukaryontischen Wirtszellen handelt es sich vorzugsweise um pflanzliche Zellen.

Ferner offenbart die Erfindung transgene Pflanzenzellen, die ein oben beschriebenes DNA-Molekül enthalten, das eine anti-sense-RNA, ein Ribozym oder eine RNA, die zu einem Cosuppressions-Effekt führt, codiert, wobei dieses DNA-Molekül verknüpft ist mit DNA-Elementen, die die Transkription in pflanzlichen Zellen gewährleisten. Diese transgenen Pflanzenzellen können nach gängigen Techniken zu ganzen Pflanzen regeneriert werden. Die Erfindung offenbart somit auch Pflanzen, die erhältlich sind durch Regeneration aus den beschriebenen transgenen Pflanzenzellen, sowie Pflanzen, die die beschriebenen transgenen Pflanzenzellen enthalten. Bei den transgenen Pflanzen kann es sich wiederum um Pflanzen jeder beliebigen Pflanzenspezies handeln, vorzugsweise um Nutzpflanzen, insbesondere stärkespeichernde, wie oben angegeben.

Durch die Expression der beschriebenen DNA-Moleküle, die antisense-RNA, ein Ribozym oder eine "Cosuppressions-RNA" codieren, in den transgenen Pflanzenzellen kommt es zu einer Verringerung der Menge an Proteinen, die durch oben beschriebenen DNA-Moleküle codiert werden, die endogen in den Zellen vorliegen. Diese Verringerung hat überraschenderweise eine drastische Veränderung der physikalischen und chemischen Eigenschaften der in den Pflanzenzellen synthetisierten Stärke zur Folge, insbesondere der Viskositätseigenschaften wäßriger Lösungen dieser Stärke, des Phosphatgehaltes als auch der Freisetzung reduzierender Zucker bei Lagerung der Pflanzenzellen oder Pflanzenteile bei niedrigen Temperaturen. Die Eigenschaften der in den transgenen Pflanzenzellen synthetisierten Stärke wird weiter unten ausführlich beschrieben.

Somit wird auch die aus den beschriebenen transgenen Pflanzenzellen und Pflanzen erhältlichen Stärke beschrieben.

Ferner offenbart die Erfindung die durch die beschriebenen DNA-Moleküle codierten antisense-RNA-Moleküle, sowie RNA-Moleküle mit Ribozymaktivität und RNA-Moleküle, die einen Cosuppressions-Effekt hervorrufen, die durch Transkription erhältlich sind.

In einem weiteren Aspekt wird ein Verfahren zur Herstellung transgener Pflanzenzellen, die im Vergleich zu nicht-transformierten Zellen eine modifizierte Stärke synthetisieren, beschrieben, bei dem in den Pflanzenzellen die Menge an Proteinen verringert wird, die durch oben beschriebene DNA-Moleküle codiert werden, die endogen in den Zellen vorliegen.

In einer Ausführungsform erfolgt diese Verringerung mit Hilfe eines antisense-Effektes. Hierzu werden findungsgemäße DNA-Moleküle oder Teile davon in antisenseoben beschriebene mit einem Promotor verknüpft, der die Transkription in pflanzlichen Zellen gewährleistet, sowie gegebenenfalls mit einem Terminationssignal, das die Termination der Transkription sowie die Polyadenylierung des Transkriptes gewährleistet. Um einen effizienten antisense-Effekt in den pflanzlichen Zellen zu gewährleisten, sollte die synthetisierte antisense-RNA eine Mindestlänge von 15 Nukleotiden, vorzugsweise von mindestens 100 Nukleotiden und besonders bevorzugt von über 500 Nukleotiden aufweisen. Ferner sollte die die antisense-RNA codierende DNA-Sequenz in bezug auf die zu transformierende Pflanzenspezies homolog sein. Es können jedoch auch DNA-Sequenzen verwendet werden, die einen hohen Grad an Homologie zu endogen in den Zellen vorhandenen DNA-Sequenzen aufweisen, vorzugsweise eine Homologie von mehr als 90%, besonders bevorzugt von mehr als 95%.

In einer weiteren Ausführungsform erfolgt die Verringerung der Menge an Proteinen, die durch die oben beschriebenen DNA-Moleküle codiert werden, durch einen Ribozym-Effekt. Die prinzipielle Wirkungsweise von Ribozymen, sowie die Konstruktion von DNA-Molekülen, die derartige RNA-Moleküle codieren, wurden bereits oben beschrieben. Um in transgenen Zellen eine RNA mit Ribozymaktivität zu exprimieren, werden die oben beschriebenen DNA-Moleküle, die für ein Ribozym codieren, mit DNA-Elementen verknüpft, die die Transkription in pflanzlichen Zellen gewährleisten, insbesondere mit einem Promotor und einem Terminationssignal. Die in den Pflanzenzellen synthetisierten Ribozyme führen zur Spaltung von Transkripten von oben beschriebenen DNA-Molekülen, die endogen in den Zellen vorliegen.

Eine weitere Möglichkeit der Verringerung der Menge an Proteinen, die durch die oben beschriebenen Nucleinsäuremoleküle codiert werden, ist die Cosuppression.

Die durch das beschriebene Verfahren erhältlichen Pflanzenzellen, sind dadurch charakterisiert daß bei ihnen die Menge an Proteinen verringert ist, die durch die oben beschriebenen DNA-Moleküle codiert werden, und im Vergleich zu Wildtyp-Zellen eine modifizierte Stärke synthetisieren.

Ferner beschreibt die Erfindung Pflanzen, die erhältlich sind durch Regeneration der beschriebenen Pflanzenzellen, sowie Pflanzen, die die beschriebenen Zellen enthalten.

Die aus den beschriebenen Pflanzenzellen und Pflanzen erhältliche Stärke weist im Vergleich zu Stärke aus Wildtyp-Pflanzen veränderte physikalische und chemische Eigenschaften auf. Beispielsweise besitzt diese Stärke im Vergleich zur Stärke aus Wildtyp-Pflanzen einen reduzierten Phosphatgehalt. Ferner zeigen wäßrige Lösungen dieser Stärke veränderte Viskositätseigenschaften.

In einer bevorzugten Ausführungsform ist der Phosphatgehalt der beschriebenen Stärke um mindestens 50%, vorzugsweise um mindestens 75% und besonders bevorzugt um mehr als 80% im Vergleich zu Stärke aus Wildtyp-Pflanzen verringert.

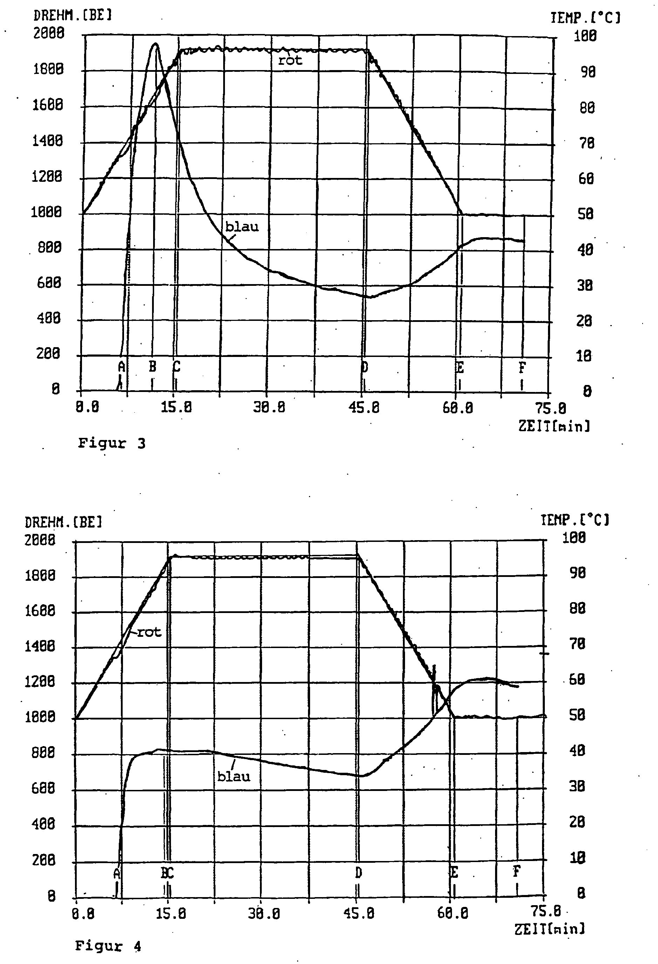

Der besondere Vorzug der beschriebenen Stärke liegt in den veränderten Viskositätseigenschaften wäßriger Lösungen dieser Stärke.
Ein gängiger Test, der verwendet wird, um die Viskositätseigenschaften zu bestimmen, ist der sogenannte Brabender-Test. Dieser Test wird durchgeführt unter der Verwendung eines Apparates, der beispielsweise als Viskograph E bekannt ist. Hergestellt und vertrieben wird dieses Instrument unter anderem von der Firma Brabender OHG Duisburg (Deutschland).
Der Test besteht im wesentlichen darin, daß Stärke in Gegenwart von Wasser zunächst erhitzt wird, um zu bestimmen, wann die Hydratisierung und das Schwellen der Stärkekörner einsetzt. Dieser Vorgang, der auch als Gelatinisierung bzw. Verkleisterung bezeichnet wird, beruht auf der Auflösung von Wasserstoffbrückenbindungen und geht einher mit einer meßbaren Viskositätszunahme der Stärkesuspension. Während eine weitere Erhitzung nach der Gelatinisierung zur vollständigen Auflösung der Stärkepartikel und einer Abnahme der Viskosität führt, kommt es bei einer Abkühlung unmittelbar nach der Gelatinisierung typischerweise zu einer Viskositätszunahme (siehe Fig. 3). Das Resultat eines Brabendertests ist eine Kurve, die die Viskosität in Abhängigkeit von der Zeit angibt, wobei zunächst eine Temperaturzunahme bis über die Gelatinisierungstemperatur und anschließend eine Abkühlung erfolgt.
Die Analyse einer Brabender-Kurve zielt in der Regel ab auf die Bestimmung der Verkleisterungsstemperatur, der maximalen Viskosität bei Erhitzen, der Viskositätszunahme bei Abkühlung, sowie der Viskosität nach dem Erkalten. Diese Parameter sind wichtige Charakteristika, die die Qualität einer Stärke sowie ihre Verwendbarkeit für verschiedene Anwendungen bestimmen.

Die Stärke, die sich beispielsweise aus Kartoffelpflanzen isolieren läßt, bei denen durch einen *antisense*-Effekt die Menge an oben beschriebenen Proteinen in den Zellen reduziert wurde, zeigt Charakteristika, die stark von denen abweichen, die Stärke zeigt, die aus Wildtyppflanzen isolierbar ist. Im Vergleich zu diesen zeigt sie nur eine geringe Viskositätszunahme beim Erhitzen, eine geringere maximale Viskosität, sowie eine stärkere Viskositätszunahme beim Erkalten (siehe Fig. 3, 4 und 5).

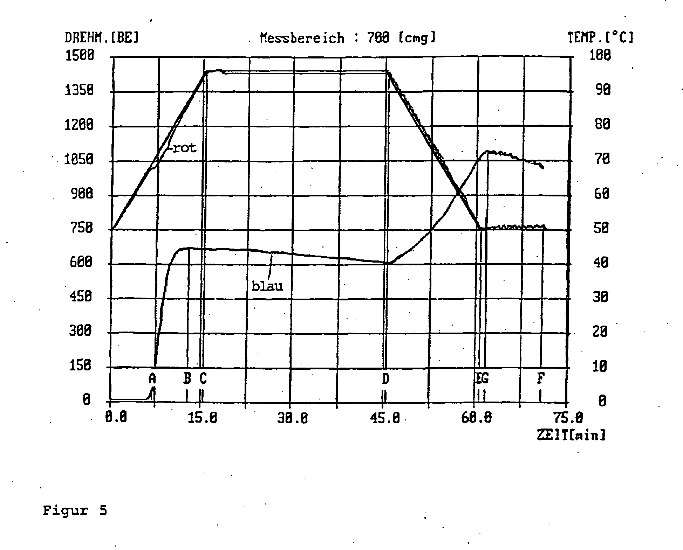

Es wird beispielsweise eine Stärke offenbart, deren wässrige Lösungen die in Fig. 4 oder 5 dargestellten charakteristischen Viskositätseigenschaften besitzen. Die modifizierte Stärke weist, insbesondere unter den in Beispiel 8 a genannten Bedingungen zur Bestimmung der Viskosität mit Hilfe eines Brabender-Viskosimeters, das Charakteristikum auf, daß während des Aufkochens im Vergleich zu Wildtypstärke nur eine geringe Viskositätszunahme erfolgt. Dies bietet die Möglichkeit, die erfindungsgemäße Stärke zur Herstellung höher konzentrierter Kleister zu verwenden.
Ferner weist die modifizierte Stärke die Eigenschaft auf, daß es nach Erreichen der maximalen Vikosität nur zu einer geringen Viskositätsabnahme kommt. Dagegen kommt es bei einem Erkalten zu einer starken Viskositätszunahme, so daß die Viskosität höher ist als die einer Stärke aus Wildtyp-Pflanzen.
Weiterhin ist es möglich, durch Verringerung der Menge an oben beschriebenen Proteinen in transgenen Pflanzenzellen eine Stärke herzustellen, die bei Lagerung von Pflanzenteilen, die diese Stärke enthalten, bei niedrigen Temperaturen, insbesondere bei 4-8 °C, zu einer veringerten Freisetzung reduzierender Zucker führt im Vergleich zu Stärke aus nicht-transformierten Zellen. Diese Eigenschaft ist beispielsweise besonders vorteilhaft für die Bereitstellung von Kartoffeln, die bei Lagerung bei niedrigen Temperaturen eine veringerte Freisetzung von reduzierenden Zuckern aufweisen, d.h. ein verringertes "cold sweetening". Derartige Kartoffeln sind besonders gut geeignet zur Herstellung von Pommes frites, Chips oder ähnlichem, da bei ihrer Verwendung unerwünschte Bräunungsreaktionen (Maillard-Reaktionen) ausbleiben oder zumindestens stark verringert sind.

Gemäß einem weiteren Aspekt wird in den transformierten Pflanzenzellen nicht nur die Synthese eines oben beschriebenen Proteins reduziert, sondern darüber hinaus auch die Synthese mindestens eines weiteren, an der Särkesynthese und/oder Modifikation beteitligten Enzyms. Bevorzugt sind dabei beispielsweise stärkekorngebundene Stärkesynthasen oder Verzweigungsenzyme. Es wurde überraschend gefunden, daß Kartoffelpflanzen, bei denen die Synthese des erfindungsgemäßen Proteins sowie auch des Verzweigungsenzyms aufgrund eines *antisense*-Effekts reduziert ist, eine Stärke synthetisieren, die in ihren Eigenschaften stark abweicht von Stärke aus Wildtyppflanzen.

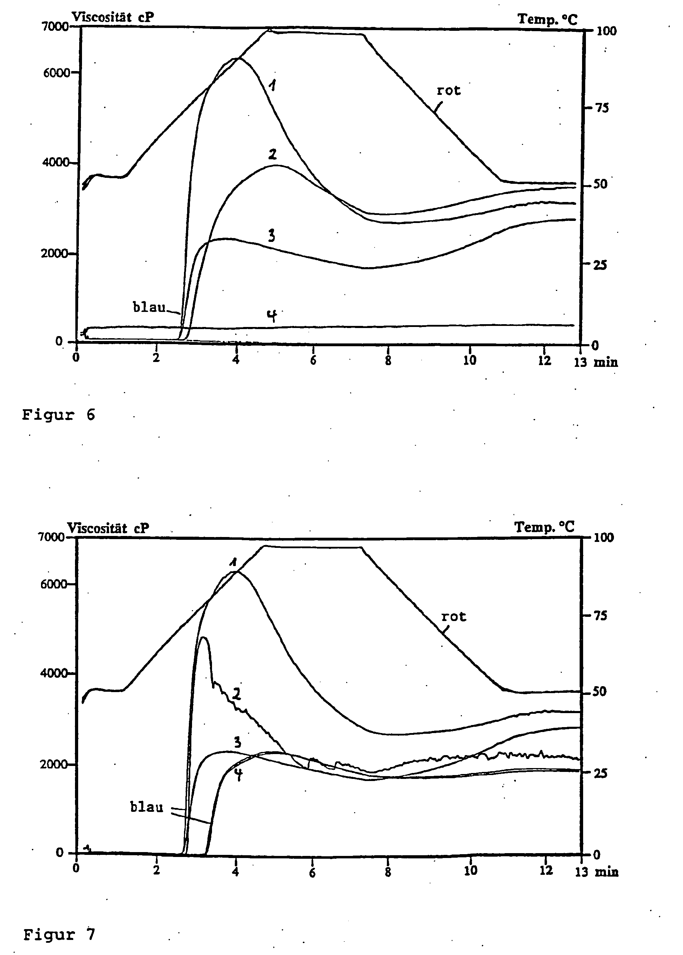

Im Vergleich zu Wildtypstärke zeigen wäßrige Lösungen dieser modifizierten Stärke so gut wie keine. Viskositätszunahme beim Erhitzen oder beim Abkühlen (vgl. Fig. 6). Des weiteren zeigt eine mikroskopische Analyse der Stärkekörner vor und nach dem Erhitzen deutlich, daß im Gegensatz zur Wildtypstärke die Stärkekörner aus derart veränderten Pflanzen nicht aufgeschlossen sind, sondern ihre ursprüngliche Struktur annähernd beibehalten. Es handelt sich somit um eine gegenüber dem Kochprozeß resistente Stärke. Wird von dieser Stärke der Amylosegehalt bestimmt mittels der in dem Beispielsteil beschriebenen Methodik, so ergeben sich Amylosegehalte von über 50 %, vorzugsweise über 60 % und besonders bevorzugt über 70 % für diese Stärke. Die wäßrigen Lösungen der aus diesen Pflanzen isolierbaren Stärke zeigen vorzugsweise die in Fig. 6 dargestellten charakteristischen Viskositätseigenschaften.

Eine derartige beschriebene hochamylosehaltige Stärke weist gegenüber Wildtypstärke eine Reihe von Vorteilen für verschiedene Verwendungen auf. So besitzen hochamylosehaltige Stärken ein hohes Potential zur Nutzung in Folien und Filmen. Die auf der Grundlage von hochamylosehaltigen Stärken erzeugten Folien und Filme, die in weitesten Bereichen der Verpackungsindustrie eingesetzt werden können, besitzen den deutlichen Vorteil, daß sie biodegradierbar sind. Neben dieser Anwendung, die im wesentlichen von in klassischer Weise auf der Erdölchemie basierenden Polymeren abgedeckt wird, besitzt die Amylose noch weitere unikate Anwendungsfelder, die durch die Eigenschaft der Amylose bedingt sind, eine Helix zu bilden. Die von der Amylose gebildete Helix ist im Inneren hydrophob und außen hydrophil. Aufgrund dessen kann Amylose zur Komplexierung und molekularen Verkapselung niedermolekularer oder auch höher molekularer Substanzen eingesetzt werden. Beispiele dafür sind:
- die molekulare Verkapselung von Vitaminen und Wirkstoffen mit dem Ziel des Schutzes vor Oxydation, Verflüchtigung, thermischem Abbau oder aber der Überführung in ein wäßriges Milieu;
- die molekulare Verkapselung von Aromastoffen zur Erhöhung der Löslichkeit;
- die molekulare Verkapselung von Düngemitteln/Pestiziden zur Stabilisierung und kontrollierten Freisetzung;
- die molekulare Verkapselung von Arzneistoffen zur Stabilisierung der Dosierbarkeit und kontrollierten Freisetzung von Retardpräparaten.

Eine weitere wichtige Eigenschaft von Amylose ist die Tatsache, daß es sich um ein chirales Molekül handelt. Aufgrund der Chiralität kann es präferentiell nach Immobilisierung beispielsweise an einer Säule zur Trennung von Enantiomeren eingesetzt werden.

Weiterhin wurde überraschend gefunden, daß Stärke, die sich aus Kartoffelpflanzen isolieren läßt, bei denen durch einen antisense-Effekt die Menge an oben beschriebenen Proteinen in den Zellen reduziert wird, in Kombination mit einer Reduktion der Proteine, die die enzymatische Aktivität einer stärkekorngebundenen Stärkesynthase der Isoform I (GBSSI) aufweisen, Charakteristika zeigt, die stark von denen abweichen, die Stärke zeigt, die aus Wildtyppflanzen isolierbar ist. Im Vergleich zu Stärke aus Wildtyppflanzen zeigen wäßrige Lösungen dieser Stärke nur eine geringe Viskositätszunahme beim Erhitzen, eine geringere maximale Viskosität sowie so gut wie keine Viskositätszunahme beim Erkalten (vgl. Fig. 7). Wenn von dieser Stärke das Verhältnis Amylose zu Amylopektin bestimmt wird, zeichnet sich diese Stärke dadurch aus, daß fast keine Amylose mehr nachweisbar ist. Der Amylosegehalt dieser Stärke liegt vorzugsweise unter 5%, besonders bevorzugt unter 2 %. Die beschriebene Stärke unterscheidet sich ferner von der bekannten Stärke, die durch Inhibierung des GBSSI-Gens alleine mittels gentechnischer Verfahren in transgenen Kartoffelpflanzen erzeugt werden kann. So weist diese Stärke eine starke Viskositätszunahme beim Erhitzen auf. Die wäßrigen Lösungen der beschriebenen Stärke zeigen vorzugsweise die in Fig. 7 dargestellten charakteristischen Viskositätseigenschaften. Insbesondere unter den im Beispiel 13 genannten Bedingungen zur Bestimmung der Viskosität mit Hilfe eines Rapid Visco Analysers weist die modifizierte Stärke das Charakteristikum auf, daß während des Aufkochens im Vergleich zur Wildtypstärke, aber auch im Vergleich zur *waxy-* Stärke nur eine geringe Viskositätszunahme erfolgt. Dies bietet die Möglichkeit, die beschriebene Stärke zur Herstellung höher konzentrierter Kleister zu verwenden. Ferner weist die modifizierte Stärke die Eigenschaft auf, daß es nach Erreichen der maximalen Viskosität nur zu einer geringeren Viskositätsabnahme kommt sowie zu so gut wie keiner Viskositätszunahme beim Erkalten.

Möglichkeiten zur Verringerung der Aktivität eines Verzweigungsenzyms in pflanzlichen Zellen wurden bereits beschrieben, beispielsweise in der WO 92/14827 und der WO 95/26407. Die Verringerung der Aktivität einer stärkekorngebundenen Stärkesynthase der Isoform I (GBSSI) kann unter der Verwendung von dem Fachmann bekannten Methoden erfolgen, beispielsweise mittels eines *antisense*-Effekts. DNA-Sequenzen, die eine GBSSI aus Kartoffel codieren, sind beispielsweise bekannt aus Hergersberg (Dissertation (1988) Universität Köln, Visser et al. (Plant Sci. 64 (1989), 185-192) oder van der Leiy et al. (Mol. Gen. Genet. 228 (1991), 240-248).

Das erfindungsgemäße Verfahren kann prinzipiell auf alle Pflanzenspezies angewendet werden. Von Interesse sind sowohl monokotyle als auch dikotyle Pflanzen, insbesondere Nutzpflanzen und hierbei bevorzugt stärkespeichernde Pflanzen, wie z.B. Getreidepflanzen (Roggen, Gerste, Hafer, Weizen, etc.), Reis, Mais, Erbse, Maniok und Kartoffel.
Unter dem Begriff "regulatorische DNA-Elemente, die die Transkription in pflanzlichen Zellen gewährleisten" werden im Rahmen der vorliegenden Erfindung DNA-Abschnitte verstanden, die die Initiation bzw. die Termination der Transkription in pflanzlichen Zellen ermöglichen. Zu den DNA-Abschnitten, die die Initiation der Transkription gewährleisten zählen insbesondere Promotoren. Für die Expression der verschiedenen oben beschriebenen

DNA-Moleküle in Pflanzen kommt im Prinzip jeder in pflanzlichen Zellen funktionale Promotor in Beitracht. Der Promotor kann homolog oder heterolog in bezug auf die verwendete Pflanzenspezies sein. Geeignet ist beispielsweise der 35S-Promotor des Cauliflower-Mosaik-Virus (Odell et al., Nature 313 (1985), 810-812), der eine konstitutive Expression in allen Geweben einer Pflanze gewährleistet und das in der WO/9401571 beschriebene Promotorkonstrukt. Es können jedoch auch Promotoren verwendet werden, die nur zu einem durch äußere Einflüsse determinierten Zeitpunkt (siehe beispielsweise WO/9307279) oder in einem bestimmten Gewebe der Pflanze zu einer Expression nachfolgender Sequenzen führen (siehe z. B. Stockhaus et al., EMBO J. 8 (1989), 2245-2251). Präferentiell werden Promotoren eingesetzt, die in den stärkespeichernden Organen der zu transformierenden Pflanzen aktiv sind. Dies sind beim Mais die Maiskörner, während es bei der Kartoffel die Knollen sind. Zur Transformation der Kartoffel kann insbesondere, aber nicht ausschließlich, der knollenspezifische B33-Promotor (Rocha-Sosa et al., EMBO J. 8 (1989), 23-29) verwendet werden.
Neben Promotoren können DNA-Abschnitte zur Initiation der Transkription auch DNA-Sequenzen enthalten, die eine weitere Steigerung der Transkription gewährleisten, beispielsweise sogenannte Enhancer-Elemente.
Ferner kann der Begriff "regulatorische DNA-Elemente" auch Terminationssignale umfassen, die der korrekten Beendigung der Transkription sowie der Addition eines Poly-A-Schwanzes an das Transkript dienen, dem eine Funktion bei der Stabilisierung der Transkripte beigemessen wird. Derartige Elemente sind in der Literatur beschrieben und sind beliebig austauschbar. Beispiele für derartige Terminationssequenzen sind die 3'-nichttranslatierten Regionen, die das Polyadenylierungssignal des Nopalin-Synthase-Gens (NOS-Gen) oder des Octopinsynthase-Gens (Gielen et al., EMBO J. 8 (1989), 23-29) aus Agrobakterien umfassen, oder die 3'-nichttranslatierten Regionen der Gene der Speicherproteine aus Sojabohne, sowie die der Gene der kleinen Untereinheit der Ribulose-1,5-Bisphosphat-Carboxylase (ssRUBISCO).

Die Einführung der oben beschriebenen DNA-Moleküle in pflanzliche Zellen erfolgt vorzugsweise unter Verwendung von Plasmiden. Vorzugsweise werden dafür Plasmide verwendet, die eine stabile Integration der eingeführten DNA in das pflanzliche Genom gewährleisten.
In den Beispielen der vorliegenden Erfindung wird der binäre Vektor pBinAR (Höfgen und Willmitzer, Plant Sci. 66 (1990), 221-230) verwendet. Bei diesem Vektor handelt es sich um ein Derivat des binären Vektors pBin19 (Bevan, Nucl. Acids Res. 12 (1984), 8711-8721), der kommerziell erhältlich ist (Clontech Laboratories, Inc., USA).
Es ist jedoch auch jeder andere Pflanzentransformationsvektor geeignet, in den eine Expressionskassette inseriert werden kann, und der die Integration der Expressionskassette in das pflanzliche Genom gewährleistet.
Zur Vorbereitung der Einführung fremder Gene in höhere Pflanzen stehen eine große Anzahl von Klonierungsvektoren zur Verfügung, die ein Replikationssignal für *E.coli* und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann an einer passenden Restriktionsschnittstelle in den Vektor eingeführt werden. Das erhaltene Plasmid wird für die Transformation von *E.coli*-Zellen verwendet. Transformierte *E.coli*-Zellen werden in einem geeigneten Medium kultiviert, anschließend geerntet und lysiert. Das Plasmid wird nach Standardmethoden wiedergewonnen. Als Analysemethode zur Charakterisierung der gewonnenen Plasmid-DNA werden im allgemeinen Restriktionsanalysen und Sequenzanalysen eingesetzt. Nach jeder Manipulation kann die Plasmid DNA gespalten werden und resultierende DNA-Fragmente mit anderen DNA-Sequenzen verknüpft werden.
Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung von DNA mittels der biolistischen Methode sowie weitere Möglichkeiten.
Bei der Injektion und Elektroporation von DNA in Pflanzenzellen werden an sich keine speziellen Anforderungen an die verwendeten Plasmide gestellt. Es können einfache Plasmide wie z.B. pUC-Derivate verwendet werden. Sollen aber aus derartig transformierten Zellen ganze Pflanzen regeneriert werden, ist die Anwesenheit eines selektierbaren Markergens notwendig.
Je nach Einführungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muß mindestens die rechte Begrenzung, häufig jedoch die rechte und linke Begrenzung der Ti- und Ri-Plasmid T-DNA als Flankenbereich mit den einzuführenden Genen verbunden werden.
Werden für die Transformation Agrobakterien verwendet, muß die einzuführende DNA in spezielle Plasmide kloniert werden, und zwar entweder in einen intermediären Vektor oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige *vir-*Region. Intermediäre Vektoren können nicht in Agrobakterien replizieren. Mittels eines Helferplasmids kann der intermediäre Vektor auf *Agrobacterium tumefaciens* übertragen werden (Konjugation). Binäre Vektoren können sowohl in *E.coli* als auch in Agrobakterien replizieren. Sie enthalten ein Selektionsmarker-Gen und einen *Linker* oder *Polylinker,* welche von der rechten und linken T-DNA Grenzregion eingerahmt werden. Sie können direkt in die Agrobakterien transformiert werden (Holsters et al., Mol. Gen. Genet. 163 (1978), 181-187). Die zur Transformation der Agrobakterien verwendeten Plasmide enthalten weiterhin ein Selektionsmarkergen, beispielsweise das NPT II-Gen, das die Selektion transformierter Bakterein erlaubt. Das als Wirtszelle dienende Agrobakterium soll ein Plasmid, das eine *vir*-Region trägt, enthalten. Die *vir-*Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet.
Die Verwendung von T-DNA für die Transformation von Pflanzenzellen ist intensiv untersucht und ausreichend in EP 120516; Hoekema, In: The Binary Plant Vector System Offsetdrukkerij Kanters B.V., Alblasserdam (1985), Chapter V; Fraley et al., Crit. Rev. Plant. Sci., 4: 1-46 und An et al., EMBO J. 4 (1985), 277-287 beschrieben worden. Binäre Vektoren sind bereits z.T. kommerziell erhältlich, z.B. pBIN19 (Clontech Laboratories, Inc., USA).
Für den Transfer der DNA in die Pflanzenzelle können Pflanzen-Explantate zweckmäßigerweise mit *Agrobacterium* tumefaciens oder *Agrobacterium rhizogenes* kokultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden. Die so erhaltenen Pflanzen können dann auf Anwesenheit der eingeführten DNA untersucht werden.
Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin oder Phosphinotricin u.a. vermittelt. Der individuelle gewählte Marker sollte daher die Selektion transformierter Zellen gegenüber Zellen, denen die eingeführte DNA fehlt, gestatten.
Die transformierten Zellen wachsen innerhalb der Pflanze in der üblichen Weise (siehe auch McCormick et al., Plant Cell Reports 5 (1986), 81-84). Die resultierenden Pflanzen können normal angezogen werden und mit Pflanzen, die die gleiche transformierte Erbanlage oder andere Erbanlagen besitzen, gekreuzt werden. Die daraus entstehenden hybriden Individuen haben die entsprechenden phänotypischen Eigenschaften.
Es sollten zwei oder mehrere Generationen angezogen werden, um sicherzustellen, daß das phänotypische Merkmal stabil beibehalten und vererbt wird. Auch sollten Samen geerntet werden, um sicherzustellen, daß der entsprechende Phänotyp oder andere Eigenarten erhalten geblieben sind.

Die aus den beschriebenen Pflanzenzellen bzw. Pflanzen erhältliche Stärke eignet sich aufgrund ihrer Eigenschaften neben den bereits oben angesprochenen speziellen Verwendungszwecken für verschiedene industrielle Verwendungen.
Grundsätzlich läßt sich Stärke in zwei große Kategorien unterteilen, die Hydrolyseprodukte der Stärke und die sogenannten nativen Stärken. Zu den Hydrolyseprodukten zählen im wesentliche die über enzymatische oder chemische Verfahren erhaltene Glucose sowie Glucosebausteine, die für weitere Prozesse, wie Fermentation, oder auch weitere chemische Modifikationen eingesetzt werden können. In diesem Bereich kann die Einfachheit und kostengünstige Ausführung eines Hydrolyseverfahrens, wie es gegenwärtig im wesentlichen enzymatisch unter Verwendung von Amyloglucosidase verläuft, von Bedeutung sein. Darunter läßt sich vorstellen, daß ein geringerer Einsatz von für die Hydrolyse eingesetzten Enzymen durch Veränderung der Struktur der Stärke, z.B. größere Oberfläche des Korns, leichtere Verdaulichkeit durch geringeren Verzweigungsgrad oder sterische, die Zugänglichkeit für die eingesetzten Enzyme limitierende Struktur, zu einer Kosteneinsparung führen kann.
Die Verwendungen der sogenannten nativen Stärken, die wegen ihrer polymeren Struktur eingesetzt werden, lassen sich in zwei große Bereiche unterteilen:

### (a) Verwendung im Nahrungsmittelbereich

Stärke ist ein klassischer Zusatzstoff für viele Nahrungsmittel, bei denen sie im wesentlichen die Funktion des Bindens von wäßrigen Zusatzstoffen übernimmt bzw. eine Erhöhung der Viskosität oder aber eine erhöhte Gelbildung hervorruft. Wichtige Eigenschaftsmerkmale sind das Fließ- und Sorptionsverhalten, die Quell- und Verkleisterungs-temperatur, die Viskosität und Dickungsleistung, die Löslichkeit der Stärke, die Transparenz und Kleisterstruktur, die Hitze-, Scher- und Säurestabilität, die Neigung zur Retrogradation, die Fähigkeit zur Filmbildung, die Gefrier/Taustabilität, die Verdaulichkeit sowie die Fähigkeit zur Komplexbildung mit z.B. anorganischen oder organischen Ionen.

### (b) Einsatz im Nicht-Nahrungsmittelbereich

Der andere große Einsatzbereich liegt bei der Verwendung der Stärke als Hilfsstoff bei unterschiedlichen Herstellungsprozessen bzw. als Zusatzstoff in technischen Produkten. Der wesentliche Einsatzbereich für die Verwendung von Stärke als Hilfsstoff ist zum einen die Papier- und Pappeindustrie. Stärke wird dabei in erster Linie zur Retardation (Zurückhaltung von Feststoffen), der Abbindung von Füllstoff- und Feinstoffteilchen, als Festigungsstoff und zur Entwässerung eingesetzt. Darüberhinaus werden die günstigen Eigenschaften der Stärke in bezug auf die Steifigkeit, die Härte, den Klang, den Griff, den Glanz, die Glätte, die Spaltfestigkeit sowie die Oberflächen ausgenutzt.
Innerhalb des Papierherstellungsprozesses sind vier Anwendungsbereiche, nämlich Oberfläche, Strich, Masse und Sprühen, zu unterscheiden.
Die Anforderungen an die Stärke in bezug auf die Oberflächenbehandlung sind im wesentlichen ein hoher Weißegrad, eine angepaßte Viskosität, eine hohe Viskositätsstabilität, eine gute Filmbildung sowie eine geringe Staubbildung. Bei der Verwendung im Strich ist der Feststoffgehalt, eine angepaßte Viskosität, ein hohes Bindevermögen sowie eine hohe Pigmentaffinität wichtig. Als Zusatz zur Masse ist eine rasche, gleichmäßige, verlustfreie Verteilung, eine hohe mechanische Stabilität und eine vollständige Zurückhaltung im Papierfließ von Bedeutung. Beim Einsatz der Stärke im Sprühbereich sind ebenfalls ein angepaßter Feststoffgehalt, hohe Viskosität sowie ein hohes Bindevermögen von Bedeutung.
Ein großer Einsatzbereich besteht beispielsweise in der Klebstoffindustrie, wo man die Einsatzmöglichkeiten in vier Teilbereiche gliedert: die Verwendung als reinem Stärkeleim, die Verwendung bei mit speziellen Chemikalien aufbereiteten Stärkeleimen, die Verwendung von Stärke als Zusatz zu synthetischen Harzen und Polymerdispersionen sowie die Verwendung von Stärken als Streckmittel für synthetische Klebstoffe. 90% der Klebstoffe auf Stärkebasis werden in den Bereichen Wellpappenherstellung, Herstellung von Papiersäcken, Beuteln oder Tüten, Herstellung von Verbundmaterialien für Papier und Aluminium, Herstellung von Kartonagen und Wiederbefeuchtungsleim für Briefumschläge, Briefmarken usw. eingesetzt.

Eine weitere mögliche Verwendung als Hilfsmittel und Zusatzstoff besteht bei der Herstellung von Textilien und Textilpflegemitteln. Innerhalb der Textilindustrie sind die folgenden vier Einsatzbereiche zu unterscheiden: Der Einsatz der Stärke als Schlichtmittel, d.h. als Hilfsstoff zur Glättung und Stärkung des Klettverhaltens zum Schutz gegen die beim Weben angreifenden Zugkräfte sowie zur Erhöhung der Abriebfestigkeit beim Weben, als Mittel zur Textilaufrüstung vor allem nach qualitätsverschlechternden Vorbehandlungen, wie Bleichen, Färben usw., als Verdickungsmittel bei der Herstellung von Farbpasten zur Verhinderung von Farbstoffdiffusionen sowie als Zusatz zu Kettungsmitteln für Nähgarne.
Ferner kann die Stärke als Zusatz bei Baustoffen verwendet werden. Ein Beispiel ist die Herstellung von Gipskartonplatten, bei der die im Gipsbrei vermischte Stärke mit dem Wasser verkleistert, an die Oberfläche der Gipsplatte diffundiert und dort den Karton an die Platte bindet. Weitere Einsatzbereiche sind die Beimischung zu Putz- und Mineralfasern. Bei Transportbeton kann die Stärke zur Verzögerung der Abbindung eingesetzt werden.
Ferner bietet sich die Stärke zur Herstellung von Mitteln zur Bodenstabilisation an, die bei künstlichen Erdbewegungen zum temporären Schutz der Bodenpartikel gegenüber Wasser eingesetzt werden. Kombinationsprodukte aus Stärke und Polymeremulsionen sind nach heutiger Kenntnis in ihrer Erosions- und verkrustungsmindernden Wirkung den bisher eingesetzten Produkten gleichzusetzen, liegen preislich aber deutlich unter diesen.
Ferner kann die Stärke in Pflanzenschutzmitteln zur Veränderung der spezifischen Eigenschaften der Präparate verwendet werden. So werden Stärken beispielsweise zur Verbesserung der Benetzung von Pflanzenschutz- und Düngemitteln, zur dosierten Freigabe der Wirkstoffe, zur Umwandlung flüssiger, flüchtiger und/oder übelriechender Wirkstoffe in mikrokristalline, stabile, formbare Substanzen, zur Mischung inkompatibler Verbindungen und zur Verlängerung der Wirkdauer durch Verminderung der Zersetzung eingesetzt.
Ein wichtiges Einsatzgebiet besteht ferner im Bereich der Pharmaka, Medizin und Kosmetikindustrie. In der pharmazeutischen Industrie kann die Stärke als Bindemittel für Tabletten oder zur Bindemittelverdünnung in Kapseln eingesetzt werden. Weiterhin eignet sich die Stärke als Tablettensprengmittel, da sie nach dem Schlucken Flüssigkeit absorbiert und nach kurzer Zeit so weit quillt, daß der Wirkstoff freigesetzt wird. Medizinische Gleit- und Wundpuder sind weitere Anwendungsmöglichkeiten. Im Bereich der Kosmetik kann die Stärke beispielsweise als Träger von Puderzusatzsoffen, wie Düften und Salicylsäure eingesetzt werden. Ein relativ großer Anwendungsbereich für die Stärke liegt bei Zahnpasta.
Auch die Verwendung der Stärke als Zusatzstoff zu Kohle und Briketts ist denkbar. Kohle kann mit einem Stärkezusatz quantitativ hochwertig agglomeriert bzw. brikettiert werden, wodurch ein frühzeitiges Zerfallen der Briketts verhindert wird. Der Stärkezusatz liegt bei Grillkohle zwischen 4 und 6%, bei kalorierter Kohle zwischen 0,1 und 0,5%. Desweiteren eignet sich die Stärke als Bindemittel, da durch ihren Zusatz zu Kohle und Brikett der Ausstoß schädlicher Stoffe deutlich vermindert werden kann.
Die Stärke kann ferner bei der Erz- und Kohleschlammaufbereitung als Flockungsmittel eingesetzt werden.
Ein weiterer Einsatzbereich besteht als Zusatz zu Grießereihilfsstoffen. Bei verschiedenen Gußverfahren werden Kerne benötigt, die aus Bindemittel-versetzten Sänden hergestellt werden. Als Bindemittel wird heute überwiegend Bentonit eingesetzt, das mit modifizierten Stärken, meist Quellstärken, versetzt ist. Zweck des Stärkezusatzes ist die Erhöhung der Fließfestigkeit sowie die Verbesserung der Bindefestigkeit. Darüberhinaus können die Quellstärken weitere produktionstechnische Anforderungen, wie im kalten Wasser dispergierbar, rehydratisierbar, gut in Sand mischbar und hohes Wasserbindungsvermögen, aufweisen.
In der Kautschukindustrie kann die Stärke zur Verbesserung der technischen und optischen Qualität eingesetzt werden. Gründe sind dabei die Verbesserung des Oberflächenglanzes, die Verbesserung des Griffs und des Aussehens. Dafür wird Stärke vor der Kaltvulkanisation auf die klebrigen gummierten Flächen von Kautschukstoffen gestreut. Ebenso kann sie für die Verbesserung der Bedruckbarkeit des Kautschuks eingesetzt werden.
Eine weitere Einsatzmöglichkeit der modifizierten Stärke besteht bei der Herstellung von Lederersatzstoffen.
Auf dem Kunststoffsektor zeichnen sich folgende Einsatzgebiete ab: die Einbindung von Stärkefolgeprodukten in den Verarbeitungsprozess (Stärke ist nur Füllstoff, es besteht keine direkte Bindung zwischen synthetischem Polymer und Stärke) oder alternativ die Einbindung von Stärkefolgeprodukten in die Herstellung von Polymeren (Stärke und Polymer gehen eine feste Bindung ein).
Die Verwendung der Stärke als reinem Füllstoff ist verglichen mit den andere Stoffen wie Talkum nicht wettbewerbsfähig. Anders sieht es aus, wenn die spezifischen Stärkeeigenschaften zum Tragen kommen und hierdurch das Eigenschaftsprofil der Endprodukte deutlich verändert wird. Ein Beispiel hierfür ist die Anwendung von Stärkeprodukten bei der Verarbeitung von Thermoplasten, wie Polyethylen. Hierbei werden die Stärke und das synthestische Polymer durch Koexpression im Verhältnis von 1 : 1 zu einem 'master batch' kombiniert, aus dem mit granuliertem Polyethylen unter Anwendung herkömmlicher Verfahrenstechniken diverse Produkte hergestellt werden. Durch die Einbindung der Stärke in Polyethylenfolien kann eine erhöhte Stoffdurchlässigkeit bei Hohlkörpern, eine verbesserte Wasserdampfdurchlässigkeit, ein verbessertes Antistatik-verhalten, ein verbessertes Antiblockverhalten sowie eine verbesserte Bedruckbarkeit mit wäßrigen Farben erreicht werden.
Eine andere Möglichkeit ist die Anwendung der Stärke in Polyurethanschäumen. Mit der Adaption der Stärkederivate sowie durch die verfahrenstechnische Optimierung ist es möglich, die Reaktion zwischen synthetischen Polymeren und den Hydroxygruppen der Stärke gezielt zu steuern Das Ergebnis sind Polyurethanfolien, die durch die Anwendung von Stärke folgende Eigenschaftsprofile erhalten: eine Verringerung des Wärmeausdehnungskoeffizienten, Verringerung des Schrumpfverhaltens, Verbesserung des Druck/Spannungsverhaltens, Zunahme der Wasserdampfdurchlässigkeit ohne Veränderung der Wasseraufnahme, Verringerung der Entflammbarkeit und der Aufrißdichte, kein Abtropfen brennbarer Teile, Halogenfreiheit und verminderte Alterung. Nachteile, die gegenwärtig noch vorhanden sind, sind verringerte Druckfestigkeit sowie eine verringerte Schlagfestigkeit.
Möglich ist nicht nur die Produktentwicklung von Folien. Auch feste Kunststoffprodukte, wie Töpfe, Platten und Schalen sind mit einem Stärkegehalt von über 50% herzustellen. Ferner weisen die Stärke/Polymermischungen den Vorteil auf, daß sie eine sehr viel höhere biologische Abbaubarkeit aufweisen.
Außerordentliche Bedeutung haben weiterhin aufgrund ihres extremen Wasserbindungsvermögens Stärkepfropfpolymerisate gewonnen. Dies sind Produkte mit einem Rückgrat aus Stärke und einer nach dem Prinzip des Radikalkettenmechanismus aufgepfropften Seitengitters eines synthetischen Monomers. Die heute verfügbaren Stärkepfropfenpolymerisate zeichnen sich durch ein besseres Binde- und Rückhaltevermögen von bis zu 1000 g Wasser pro g Stärke bei hoher Viskosität aus. Diese Superabsorber werden hauptsächlich im Hygienebereich verwendet, z.B. bei Produkten wie Windeln und Unterlagen sowie im landwirtschaftlichen Sektor, z.B. bei Saatgutpillierungen.

Entscheidend für den Einsatz der neuen gentechnischen veränderten Stärke sind zum einen die Struktur, Wassergehalt, Proteingehalt, Lipidgehalt,Fasergehalt, Asche/Phosphatgehalt, Amylose/Amylopektinverhältnis, Molmassenverteilung, Verzweigungsgrad, Korngröße und -form sowie Kristallisation, zum anderen auch die Eigenschaften, die in folgenden Merkmalen münden: Fließ- und Sorptionsverhalten, Verkleisterungstemperatur, Dickungsleistung, Löslichkeit, Kleisterstruktur, Transparenz, Hitze-, Scher- und Säurestabilität, Retrogradationsneigung, Gelbildung, Gefrier/Taustabilität, Komplexbildung, Jodbindung, Filmbildung, Klebekraft, Enzymstabilität, Verdaulichkeit und Reaktivität. Besonders hervorzuheben ist ferner die Viskosität.

Ferner kann die aus den beschriebenen Pflanzenzellen bzw. Pflanzen erhältliche modifizierte Stärke weiteren chemischen Modifikationen unterworfen werden, was zu weiteren Verbesserungen der Qualität für bestimmte der oben beschriebenen Einsatzgebiete führt oder zu neuen Einsatzgebieten. Diese chemischen Modifikationen sind dem Fachmann grundsätzlich bekannt. Insbesondere handelt es sich dabei um Modifikationen durch
- Säurebehandlung
- oxidation
- Veresterung (Enstehung von Phosphat-, Nitrat-, Sulfat-, Xanthat-, Acetat- und Citratstärken. Weitere organische Säuren können ebenfalls zur Veresterung eingesetzt werden.)
- Erzeugung von Stärkeethern (Stärke-Alkylether, O-Allylether, Hydroxylalkylether, O-Carboxylmethylether, N-haltige Stärkeether, S-haltige Stärkeether)
- Erzeugung von vernetzten Stärken
- Erzeugung von Stärke-Pfropf-Polymerisaten.

Folgende im Rahmen der vorliegenden Erfindung hergestellten und/oder verwendeten Plasmide wurden bei der als internationale Hinterlegungsstelle anerkannten Deutschen Sammlung von Mikroorganismen (DSM) in Braunschweig, Bundesrepublik Deutschland, entsprechend den Anforderungen des Budapester Vertrages für die internationale Anerkennung der Hinterlegung von Mikroorganismen zum Zwecke der Patentierung hinterlegt

**(Hinterlegungsnummer; Hinterlegungsdatum):**

| | | | |
|---|---|---|---|
| Plasmid | pBinAR Hyg | (DSM 9505) | (20.10.1994) |
| Plasmid | p33-anti-BE | (DSM 6146) | (20.08.1990) |
| Plasmid | pRL2 | (DSM 10225) | (04.09.1995) |

### Verwendete Medien und Lösungen

| | | | |
|---|---|---|---|
| Elutionspuffer | 25 | mM | Tris pH 8,3 |
| | 250 | mM | Glycin |
| | | | |
| Dialysepuffer | 50 | mM | Tris-HCl pH 7,0 |
| | 50 | mM | NaCl |
| | 2 | mM | EDTA |
| | 14,7 | mM | β-Mercaptoethanol |
| | 0,5 | mM | PMSF |
| | | | |
| Proteinpuffer | 50 | mM | Natriumphosphatpuffer pH 7,2 |
| | 10 | mM | EDTA |
| | 0,5 | mM | PMSF |
| | 14,7 | mM | β-Mercaptoethanol |
| | | | |
| Lugolsche Lösung | 12 | g KI | |
| | 6 | g I₂ | |
| | | ad 1,8 l mit ddH₂O | |
| 20 x SSC | 175.3 | g | NaCl |
| | 88.2 | g | Natrium-Citrat |
| | | ad 1000 ml mit ddH₂O | |
| | | pH 7,0 mit 10 N NaOH | |
| | | | |
| 10 x MEN | 200 | mM | MOPS |
| | 50 | mM | Natriumacetat |
| | 10 | mM | EDTA |
| | | pH 7, 0 | |
| | | | |
| NSEB-Puffer | 0,25 | M | Natriumphosphatpuffer pH 7,2 |
| | 7% | SDS | |
| | 1 | mM | EDTA |
| | 1% | BSA | (w/v) |

### Beschreibung der Abbildungen

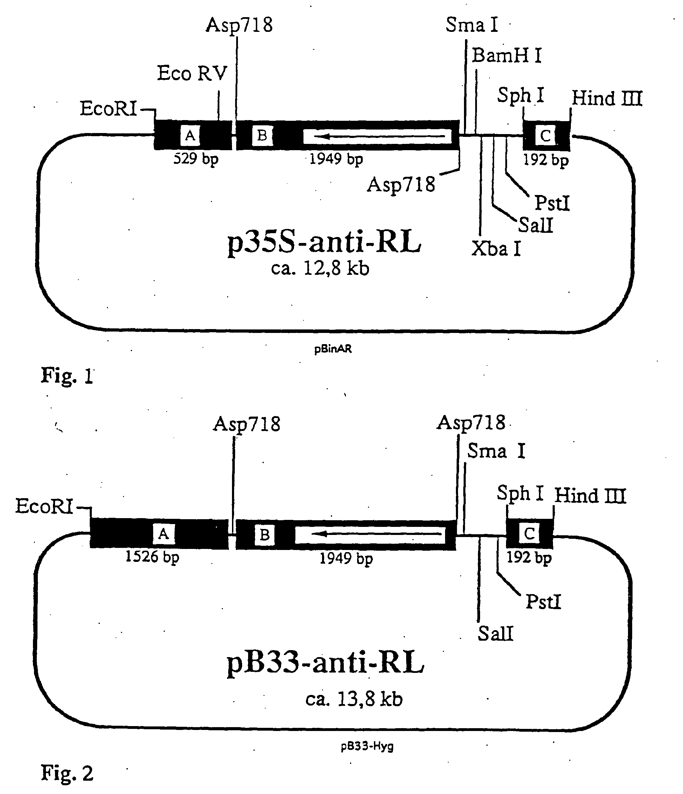

**Fig. 1** zeigt das Plasmid p35S-anti-RL.
Aufbau des Plasmids:
A = Fragment A: CaMV 35S-Promotor, nt 6909-7437 (Franck et al., Cell 21 (1980), 285-294)
B = Fragment B: ca. 1949 bp-langes *Asp*718-Fragment aus pRL1
   Orientierung zum Promotor: anti-sense
   Der Pfeil gibt die Richtung des offenen Leserasters an.
C = Fragment C: nt 11748-11939 der T-DNA des Ti-Plasmids pTiACH5 (Gielen et al., EMBO J. 3 (1984), 835-846)

**Fig. 2** zeigt das Plasmid pB33-anti-RL
Aufbau des Plasmids:
A = Fragment A: B33-Promotor des Patatin-Gens B33 aus *Solanum tuberosum* (Rocha-Sosa et al., EMBO J. 8 (1989), 23-29)
B = Fragment B: ca. 1949 bp-langes Asp718-Fragment aus pRL1
   Orientierung zum Promotor: *anti-sense*
   Der Pfeil gibt die Richtung des offenen Leserasters an.
C = Fragment C: nt 11748-11939 der T-DNA des Ti-Plasmids pTiACH5 (Gielen et al., EMBO J. 3 (1984), 835-846)

**Fig. 3** zeigt eine mit einem Brabender-Viskograph vom Typ Viskograph E aufgezeichnete Brabender-Kurve einer wäßrigen Lösung von Stärke, die aus nicht-transformierten Kartoffelpflanzen der Varietät Désirée isoliert wurde (siehe Beispiel 8).

| | | |
|---|---|---|
| Dabei bedeuten: | Drehm. | Drehmoment |
| | [BE] | Brabender-Einheiten |
| | Temp. | Temperatur |
| | A | Verkleisterungsbeginn |
| | B | Maximale Viskosität |
| | C | Start der Haltezeit |
| | D | Start der Kühlzeit |
| | E | Ende der Kühlzeit |
| | F | Ende der End-Haltezeit |

Die blaue Linie gibt die Viskosität an; die rote den Temperaturverlauf.
**Fig. 4** zeigt eine mit einem Brabender-Viskograph vom Typ Viskograph E aufgezeichnete Brabender-Kurve einer wäßrigen Lösung von Stärke, die aus Kartoffelpflanzen isoliert wurde, die mit dem Plasmid p35S-anti-RL transformiert worden waren (siehe Beispiel 8). Für die Bedeutung der Abkürzungen siehe Figur 3.
**Fig. 5** zeigt eine mit einem Brabender-Viskograph vom Typ Viskograph E aufgezeichnete Brabender-Kurve einer wäßrigen Lösung von Stärke aus Kartoffeln, die mit dem Plasmid pB33-anti-RL transformiert worden waren (siehe Beispiel- 8). Für die Bedeutung der Abkürzungen siehe Figur 3.
**Fig. 6** zeigt mit einem Rapid Visco Analyser aufgezeichnete Kurven wäßriger Stärkelösungen, die aus Kartoffelpflanzen isoliert wurden (siehe Beispiel 12). Die rote Linie gibt den Temperaturverlauf an, die blauen Linien 1, 2, 3 und 4 die Viskositäten folgender Stärkelösungen:

| | |
|---|---|
| Linie 1: | Stärke, die aus Wildtyppflanzen isoliert worden ist, |
| Linie 2: | Stärke, die aus Pflanzen isoliert worden ist, bei denen das Verzweigungsenzym alleine inhibiert wurde (vgl. Beispiel 1 der Patentanmeldung WO92/14827), |
| Linie 3: | Stärke, die aus Pflanzen isoliert worden ist, bei denen lediglich die erfindungsgemäßen Proteine in ihrer Konzentration verringert wurden (vgl. Beispiel 6). |
| Linie 4: | Stärke, die aus Pflanzen isoliert worden ist, die mit dem Plasmid p35S-anti-RL in Kombination mit dem Plasmid p35SH-anti-BE (vgl. Beispiel 12) transformiert worden sind. |

**Fig. 7** zeigt mit einem Rapid Visco Analyser aufgezeichnete Kurven wäßriger Stärkelösungen, die aus Kartoffelpflanzen isoliert wurden (siehe Beispiel 13). Die rote Linie gibt den Temperaturverlauf an, die blauen Linien 1, 2, 3 und 4 die Viskositäten folgender Stärkelösungen:

| | |
|---|---|
| Linie 1: | Stärke, die aus Wildtyppflanzen isoliert worden ist, |
| Linie 2: | Stärke, die aus Pflanzen isoliert worden ist, die allein mit dem Plasmid pB33-anti-GBSSI isoliert wurde (sog. waxy-Kartoffel), |
| Linie 3: | Stärke, die aus Pflanzen isoliert worden ist, die allein mit dem Plasmid p35S-anti-RL transformiert wurde (vgl. Beispiel 6), |
| Linie 4: | Stärke, die aus Pflanzen isoliert worden ist, die mit dem Plasmid pB33-anti-RL in Kombination mit dem Plasmid pB33-anti-GBSSI (vgl. Beispiel 13) transformiert worden sind. |

Die Beispiele erläutern die Erfindung.

In den Beispielen wurden folgende Standardtechniken angewendet:

### 1. Klonierungsverfahren

Zur Klonierung in *E.coli* wurde der Vektor pBluescriptSK verwendet.

Für die Pflanzentransformation wurden die Genkonstruktionen in den binären Vektor pBinAR (Höfgen und Willmitzer, Plant Sci. 66 (1990), 221-230) und B33-Hyg kloniert.

### 2. Bakterienstämme

Für den pBluescript-Vektor und für die pBinAR- und B33-Hyg-Konstrukte wurde der *E.coli*-Stamm DH5α (Bethesda Research Laboratories, Gaithersburgh, USA) verwendet.

Die Transformation der Plasmide in die Kartoffelpflanzen wurde mit Hilfe des *Agrobacterium tumefaciens*-stammes C58C1 pGV2260 durchgeführt (Deblaere et al., Nucl. Acids Res. 13 (1985), 4777:4788).

### 3. Transformation von Agrobacterium tumefaciens

Der Transfer der DNA erfolgte durch direkte Transformation nach der Methode von Höfgen & Willmitzer (Nucleic Acids Res. 16 (1988), 9877). Die Plasmid-DNA transformierter Agrobakterien wurde nach der Methode von Birnboim & Doly (Nucleic Acids Res. 7 (1979), 1513-1523) isoliert und nach geeigneter Restriktiorisspaltung gelelektrophoretisch analysiert.

### 4. Transformation von Kartoffeln

Zehn kleine mit dem Skalpell verwundete Blätter einer Kartoffel-Sterilkultur (Solanum tuberosum L.cv. Desiree) wurden in 10 ml MS-Medium (Murashige & Skoog, Physiol. Plant. 15 (1962), 473-497) mit 2% Saccharose gelegt, welches 50 µl einer unter Selektion gewachsenen *Agrobacterium tumefaciens*-Übernachtkultur enthielt. Nach 3-5 minütigem, leichtem Schütteln erfolgte eine weitere Inkubation für 2 Tage im Dunkeln. Daraufhin wurden die Blätter zur Kallusinduktion auf MS-Medium mit 1,6% Glucose, 5 mg/l Naphthylessigsäure, 0,2 mg/l Benzylaminopurin, 250 mg/l Claforan, 50 mg/l Kanamycin bzw. 1 mg/l Hygromycin B, und 0,80% Bacto Agar gelegt. Nach einwöchiger Inkubation bei 25°C und 3000 Lux wurden die Blätter zur Sproßinduktion auf MS-Medium mit 1,6% Glucose, 1,4 mg/l Zeatinribose, 20 mg/l Naphthylessigsäure, 20 mg/l Giberellinsäure, 250 mg/l Claforan, 50 mg/l Kanamycin bzw. 3 mg/l Hygromycin B, und 0,80% Bacto Agar gelegt.

### 5. Radioaktive Markierung von DNA-Fragmenten

Die radiokative Markierung von DNA-Fragmenten wurde mit Hilfe eines DNA-Random Primer Labelling Kits der Firma Boehringer (Deutschland) nach den Angaben des Herstellers durchgeführt.

### 6. Northern Blot-Analyse

RNA wurde nach Standardprotokollen aus Blattgewebe von Pflanzen isoliert. 50 µg der RNA wurden auf einem Agarosegel aufgetrennt (1,5% Agarose, 1 x MEN-Puffer, 16,6% Formaldehyd). Das Gel wurde nach dem Gellauf kurz in Wasser gewaschen. Die RNA wurde mit 20 x SSC mittels Kapillarblot auf eine Nylonmembran vom Typ Hybond N (Amersham, UK) transferiert. Die Membran wurde anschließend bei 80°C unter Vakuum für zwei Stunden gebacken. Die Membran wurde in NSEB-Puffer für 2 h bei 68°C prähybridisiert und anschließend in NSEB-Puffer über Nacht bei 68°C in Gegenwart der radioaktiv markierten Probe hybridisiert.

### 7. Pflanzenhaltung

Kartoffelpflanzen wurden im Gewächshaus unter folgenden Bedingungen gehalten:

| | |
|---|---|
| Lichtperiode | 16 h bei 25000 Lux und 22°C |
| Dunkelperiode | 8 h bei 15 °C |
| Luftfeuchte | 60% |

### 8. Bestimmung des Amylose/Amylopektinverhältnisses in Stärke aus Kartoffelpflanzen

Stärke wurde nach Standardmethoden aus Kartoffelpflanzen isoliert und das Verhältnis Amylose zu Amylopektin nach der von Hovenkamp-Hermelink et al. beschriebenen Methode (Potato Research 31 (1988) 241-246) bestimmt.

### 9. Bestimmung von Glucose, Fructose und Saccharose

Zur Bestimmung des Glucose-, Fructose- bzw. Saccharosegehalts werden kleine Knollenstücke (Durchmesser ca. 10 mm) von Kartoffelknollen in flüssigem Stickstoff eingefroren und anschließend für 30 min bei 80 °C in 0,5 ml 10 mM HEPES, pH 7,5; 80% (Vol./Vol.) Ethanol extrahiert. Der Überstand, der die löslichen Bestandteile enthält, wird abgenommen und das Volumen bestimmt. Der Überstand wird zur Bestimmung der Menge an löslichen Zuckern verwendet. Die quantitative Bestimmung von löslicher Glucose, Fructose und Saccharose wird in einem Ansatz mit folgender Zusammensetzung durchgeführt:

| | | |
|---|---|---|
| 100,0 | mM | Imidazol/HCl, pH 6,9 |
| 1,5 | mM | MgCl₂ |
| 0,5 | mM | NADP⁺ |
| 1,3 | mM | ATP |
| 10-50 | µl | Probe |
| 1,0 | U | Glucose-6-Phosphatdehydrogenase aus Hefe |

Der Ansatz wird für 5 min bei Raumtemperatur inkubiert. Die Bestimmung der Zucker erfolgt anschließend mittels gängiger photometrischer Methoden durch Messung der Absorption bei 340 nm nach der aufeinanderfolgenden Zugabe von

| | | |
|---|---|---|
| 1,0 | Einheiten | Hexokinase aus Hefe (zur Bestimmung von Glucose) |
| 1,0 | Einheiten | Phosphoglucoisomerase aus Hefe (zur Bestimmung von Fructose) und |
| 1,0 | Einheiten | Invertase aus Hefe (zur Bestimmung von Saccharose). |

### Beispiel 1

### Isolierung stärkekorn-gebundener Proteine aus Kartoffelstärke

Die Isolierung von Stärkekorn-gebundenen Proteinen aus Kartoffelstärke erfolgte durch Elektroelutioh in einer Elutionsvorrichtung, die analog zu dem "Model 422 Electro-Eluter" (BIORAD Laboratories Inc., USA) konstruiert war, aber ein wesentlich größeres Volumen aufwies (ca. 200 ml). Es wurden 25 g getrocknete Stärke in Elutionspuffer aufgenommen (Endvolumen 80 ml). Die Stärke stammte aus Kartoffeln, die aufgrund der anti-sense-Expression einer DNA-Sequenz, die für die Stärkekorn-gebundene Stärkesynthase I (GBSS I) aus Kartoffel kodiert, eine nahezu amylosefreie Stärke produzieren. Die Suspension wurde im Wasserbad auf 70-80°C erwärmt. Anschließend wurden 72,07 g Harnstoff zugegeben (Endkonzentration 8 M) und das Volumen mit Elutionspuffer auf 180 ml aufgefüllt. Die Stärke löste sich unter ständigem Rühren und bekam eine kleisterartige Konsistenz. Die Proteine wurden aus der Lösung mit Hilfe des Elutionsvorrichtung über Nacht elektroeluiert (100 V; 50-60 mA). Die eluierten Proteine wurden vorsichtig aus der Appartur entnommen. Schwebstoffe wurden durch kurze Zentrifugation entfernt. Der Überstand wurde 2-3 mal je eine Stunde bei 4°C gegen Dialysepuffer dialysiert. Anschließend wurde das Volumen der Proteinlösung bestimmt. Die Proteine wurden durch Zugabe von Ammoniumsulfat (90% Endkonzentration) gefällt. Die Zugabe erfolgte unter ständigem Rühren bei 0°C. Die gefällten Proteine wurden durch Zentrifugation pelletiert und in Proteinpuffer aufgenommen.

### Beispiel 2

### Identifizierung und Isolierung von cDNA-Sequenzen, die für Stärkekorn-qebundene Proteine kodieren

Die gemäß Beispiel 1 isolierten Proteine wurden zur Herstellung von polyclonalen Antikörpern aus Kaninchen verwendet, die spezifisch Stärkekorn-gebundene Proteine erkennen. Mit Hilfe derartiger Antikörper wurde anschließend nach Standardmethoden eine cDNA-Expressionsbibliothek nach Sequenzen durchgemustert, die für Stärkekorn-gebundene Proteine kodieren.
Die Expressionsbibliothek wurde folgendermaßen hergestellt: Aus Kartoffelknollen der Kartoffelvarietät "Berolina" wurde nach Standardmethoden poly(A⁺)-mRNA isoliert. Ausgehend von der poly(A⁺)-mRNA wurde nach der Methode von Gubler und Hoffmann (Gene 25 (1983), 263-269) unter Verwendung eines *Xho* I-Oligo d(t)₁₈-Primers cDNA hergestellt. Diese wurde nach *Eco*R I-Linkeraddition mit *Xho* I nachgeschnitten und orientiert in einen mit *Eco*R I und *Xho* I geschnittenen Lambda ZAP II-Vektor (Stratagene) ligiert. Ca. 500.000 Plaques einer derart konstruierten cDNA-Bibliothek wurden nach Sequenzen durchgemustert, die von polyclonalen Antikörpern, die gegen Stärkekorn-gebundene Proteine gerichtet sind, erkannt wurden.
Zur Analyse der Phagenplaques wurden diese auf Nitrozellulosefilter übertragen, die vorher für 30-60 min in einer 10 mM. IPTG-Lösung inkubiert und anschließend auf Filterpapier getrocknet wurden. Der Transfer erfolgte für 3 h bei 37°C. Anschließend werden die Filter für 30 min bei Raumtemperatur in Blockreagenz inkubiert und zweimal für 5-10 min in TBST-Puffer gewaschen. Die Filter wurden mit den gegen Stärkekorn-gebundene Proteine gerichteten polyclonalen Antikörpern in geeigneter Verdünnung für 1 h bei Raumtemperatur oder für 16 h bei 4°C geschüttelt. Die Identifizierung von Plaques, die ein Protein exprimierten, das von den polyclonalen Antikörpern erkannt wurde, erfolgte mit Hilfe des "Blotting detection kit for rabbit antibodies RPN 23" (Amersham UK) nach den Angaben des Herstellers.
Phagenclone der cDNA-Bibliothek, die ein Protein exprimierten, das von den polyclonalen Antikörpern erkannt wurde, wurden unter Anwendung von Standardverfahren weiter gereinigt.
Mit Hilfe der *in-vivo-excision*-Methode wurden von positiven Phagenclonen *E. coli*-Klone gewonnen, die ein doppelsträngiges pBluescript-Plasmid mit der jeweiligen cDNA-Insertion enthalten. Nach Überprüfung der Größe und des Restriktionsmusters der Insertionen wurde ein geeigneter Clon, pRL1, weiter analysiert.

### Beispiel 3

### Sequenzanalyse der cDNA-Insertion des Plasmids pRL1

Aus einem entsprechend Beispiel 2 erhaltenen *E. coli*-Clon wurde das Plasmid pRL1 isoliert und ein Teil der Sequenz seiner cDNA-Insertion durch Standardverfahren mittels der Didesoxynukleotidmethode (Sanger et al., Proc. Natl. Acad. Sci. USA 74 (1977), 5463-5467) bestimmt. Die Insertion ist ca. 2450 bp lang. Ein Teil der Nukleotidsequenz sowie die daraus abgeleitete Aminosäuresequenz ist unter Seq ID No. 3 und Seq ID No. 4 angegeben.
Eine Sequenzanalyse und ein Sequenzvergleich mit bekannten DNA-Sequenzen zeigte, daß die unter Seq ID No. 3 dargestellte Sequenz neu ist und keine signifikante Homologie zu bisher bekannten DNA-Sequenzen aufweist. Die Sequenzanalyse zeigte weiterhin, daß es sich bei der cDNA-Insertion nur um eine partielle cDNA handelt, bei der ein Teil der codierenden Region am 5'-Ende fehlt.

### Beispiel 4

### Identifizierung und Isolierung einer vollständigen cDNA, die für ein Stärkekorn-gebundenes Protein aus Solanum tuberosum codiert

Zur Isolierung einer, der partiellen cDNA-Insertion des Plasmids pRL1 entsprechenden, vollständigen cDNA, wurde eine weitere cDNA-Bibliothek hergestellt. Dabei handelte es sich um eine Schließzellen-spezifische cDNA-Bibliothek aus *Solanum tuberosum,* die folgendermaßen konstruiert wurde:
Zunächst wurden Epidermisfragmente von Blättern von Kartoffelpflanzen der Varietät Desirée im wesentlichen nach der Methode von Hedrich et al. (Plant Physiol. 89 (1989), 148) hergestellt indem ca. 60 Blätter von sechs Wochen alten, im Gewächshaus gehaltenen Kartoffelpflanzen geerntet wurden. Aus den Blättern wurde die Mittelrippe entfernt. Anschließend wurden die Blätter in einem großen "Waring blender" (Volumen 1 Liter) in gekühltem destilliertem H₂O viermal für je 15 Sekunden auf höchster Stufe zerkleinert. Die Suspension wurde durch ein Nylonsieb mit einer Porengröße von 220 µm (Nybolt, Zürich, Schweiz) filtriert und mehrmals mit kaltem destilliertem Wasser gewaschen. Die Suspension wurde wiederum durch ein 220 µm-Nylonsieb filtriert und ausgiebig mit kaltem destilliertem Wasser gewaschen. Der Rückstand (Epidermisfragmente) wurde in einen kleineren "Waring blender" (Volumen 250 ml) gegeben und in destilliertem Wasser und Eis viermal für je 15 Sekunden bei auf kleiner Stufe zerkleinert. Die Suspension wurde durch ein 220 µm-Nylonsieb filtriert und ausgiebig mit kaltem destilliertem Wasser gewaschen. Die Epidermisfragmente (Rückstand) wurden mikroskopisch hinsichtlich einer Kontamination durch Mesophyllzellen untersucht. Wenn dies der Fall war, wurde der Zerkleinerungsschritt im kleinen "Waring blender" wiederholt.
Der Aufschluß der Schließzellen der Epidermisfragmente erfolgte durch Zermörsern in flüssigem Stickstoff in einem gekühlten Mörser für ca. 2 h. Zur Überprüfung des Aufschlusses der Schließzellen wurden regelmäßig Proben genommen und mikroskopisch überprüft. Nach 2 h oder wenn eine genügend große Anzahl von Schließzellen aufgeschlossen wurde, wurde das entstandene Pulver in ein Reaktionsgefäß (Volumen 50 ml) überführt und in einem Volumen GTC-Puffer (Chirgwin et al., Biochem. 18 (1979), 5294-5299) aufgenommen. Die Suspension wurde zentrifugiert und der Überstand durch Miracloth (Calbiochem, La Jolla, Kalifornien) filtriert. Das Filtrat wurde wie in Glisin et al. (Biochemistry 13 (1974), 2633-2637) und Mornex et al. (J. Clin. Inves. 77 (1986), 1952-1961) für 16 h einer Ultrazentrifugation unterzogen. Nach der Zentrifugation wurde der RNA-Niederschlag in 250 µl GTC-Puffer aufgenommen. Die RNA wurde durch Zugabe von 0,05 Volumen 1 M Essigsäure und 0,7 Volumen Ethanol gefällt. Die RNA wurde abzentrifugiert und der Niederschlag mit 3 M Natriumazetat (pH 4,8) und 70% Ethanol gewaschen. Die RNA wurde kurz getrocknet und in DEPC-behandeltem Wasser gelöst. Aus der isolierten RNA wurde nach Standardverfahren poly A⁺-RNA isoliert. Ausgehend von der poly(A⁺)-mRNA wurde nach der Methode von Gubler und Hoffmann (Gene 25 (1983), 263-269) unter Verwendung eines *Xho* I-Oligo d(t)₁₈-Primers cDNA hergestellt. Diese wurde nach *Eco*R I-Linkeraddition mit *Xho* I nachgeschnitten und orientiert in einen mit *Eco*R I und *Xho* I geschnittenen Lambda ZAP II-Vektor (Stratagene, GmbH, Heidelberg, Deutschland) ligiert. Das Verpacken in Phagenköpfe erfolgte unter Verwendung des Gigapack II Gold kit's (Stratagene, GmbH, Heidelberg, Deutschland) nach den Angaben des Herstellers.
Aus einer derartigen cDNA-Bibliothek wurden nach Standardverfahren Phagenclone isoliert und gereinigt, die mit der cDNA-Insertion aus dem Plasmid pRL1 hybridisieren. Mit Hilfe der *in-vivo-excision*-Methode wurden von positiven Phagenclonen *E. coli*-Klone gewonnen, die ein doppelsträngiges pBluescript-Plasmid mit der jeweiligen cDNA-Insertion enthalten. Nach Überprüfung der Größe und des Restriktionsmusters der Insertionen wurden geeignete Klone einer Restriktionskartierung und einer Sequenzanalyse unterzogen. Aus einem geeigneten Clon wurde das Plasmid pRL2 (DSM 10225) isoliert, das eine vollständige cDNA enthält, die für ein Stärkekorn-gebundenes Protein aus Kartoffel codiert.

### Beispiel 5

### Sequenzanalyse der cDNA-Insertion des Plasmids pRL2

Die Nukleotidsequenz der cDNA-Insertion des Plasmids pRL2 wurde wie in Beispiel 3 beschrieben bestimmt. Die Insertion ist 4856 bp lang. Die Nukleotidsequenz sowie die daraus abgeleitete Aminosäuresequenz ist in Seq ID No. 1 bzw. Seq ID No. 2 angegeben. Das entsprechende Gen wird im folgenden RL-Gen genannt.

### Beispiel 6

### Konstruktion des Plasmids p35S-anti-RL und Einführung des Plasmids in das Genom von Kartoffelpflanzen

Aus dem Plasmid pRL1 wurde mit Hilfe der Restriktionsendonuklease *Asp*718 ein ca. 1800 bp langes DNA-Fragment isoliert. Dieses entspricht der unter Seq ID No. 3 dargestellten DNA-Sequenz und enthält einen Teil des offenen Leserahmens. Das Fragment wurde in den mit *Asp*718 geschnittenen binären Vektor pBinAR (Höfgen und Willmitzer, Plant Sci. 66 (1990), 221-230) ligiert. Bei diesem handelt es sich um ein Derivat des binären Vektors pBin19 (Bevan, Nucl. Acids Res. 12 (1984), 8711-8721). pBinAR wurde folgendermaßen konstruiert:
Ein 529 bp langes Fragment, das die Nukleotide 6909-7437 des 35S-Promotor des Cauliflowermosaic-Virus umfaßt (Franck et al., Cell 21 (1980), 285-294), wurde als *EcoR* I/*Kpn I-*Fragment aus dem Plasmid pDH51 (Pietrzak et al., Nucl. Acids Res. 14, 5857-5868) isoliert und zwischen die *Eco*R I- und die *Kpn* I-Schnittstellen des Polylinkers von pBin19 ligiert. Dabei entstand das Plasmid pBin19-A.
Aus dem Plasmid pAGV40 (Herrera-Estrella et al., Nature 303, 209-213) wurde mit Hilfe der Restriktionsendonukleasen *Pvu* II und *Hind* III ein 192 bp langes Fragment isoliert, das das Polyadenylierungssignal des Gens 3 der T-DNA des Ti-Plasmids pTiACH5 (Gielen et al., EMBO J. 3, 835-846) umfaßt (Nukleotide 11749-11939). Nach Addition von *Sph* I-Linkern an die *Pvu* I-Schnittstelle wurde das Fragment zwischen die *Sph* I- und *Hind* III-Schnittstellen pBin19-A ligiert. Dabei entstand pBinAR.
Mit Hilfe von Restriktions- und Sequenzanalysen wurden rekombinante Vektoren identifiziert, bei denen das DNA-Fragment derart in den Vektor inseriert ist, daß ein Teil der codierenden Region der cDNA-Insertion aus pRL1 in *anti-sense*-Orientierung mit dem 35S-Promotor verknüpft ist. Das resultierende Plasmid, p35S-anti-RL, ist in Fig. 1 dargestellt.
Durch die Insertion des cDNA-Fragmentes entsteht eine Expressionskassette, die folgendermaßen aus den Fragmenten A, B und C aufgebaut ist:
Das Fragment A (529 bp) enthält den 35S-Promotor des Cauliflower-Mosaik-Virus (CaMV). Das Fragment umfaßt die Nukleotide 6909 bis 7437 des CaMV (Franck et al., Cell 21 (1980), 285-294).
Das Fragment B enthält neben flankierenden Bereichen einen Teil der proteincodierenden Region der cDNA-Insertion aus dem Plasmid pRL1. Diese wurde wie oben beschrieben als *Asp*718-Fragment aus pRL1 isoliert und in *anti-sense-*Orientierung an den 35S-Promotor in pBinAR fusioniert.
Fragment C (192 bp) enthält das Polyadenylierungssignal des Gens 3 der T-DNA des Ti-Plasmids pTiACH5 (Gielen et al., EMBO J. 3 (1984), 835-846).
Die Größe des Plasmids p35S-anti-RL beträgt ca. 12,8 kb. Das Plasmid wurde mit Hilfe Agrobakterien-vermittelter Transformation in Kartoffelpflanzen transferiert wie oben beschrieben. Aus den transformierten Zellen wurden ganze Pflanzen regeneriert. Die transformierten Pflanzen wurden unter Gewächshausbedingungen kultiviert.
Die Überprüfung des Erfolges der genetischen Veränderung der Pflanzen erfolgte durch Analyse der Gesamt-RNA in einer Northern-Blot-Analyse bezüglich des Verschwindens der zu der cDNA komplementären Transkripte. Hierzu wurde Gesamt-RNA aus Blättern transformierter Pflanzen nach Standardmethoden isoliert, gelelektrophoretisch auf einem Agarosegel aufgetrennt, auf eine Nylonmembran transferiert und mit einer radioaktiv markierten Probe hybridisiert, die die unter Seq ID No. 1 dargestellte Sequenz oder einen Teil dieser Sequenz aufweist. In ca. 5-10% der transformierten Pflanzen fehlte in der Northern-Blot-Analyse die Bande, die das spezifische Transkript der unter Seq ID No. 1 dargestellten Sequenz darstellt. Diese Pflanzen wurden zur Analyse der Stärkequalität verwendet.

### Beispiel 7

### Konstruktion des Plasmids pB33-anti-RL und Einführung des Plasmids in das Genom von Kartoffelpflanzen

Aus dem Plasmid pRL1 wurde mit Hilfe der Restriktionsendonuklease *Asp*718 ein ca. 1800 bp langes DNA-Fragment isoliert, das einen Teil des offenen Leserahmens der cDNA-Insertion umfaßt, und in den mit *Asp*718 geschnittenen Vektor B33-Hyg ligiert. Dieser Vektor wurde folgendermaßen hergestellt: Aus dem Vektor pBinAR Hyg (DSM 9505) wurde mit Hilfe der Restriktionsendonukleasen *Eco*R I und *Asp*718 der 35S-Promotor entfernt. Aus dem Plasmid p33-anti-BE (DSM 6146) wurde mit Hilfe von *EcoR* I und *Asp*718 ein ca. 1526 bp langes Fragment, das den B33-Promotor umfaßt, isoliert und in den mit *Eco*R I und *Asp*718 geschnittenen Vektor pBinAR Hyg (DSM 9505) inseriert.
Durch die Insertion des cDNA-Fragmentes in die *Asp*718-Schnittstelle des Plasmids B33-Hyg entsteht eine Expressionskassette, die folgendermaßen aus den Fragmenten A, B und C aufgebaut ist (Fig. 4):
Das Fragment A enthält den B33-Promotor aus *Solanum tuberosum* (EP 3775 092; Rocha-Sosa et al., EMBO J. 8 (1989), 23-29)
Das Fragment B enthält neben flankierenden Bereichen einen Teil der proteincodierenden Region der cDNA-Insertion aus dem Plasmid pRL1. Diese wurde wie oben beschrieben als *Asp*718-Fragment aus pRL1 isoliert und in *anti-sense-*Orientierung an den B33-Promotor in B33-Hyg fusioniert.
Fragment C (192 bp) enthält das Polyadenylierungssignal des Gens 3 der T-DNA des Ti-Plasmids pTiACH5 (Gielen et al., EMBO J. 3 (1984), 835-846).
Die Größe des Plasmids pB33-anti-RL beträgt ca. 12,8 kb.
Das Plasmid wurde mit Hilfe Agrobakterien-vermittelter Transformation in Kartoffelpflanzen transferiert wie oben beschrieben. Aus den transformierten Zellen wurden ganze Pflanzen regeneriert. Die transformierten Pflanzen wurden unter Gewächshausbedingungen kultiviert.
Die Überprüfung des Erfolges der genetischen Veränderung der Pflanzen erfolgte durch Analyse der Gesamt-RNA in einer Northern-Blot-Analyse bezüglich des Verschwindens der zu der cDNA komplementären Transkripte. Hierzu wurde Gesamt-RNA aus Knollen transformierter Pflanzen nach Standardmethoden isoliert, gelelektrophoretisch auf einem Agarosegel aufgetrennt, auf eine Nylonmembran transferiert und mit einer radioaktiv markierten Probe hybridisiert, die die unter Seq ID No. 1 dargestellte Sequenz oder einen Teil dieser Sequenz aufweist. In ca. 5-10% der transformierten Pflanzen fehlte in der Northern-Blot-Analyse die Bande, die Transkripte darstellt, die mit der erfindungsgemäßen cDNA hybridisieren. Aus diesen Pflanzen wurde aus Knollen die Stärke isoliert und wie in Beispiel 8 beschrieben analysiert.

### Beispiel 8

### Analyse der transformierten Kartoffelpflanzen

Die gemäß Beispiel 6 und Beispiel 7 transformierten Kartoffelpflanzen wurden hinsichtlich der Eigenschaften der synthetisierten Stärke untersucht. Die Analysen wurden an verschiedenen Linien von Kartoffelpflanzen durchgeführt, die mit dem Plasmid p35S-anti-RL bzw. mit dem Plasmid pB33-anti-RL transformiert worden waren und die in der Northern-Blot-Analyse die Bande nicht mehr aufwiesen, die Transkripte darstellt, die mit den erfindungsgemäßen DNA-Sequenzen hybridisieren.

### a) Bestimmung der Viskosität wäßriger Lösungen der Stärke

Zur Bestimmung der Viskosität der wäßrigen Lösungen der in transformierten Kartoffelpflanzen synthetisierten Stärke wurde aus Knollen von Pflanzen, die mit dem Plasmid p35S-anti-RL bzw. mit dem Plasmid pB33-anti-RL transformiert worden waren, Stärke nach Standardverfahren isoliert. Es wurden jeweils 30 g Stärke in 450 ml H₂O aufgenommen und für die Analyse in einem Viskograph E (Brabender OHG Duisburg (Deutschland)) verwendet. Der Betrieb des Gerätes erfolgte nach den Angaben des Herstellers. Zur Bestimmung der Viskosität der wäßrigen Lösung der Stärke wurde die Stärkesuspension zunächst von 50°C auf 96°C erhitzt mit einer Geschwindigkeit von 3°C pro min. Anschließend wurde die Temperatur für 30 min bei 96°C gehalten. Danach wurde die Lösung von 96°C auf 50°C abgekühlt mit einer Geschwindigkeit von 3°C pro mine Während der gesamten Dauer wurde die Viskosität bestimmt. Repräsentative Ergebnisse derartiger Messungen sind in Form von Kurven, in denen die Viskosität in Abhängigkeit der Zeit dargestellt ist, in Fig. 3, Fig. 4 und Fig. 5 wiedergegeben. Fig. 3 zeigt eine typische Brabenderkurve für Stärke, die aus Wildtyp-Pflanzen der Kartoffelvarietät Désirée isoliert wurde. Fig. 4 und 5 zeigen eine typische Brabenderkurve für Stärke, die aus Kartoffelpflanzen isoliert wurde, die mit dem Plasmid p35S-anti-RL bzw. pB33-anti-RL transformiert worden waren. Aus den Kurven lassen sich verschiedene charakteristische Werte ableiten.

Für Wildtyppflanzen ergeben sich dabei folgende charakteristische Werte:

**Tabelle 1**

| Wert | Zeit | Drehmoment | Temperatur |
|---|---|---|---|
| | [min : sec] | [BE] | [°C] |
| A | 6 : 30 | 60,5 ± 17,7 | 69,9 ± 0,57 |
| B | 11 : 30 | 1838,0 ± 161,2 | 86,0 ± 2,1 |
| C | 15 15 | 1412,0 ± 18,4 | 96,0 |
| D | 45 : 15 | 526,0 ± 17,0 | 96,0 |
| E | 60 : 30 | 812,0 ± 8,5 | 50,0 |
| F | 70 : 45 | 853,0 ± 5,7 | 50,0 |

Die Werte geben Mittelwerte aus zwei verschiedenen Messungen wieder.

In der Tabelle 1 und den folgenden Tabellen 2 und 3 bedeuten:

| | |
|---|---|
| A: | Verkleisterungsbeginn |
| B: | Maximale Viskosität |
| C: | Start der Haltezeit |
| D: | Start der Kühlzeit |
| E: | Ende der Kühlzeit |
| F: | Ende der End-Haltezeit. |

Für Pflanzen, die mit dem Plasmid p35S-anti-RL transformiert worden waren (Linie P2), ergeben sich dabei folgende charakteristische Werte:

**Tabelle 2**

| Wert | Zeit | Drehmoment | Temperatur |
|---|---|---|---|
| | [min : sec] | [BE] | [°C] |
| A | 6 : 00 | 50,0 | 69,0 |
| B | 14 : 00 | 820,0 | 93,0 |
| C | 15 : 15 | 815,0 | 96,0 |
| D | 45 : 15 | 680,0 | 96,0 |
| E | 60 : 30 | 1150,0 | 50,0 |
| F | 70 : 45 | 1200,0 | 50,0 |

Für Pflanzen, die mit dem Plasmid pB33-anti-RL transformiert worden waren (Linie P3), ergeben sich dabei folgende Werte:

**Tabelle 3**

| Wert | Zeit | Drehmoment | Temperatur |
|---|---|---|---|
| | [min : sec] | [BE] | [°C] |
| A | 7:0 | 31,0 | 71,0 |
| B | 12:45 | 671,0 | 88,3 |
| C | 15:15 | 662,0 | 96,0 |
| D | 45:15 | 607,0 | 96,0 |
| E | 60:30 | 1063,0 | 50,0 |
| F | 70:45 | 1021,0 | 50,0 |

Aus den Figuren 3, 4 und 5 geht deutlich hervor, daß die Stärke aus transformierten Pflanzen sich von der aus Wildtyp-Pflanzen insbesondere dadurch unterscheidet, daß beim Aufkochen nur eine sehr geringe Viskositätszunahme erfolgt. So liegt die maximale Viskosität bei der modifizierten Stärke aus transformierten Pflanzen beim Aufkochen um mehr als 50% unter dem Wert der Wildtyp-Stärke.
Andererseits steigt die Viskosität der aus transformierten Pflanzen isolierten Stärke nach dem Abkühlen stärker an als bei Wildtyp-Stärke.

### b) Bestimmung des Phosphatgehaltes der Stärke

Der Phosphatgehalt der Stärke wurde bestimmt, indem die Menge an Phosphat, das an der C-6-Position von Glucoseresten gebunden war, gemessen wurde. Hierzu wurde Stärke zunächst durch Säurehydrolyse gespalten und anschließend der Gehalt an Glucose-6-Phosphat mittels eines Enzymtests bestimmt, wie im folgenden beschrieben.

100 mg Stärke wurden in 500 µl 0,7 N HCl 4 h bei 100 °C inkubiert. Nach der Säurehydrolyse wurden 10 µl des Ansatzes in 600 µl Imidazolpuffer (100 mM Imidazol, 5 mM MgCl₂, pH 6,9, 0,4 mM NAD⁺) gegeben. Die Bestimmung der Menge an Glucose-6-Phosphat in dem Ansatz erfolgte durch Umsetzung mit dem Enzym Glucose-6-Phosphat-Dehydrogenase. Dazu wurde dem Ansatz 1 U Glucose-6-Phosphat-Dehydrogenase (aus Leuconostoc mesenteroides (Boehringer Mannheim)) zugesetzt und die Menge an gebildetem NADH durch Messung der Absorption bei 340 nm bestimmt.

Der Gehalt an Glucose-6-Phosphat/Milligramm Stärke ist in der folgenden Tabelle für nicht-transformierte Kartoffelpflanzen der Varietät Désirée sowie für zwei Linien (P1(35S-anti-RL; P2(35S-anti-RL)) transgener Kartoffelpflanzen, die mit dem Plasmid p35S-anti-RL transformiert worden waren, angegeben.

**Tabelle 4**

| Pflanzen | nmol Glucose-6-Phosphat/mg Stärke | %. |
|---|---|---|
| Wildtyp | 12,89 ± 1,34 | 100 |
| P1(35S-anti-RL) | 2,25 ± 0,41 | 17,4 |
| P2(35S-anti-RL) | 1,25 ± 0 | 9,7 |

Die folgende Tabelle zeigt den Glucose-6-Phosphat-Gehalt pro Milligramm Stärke bei Kartoffelpflanzen, die mit dem Plasmid pB33-anti-RL transformiert worden waren, im Vergleich zu Stärke aus nicht-transformierten Pflanzen (S. tuberosum c.v. Désirée).

**Tabelle 5**

| Pflanzen | nmol Glucose-6-Phosphat/mg Stärke | % |
|---|---|---|
| Wildtyp | 9,80 ± 0,68 | 100 |
| 7 | 4,50 ± 0,73 | 45,9 |
| 37 | 2,64 ± 0,99 | 26,9 |
| 45 | 1,14 ± 0,44 | 11,6 |
| 31 | 1,25 ± 0,49 | 12,8 |

Die Pflanzen 7, 37, 45 und 31 stellen unabhängige Transformanten dar, die mit dem Plasmid pB33-anti-RL transformiert worden waren. Die Pflanze 37 repräsentiert die Linie P3, für die in Figur 5 eine Brabenderkurve dargestellt ist.

Die Werte zeigen, daß der Phosphatgehalt der modifizierten Stärke aus transgenen Kartoffelpflanzen um mindestens ca. 50% im Vergleich zu Stärke aus Wildtyp-Pflanzen verringert ist.

### c) Bestimmung des Glucose-, Fructose- und Saccharosegehalts von Knollen nach Lagerung bei 4 °C

Knollen von Pflanzen verschiedener transgener Linien, die mit dem antisense-Konstrukt p35S-anti-RL transformiert worden waren, und von Wildtyp-Pflanzen wurden für 2 Monate bei 4 °C bzw. bei 20 °C im Dunkeln gelagert. Anschließend wurden wie oben beschrieben die Mengen an Glucose, Fructose und Saccharose bestimmt. Dabei ergaben sich für zwei transgene Linien folgende repräsentative Werte:

**Tabelle 6**

| | Glucose | | Fructose | | Saccharose | |
|---|---|---|---|---|---|---|
| | 20°C | 4°C | 20°C | 4°C | 20°C. | 4°C |
| Wildtyp cv Désirée | 0,84 | 55,4 | 0,62 | 52,8 | 8,5 | 13,1 |
| | | | | | | |
| Transgene Linie 15 | 1,12 | 6,7 | 0,75 | 7,8 | 7,5 | 1.0,1 |
| | | | | | | |
| Transgene Linie 11 | 1,00 | 6,4 | 0,75 | 7,5 | 6,9 | 6,9 |

Die Werte in der Tabelle sind in µmol Hexose bzw. Saccharose/ g Frischgewicht angegeben.

Aus den Werten in Tabelle 6 wird deutlich, daß bei den transgenen Pflanzen bei einer Lagerung bei 4 °C eine wesentlich geringere Akkumulation reduzierender Zucker in den Knollen stattfindet als bei Wildtyp-Pflanzen.

Insgesamt ähnelt die aus transgenen Kartoffelpflanzen isolierte modifizierte Stärke der Stärke aus Mais-Wildtyp-Pflanzen. Im Vergleich zu dieser besitzt sie den Vorteil, daß sie geschmacksneutral ist und so für verschiedene Verwendungsmöglichkeiten im Nahrungsmittelbereich besser geeignet ist.

### Beispiel 9

### Expression der cDNA-Insertion des Plasmids pRL2 in E. coli

### (a) Transformation von Bakterienzellen

Zur Expression der cDNA-Insertion des Plasmids pRL2 wurden Zellen des E. coli-Stammes DH5α zunächst mit dem Plasmid pACAC transformiert. Dieses Plasmid enthält ein DNA-Fragment, das die ADP-Glucose-Pyrophosphorylase (AGPase) aus E. coli codiert, unter der Kontrolle des lac Z-Promotors. Das Fragment war als ca. 1,7 kb großes DraI/HaeII-Fragment aus dem Vektor pEcA-15 (siehe B. Müller-Röber (1992), Dissertation, FU Berlin) isoliert worden und nach Glättung der Enden in einen mit HindIII linearisierten pACAC184-Vektor cloniert worden. Die Expression der AGPase soll eine Steigerung der Glycogensynthese in transformierten E. coli Zellen bewirken. Die derart transformierten Zellen werden im folgenden als E. coli-Kl-Zellen bezeichnet.
Zur Bestimmung der Enzymaktivität des durch die cDNA des Plasmids pRL2 codierten Proteins, wurden E. coli-K1-Zellen mit dem Plasmid pRL2 transformiert. Die transformierten E. coli-Zellen, die sowohl das Plasmid pACAC als auch das Plasmid pRL2 enthalten, werden im folgenden als E. coli-K2-Zellen bezeichnet.
Der Transfer der Plasmid-DNA in die Bakterienzellen erfolgte jeweils nach der Methode von Hanahan (J. Mol. Biol. 166 (1983), 557-580). Die transformierten E. coli Zellen wurden auf Agarkulturschalen mit folgender Zusammensetzung ausgestrichen:

**YT-Medium mit**

| | |
|---|---|
| 1,5% | Bacto-Agar |
| 50 | mM Natriumphosphat-Puffer, pH 7,2 |
| 1% | Glucose |
| 10 µg/ml | Chloramphenicol bei E. coli-K1-Zellen bzw. |
| 10 µg/ml | Chloramphenicol und |
| 10 µg/ml | Ampicillin bei E. coli-K2-Zellen. |

Escherichia coli Zellen des Stammes DH5_, die mit dem Plasmid pRL2 + pACAC (E. coli-K2-Zellen) sowie als Kontrolle nur mit dem Plasmid pACAC (E. coli-K1-Zellen) transformiert worden sind, wurden auf Agarplatten angezogen. Das gebildete Glycogen der verschiedenen Kulturen wurde bezüglich des Phosphorylierungsgrades (an C-6-Position des Glucosemoleküls) hin untersucht, wie im folgenden beschrieben wird.

### (b) Isolierung von bakteriellem Glycogen

Zur Isolierung von bakteriellem Glycogen wurde der nach der Transformation gewachsene Bakterienrasen von jeweils 6 Agarplatten (ø 135 mm) mit 5 ml YT-Medium/Platte abgeschwemmt. Die Bakteriensuspension wurde bei 4500 xg für 5 Minuten zentrifugiert. Der Bakterienniederschlag wurde in 10 ml YT-Medium resuspendiert. Der Aufschluß der Bakterien erfolgte durch Zugabe von 2 Volumen Aufschlußmedium (0,2 N NaOH; 1% SDS) und Inkubation für 5 Minuten bei RT. Durch Zugabe von 3 Volumen EtOH abs., 30 minütiger Inkubation bei 4°C und anschließender Zentrifugation von 15 Minuten bei 8000 gx wurde das Glycogen sedimentiert. Anschließend wurde der Niederschlag mit 100 ml 70%igem EtOH gewaschen und erneut durch einen Zentrifugationsschritt (10 Minuten bei 8000 xg) sedimentiert. Der Waschvorgang wurde 4 mal wiederholt.

### (c) Bestimmung des Gesamtglycogengehaltes

Das isolierte und sedimentierte Glycogen wurde zunächst durch saure Hydrolyse (Lösen des Niederschlags in 2 ml 0,7 N HCl; Inkubation für 4 Stunden bei 100°C) in die einzelnen Glucosemoleküle aufgespalten. Der Glucosegehalt der Lösung wurde mittels gekoppelter enzymatischer Reaktion eines Stärke-Tests nach Angaben des Herstellers (Boehringer Mannheim) an einem Photometer (Firma Kontron) bei einer Wellenlänge von 340 nm bestimmt. Der Reaktionspuffer enthält:

| | |
|---|---|
| 100 | mM MOPS, pH 7,5 |
| 10 | mM MgCl₂ |
| 2 | mM EDTA |
| 0,25 | mM NADP |
| 1 | mM ATP |
| 1 | U/ml Glucose-6-Phosphat-Dehydrogenase |
| 2 | U/ml Hexokinase |

Die Messung erfolgte bei 25°C mit 10 µl Glucoselösung.

### (d) Bestimmung des Glucose-6-Phosphat Gehaltes

Zur Bestimmung des Gehaltes der an C-6-Position phosphorylierten Glucosemoleküle wurden gleiche Stoffmengen an Glucose, jeweils der verschiedenen Bakterienkulturen, eingesetzt. Durch Zugabe von gleichen Volumina an 0,7 N KOH zu dem mittels saurer Hydrolyse (siehe oben) in seine Glucosemoleküle aufgespaltenen Glycogens, wurde die Lösung neutralisiert.
Der Reaktionspuffer enthält:

| | |
|---|---|
| 100 | mM MOPS, pH 7,5 |
| 10 | mM MgCl₂ |
| 2 | mM EDTA |
| 0,25 | mM NADP |
| 2 | U/ml Glucose-6-Phosphat-Dehydrogenase |

Die Messung erfolgte bei 25°C mit 100-150 µl Glucoselösung.

### (e) Nachweis einer bakterielles Glycogen phosphorylierenden Enzymaktivität

Die Ergebnisse der Bestimmung des Phosphatgehaltes des in den Bakterienzellen synthetisierten Glycogens zeigen, daß das Glycogen der E. coli Zellen, die mit den Plasmiden pACAC + pRL2 transformiert worden waren, eine bis zu 290 ± 25% erhöhte Phosphorylierung an C-6-Position der Glucose aufweist, verglichen mit dem Kontrollansatz (E. coli Zellen transformiert mit dem Plasmid pACYC) (siehe folgende Tabelle)

| | |
|---|---|
| E. coli-Zellen | Glucose-6-Phosphat:Glucose im Glycogen |
| E. coli-K1 | 1:(4600 ± 1150) |
| E. coli-K2 | 1:(1570 ± 390) |

Die hier dargestellten Phosphorylierungsgrade sind der Mittelwert aus mindestens 6 Messungen ausgehend von 6 unabhängigen Transformationen und Glycogenisolierungen.

### Beispiel 10

### Einführung des Plasmids p35S-anti-RL in Kombination mit dem Plasmid p35SH-anti-BE in das Genom von Kartoffelpflanzen

Das Plasmid p35S-anti-RL wurde konstruiert wie im Beispiel 6 beschrieben. Das Plasmid p35SH-anti-BE wurde konstruiert wie in der Anmeldung WO95/07355, Beispiel 3, beschrieben. Beide Plasmide wurden mit Hilfe der Agrobakterium vermittelter Transformation wie oben beschrieben sequentiell in Kartoffelpflanzen transferiert. Dazu wurde zunächst das Plasmid p35SH-anti-BE in Kartoffelpflanzen transformiert. Es wurden ganze Pflanzen regeneriert und auf eine verringerte Expression des *branching*-Enzymgens selektiert. Anschließend wurde das Plasmid p35S-anti-RL in die schon reduzierte Expression des *branching*-Enzyms aufweisenden transgenen Pflanzen transformiert. Aus den transformierten Zellen wurden wiederum transgene Pflanzen regeneriert, und die transformierten Pflanzen wurden unter Gewächshausbedingungen kultiviert. Die Überprüfung des Erfolges der genetischen Veränderung der Pflanzen in Bezug auf eine stark reduzierte Expression sowohl des *branching*-Enzymgens als auch in Bezug auf eine stark reduzierte Expression des RL-Gens erfolgte durch Analyse der gesamten RNA in einer RNA-Blot-Analyse bezüglich des Verschwindens der zu Verzweigungsenzym-cDNA bzw. RL-cDNA komplementären Transkripte. Hierzu wurde die Gesamt-RNA aus Blätter transformierten Pflanzen nach beschriebenen Methoden isoliert, gelelektrophoretisch aufgetrennt, auf eine Membran transferiert und mit einer radioaktiv markierten Probe hybridisiert, die die unter Seq ID No. 1 dargestellte Sequenz oder einen Teil dieser Sequenz aufweist und anschließend mit einer radioaktiv markierten Probe hybridisiert, die die Sequenz der Verzweigungsenzym-cDNA (vgl. WO92/14827, Beispiel 1) oder einen Teil derselben aufweist. In ca. 5% - 10% der transformierten Pflanzen fehlte in der RNA-Blot-Analyse sowohl die Bande, die das spezifische Transkript der unter Seq. ID No. 1 dargestellten Sequenz darstellt als auch die Bande, die das spezifische Transkript der Verzweigungsenzym-cDNA (vgl. WO92/14827, Beispiel 1) darstellt. Diese Pflanzen, welche als R4-Pflanzen bezeichnet wurden, wurden zur Analyse der Qualität der in den Knollen enthaltenen Stärke eingesetzt.

### Beispiel 11

### Einführung des Plasmids pB33-anti-RL in Kombination mit dem Plasmid pB33-anti-GBSSI in das Genom von Kartoffelpflanzen

Das Plasmid pB33-anti-RL wurde konstruiert wie im Beispiel 7 beschrieben. Das Plasmid pB33-anti-GBSSI wurde wie folgt konstruiert:
Das *DraI*/*DraI* Fragment aus der Promotorregion des Patatin Klasse I Gens B33 von *Solanum tuberosum,* umfassend die Nukleotide -1512 bis +14 (Rocha-Sosa et al., EMBO J 8 (1989), 23-29) wurde in die *Sma*I Schnittstelle des Plasmids pUC19 ligiert. Aus dem entstandenen Plasmid wurde das Promotorfragment als *Eco*RI/*Hind*III Fragment in die *polylinker* Region des Plasmids pBin19 (Bevan, Nucleic Acids Research 12 (1984), 8711-8721) ligiert. Anschließend wurde das 3' EcoRI Fragment 1181 bis 2511 des GBSSI-Gens von *Solanum tuberosum* (Hergersberg, Dissertation (1988) Universität zu Köln) in die *Eco*RI Schnittstelle des entstandenen Plasmids ligiert.

Beide Plasmide wurden mit Hilfe Agrobakterium vermittelter Transformation sequentiell in Kartoffelpflanzen transferiert wie unter Beispiel 10 beschrieben. Aus den transformierten Zellen wurden ganze Pflanzen regeneriert, und die transformierten Pflanzen wurden unter Gewächshausbedingungen kultiviert. Die Überprüfung des Erfolges der genetischen Veränderungen der Pflanzen erfolgte durch Analyse der Gesamt-RNA in einer RNA-Blot-Analyse bezüglich des Verschwindens der zu den beiden cDNAs komplementären Transkripte. Hierzu wurde die Gesamt-RNA aus Knollen transformierter Pflanzen nach Standardmethoden isoliert, gelelektrophoretisch auf einem Agarosegel aufgetrennt, auf eine Membran transferiert und mit einer radioaktiv markierten Probe hybridisiert, die die unter Seq ID No. 1 dargestellte Sequenz oder einen Teil der Sequenz aufweist. Danach wurde die gleiche Membran mit einer radioaktiv markierten Probe hybridisiert, die die Sequenz des GBSSI-Gens oder einen Teil dieser Sequenz aufweist (Hergersberg, Dissertation (1988) Universität zu Köln). In ca. 5% bis 10% der transformierten Pflanzen fehlte in der RNA-Blot-Analyse die Bande, die Transkripte darstellt, die mit der erfindungsgemäßen cDNA bzw. mit der GBSSI-cDNA hybridisierten. Aus den Knollen dieser Pflanzen, welche als R3-Pflanzen bezeichnet wurden, wurde Stärke isoliert und analysiert.

### Beispiel 12

### Stärkeanalyse der R4-Pflanzen

Die gemäß Beispiel 10 transformierten Kartoffelpflanzen wurden hinsichtlich der Eigenschaften der synthetisiertenStärke untersucht. Die Analysen wurden an verschiedenen Linien von Kartoffelpflanzen durchgeführt, die mit den beiden Plasmiden p35S-anti-RL und p35SH-anti-BE transformiert worden waren und die in der RNA-Blot-Analyse die Banden nicht mehr oder stark reduziert aufwiesen, die Transkripte darstellen, die mit den erfindungsgemäßen DNA-Sequenzen bzw. mit der Sequenz der Verzweigungs-cDNA hybridisieren.

### a) Bestimmung der Viskosität wäßriger Lösungen der Stärke

Zur Bestimmung der Viskosität der wäßrigen Lösungen der in transformierten Kartoffelpflanzen synthetisierten Stärke wurde aus Knollen von Pflanzen, die mit dem Plasmid p35S-anti-RL und mit dem Plasmid p35SH-anti-BE transformiert worden waren, Stärke nach Standardverfahren isoliert. Es wurden jeweils 2 g Stärke in 25 ml H₂O aufgenommen und für die Analyse in einem Rapid Visco Analyser (Newport Scientific Pty Ltd, Investment Support Group, Warriewood NSW 2102, Australien) verwendet. Der Betrieb des Gerätes erfolgte nach den Angaben des Herstellers. Zur Bestimmung der Viskosität der wäßrigen Lösung der Stärke wurde die Stärkesuspension zunächst von 50°C auf 95°C erhitzt mit einer Geschwindigkeit von 12°C pro mine Anschließend wurde die Temperatur für 2,5 min bei 95°C gehalten. Danach wurde die Lösung von 95°C auf 50°C abgekühlt mit einer Geschwindigkeit von 12°C pro mine Während der gesamten Dauer wurde die Viskosität bestimmt. Repräsentative Ergebnisse derartiger Messungen sind in Form von Kurven, in denen die Viskosität in Abhängigkeit von der Zeit dargestellt ist, wiedergegeben. Fig. 6 zeigt unter 1 eine typische RVA-Kurve für Stärke, die aus Wildtyp-Pflanzen der Kartoffelvarietät Désirée isoliert wurde. Linie 2 bzw. 3 zeigen typische RVA-Kurven für Stärken, die aus Kartoffelpflanzen isoliert wurde, die mit dem Plasmid p35SH-anti-BE bzw. p35S-anti-RL transformiert worden waren. Linie 4 zeigt eine typische RVA-Kurve für Stärke, die aus den Knollen von Pflanzen isoliert worden ist, die mit dem Plasmid p35SH-anti-BE in Kombination mit dem Plasmid p35S-anti-RL transformiert worden ist. Linie 4 zeichnet sich durch das Fehlen jedweder Viskositätszunahme in Abhängigkeit von der Temperatur aus.

### b) Bestimmung des Amylose/Amylopektinverhältnisses

Aus den Knollen von transformierten Kartoffelpflanzen isolierte Stärke wurde auf das Amylose zu Amylopektinverhältnis untersucht. Dabei ergab sich für die Pflanzenlinie R4-1 (dargestellt in Linie 4 der Fig. 6) ein Amylosegehalt von über 70%. Für die Pflanzenlinie R4-3 ergab sich ein Amylosewert von 27%, während der Amylosegehalt in Wildtypstärke aus der Sorte Désirée zwischen 19 und 22% liegt.

### Beispiel 13

### Stärkeanalyse der R3-Pflanzen

Die gemäß Beispiel 11 transformierten Kartoffelpflanzen wurden hinsichtlich der Eigenschaften der synthetisierten Stärke untersucht. Die Analysen wurden an verschiedenen Linien von Kartoffelpflanzen durchgeführt, die mit den beiden Plasmiden pB33-anti-RL und pB33-anti-GBSSI transformiert worden waren und die in der RNA-Blot-Analyse die Banden nicht mehr oder stark reduziert aufwiesen, die Transkripte darstellen, die mit den erfindungsgemäßen DNA-Sequenzen bzw. mit der Sequenz der GBSSI-cDNA hybridisieren.

### a) Bestimmung der Viskosität wäßriger Lösungen der Stärke

Zur Bestimmung der Viskosität der wäßrigen Lösungen der in transformierten Kartoffelpflanzen synthetisierten Stärke wurde aus Knollen von Pflanzen, die mit dem Plasmid pB33-anti-RL in Kombination mit dem Plasmid pB33-anti-GBSSI transformiert worden waren, Stärke nach Standardverfahren isoliert. Die Bestimmung der Viskosität mittels eines Rapid Visco Analysers erfolgte nach der in Beispiel 12, Teil a, beschriebenen Methode. Die Ergebnisse sind in Figur 7 dargestellt. Fig. 7 zeigt in Linie 1 eine typische RVA-Kurve für Stärke, die aus Wildtyp-Pflanzen der Kartoffelvarietät Désirée isoliert wurde. Linie 2 bzw. 3 zeigen typische RVA-Kurven für Stärken, die aus Kartoffelpflanzen isoliert wurde, die mit dem Plasmid pB33-anti-GBSSI bzw. p35S-anti-RL transformiert worden waren. Linie 4 zeigt eine typische RVA-Kurve für Stärke, die aus den Kartoffelpflanzen isoliert wurde, die mit dem Plasmid pB33-anti-GBSSI in Kombination mit dem Plasmid pB33-anti-RL transformiert worden waren. Diese Kurve zeichnet sich durch das Fehlen des viskositätsmaximums sowie dem Fehlen des Anstiegs der Viskosität bei 50°C aus. Des weiteren zeichnet sich diese Stärke dadurch aus, daß der nach RVA-Behandlung erhaltene Kleister so gut wie keine Retrogradation nach mehrtägiger Inkubation bei Raumtemperatur aufweist.

### b) Bestimmung des Amylose/Amylopektinverhältnisses

Aus den Knollen von transformierten Kartoffelpflanzen isolierte Stärke wurde auf das Amylose zu Amylopektinverhältnis untersucht. Dabei ergab sich für die Pflanzenlinie R3-5 (dargestellt in Linie 4 der Fig. 7) ein Amylosegehalt von unter 4%, für die Pflanzenlinie R3-6 ein Amylosegehalt von unter 3%. Der Amylosegehalt in Wildtypstärke aus der Sorte Désirée liegt zwischen 19 und 22% liegt.

### c) Bestimmung des Phosphatgehaltes der Stärke

Der Phosphatgehalt der Stärke wurde bestimmt, indem die. Menge an Phosphat, das an der C-6-Position von Glucoseresten gebunden war, gemessen wurde. Hierzu wurde Stärke zunächst durch Säurehydrolyse gespalten und anschließend der Gehalt an Glucose-6-Phosphat mittels eines Enzymtests bestimmt, wie im folgenden beschrieben.

100 mg Stärke wurden in 500 µl 0,7 N HCl 4 h bei 100 °C inkubiert. Nach der Säurehydrolyse wurden 10 µl des Ansatzes in 600 µl Imidazolpuffer (100 mM Imidazol, 5 mM MgCl₂, pH 6,9, 0,4 mM NAD⁺) gegeben. Die Bestimmung der Menge an Glucose-6-Phosphat in dem Ansatz erfolgte durch Umsetzung mit dem Enzym Glucose-6-Phosphat-Dehydrogenase. Dazu wurde dem Ansatz 1 U Glucose-6-Phosphat-Dehydrogenase (aus Leuconostoc mesenteroides (Boehringer Mannheim)) zugesetzt und die Menge an gebildetem NADH durch Messung der Absorption bei 340 nm bestimmt.

Der Gehalt an Glucose-6-Phosphat/Milligramm Stärke ist in der folgenden Tabelle für nicht-transformierte Kartoffelpflanzen der Varietät Désirée sowie für die Linien R3-5 und R3-6 transgener Kartoffelpflanzen, die mit dem Plasmid pB33-anti-RL in Kombination mit dem Plasmid pB33-anti-GBSSI transformiert worden waren, angegeben. Zum Vergleich ist der Wert für die Stärke aus der sog. waxy-Kartoffel (US2-10) mit angegeben, die mit dem Plasmid pB33-anti-GBSSI transformiert worden war.

**Tabelle 7**

| Pflanzen | nmol Glucose-6-Phosphat/mg Stärke | % |
|---|---|---|
| Wildtyp | 9,80 ± 0,68 | 100 |
| R3-5 | 1,32 ± 0,10 | 13 |
| R3-6 | 1,37 ± 0,15 | 14 |
| US2-10 | 10,82 ± 0,42 | 110 |

## Patentansprüche

1. Verfahren zur Herstellung einer Stärke, **dadurch gekennzeichnet, dass** Stärke aus transgenen Pflanzenzellen oder aus transgenen Pflanzen enthaltend solche transgenen Pflanzenzellen isoliert wird, wobei die transgenen Pflanzenzellen **dadurch gekennzeichnet sind, dass** sie transformiert sind mit einem Nucleinsäuremolekül, das ein Protein codiert, das in pflanzlichen Zellen sowohl an Stärkekörner gebunden vorliegt, als auch in löslicher Form, und dessen Verringerung in pflanzlichen Zellen zur Synthese einer Stärke mit reduziertem Phosphatgehalt führt oder das bei Expression in E. coli zu einer Phosphorylierung des in den Zellen synthetisierten Glycogens führt, wobei das Nucleinsäuremolekül ausgewählt ist aus der Gruppe bestehend aus:
(a) Nucleinsäuremolekülen, die für ein Protein mit der unter SEQ ID NO: 2 angegebenen Aminosäuresequenz codieren;
(b) Nucleinsäuremolekülen, die die codierende Region der unter SEQ ID No: 1 angegebenen Nucleotidsequenz umfassen;
(c) Nucleinsäuremolekülen, die mit den unter (a) oder (b) genannten Nucleinsäuremolekülen hybridisieren und die ein Protein codieren, das in pflanzlichen Zellen sowohl an Stärkekörner gebunden vorliegt, als auch in löslicher Form, und dessen Verringerung in pflanzlichen Zellen zur Synthese einer Stärke mit reduziertem Phosphatgehalt führt oder das bei Expression in E. coli zu einer Phosphorylierung des in den Zellen synthetisierten Glycogens führt;
(d) Nucleinsäuremolekülen, nach (c), wobei die Hybridisierung unter stringenten Bedingungen erfolgt;
(e) Nucleinsäuremoleküle, deren Sequenz zu mindestens 60% identisch ist mit der codierenden Region der in SEQ ID NO:1 angegebenen Nucleotidsequenz und die ein Protein codieren, das in pflanzlichen Zellen sowohl an Stärkekörner gebunden vorliegt, als auch in löslicher Form, und dessen Verringerung in pflanzlichen Zellen zur Synthese einer Stärke mit reduziertem Phosphatgehalt führt oder das bei Expression in E. coli zu einer Phosphorylierung des in den Zellen synthetisierten Glycogens führt; und
(f) Fragmenten der unter (a) bis (e) genannten Nucleinsäuremoleküle, wobei die Fragmente eine ausreichende Länge besitzen, um für ein Protein zu codieren, das in pflanzlichen Zellen sowohl an Stärkekörner gebunden vorliegt, als auch in löslicher Form und dessen Verringerung in pflanzlichen Zellen zur Synthese einer Stärke mit reduziertem Phosphatgehalt führt oder das bei Expression in E. coli zu einer Phosphorylierung des in den Zellen synthetisierten Glycogens führt;
wobei dieses Nucleinsäuremolekül mit regulatorischen DNA-Elementen verknüpft ist, die die Transkription und Translation in pflanzlichen Zellen gewährleisten.

2. Verfahren nach Anspruch 1, wobei die transgenen Pflanzenzellen weiterhin **dadurch gekennzeichnet sind, dass** sie Mais-, Reis- oder Weizen-Pflanzenzellen sind,

3. Verfahren zur Herstellung einer Stärke, **dadurch gekennzeichnet, dass** Stärke aus transgenen Pflanzenzellen oder aus transgenen Pflanzen enthaltend solche transgenen Pflanzenzellen isoliert wird, wobei die transgenen Pflanzenzellen **dadurch gekennzeichnet sind, dass**
(A) in ihnen die Expression eines Proteins inhibiert ist, das in pflanzlichen Zellen sowohl an Stärkekörner gebunden vorliegt, als auch in löslicher Form und dessen Verringerung in pflanzlichen Zellen zur Synthese einer Stärke mit reduziertem Phosphatgehalt führt oder das bei Expression in E. coli zu einer Phosphorylierung des in den Zellen synthetisierten Glycogens führt, und das von einem Nucleinsäuremolekül codiert wird ausgewählt aus der Gruppe bestehend aus:
(a) Nucleinsäuremolekülen, die für ein Protein mit der unter SEQ ID No: 2 angegebenen Aminosäuresequenz codieren;
(b) Nucleinsäuremolekülen, die die codierende Region der unter SEQ ID No: 1 angegebenen Nucleotidsequenz umfassen;
(c) Nucleinsäuremolekülen, die mit den unter (a) oder (b) genannten Nucleinsäuremolekülen hybridisieren und die ein Protein codieren, das in pflanzlichen Zellen sowohl an Stärkekörner gebunden vorliegt, als auch in löslicher Form und dessen Verringerung in pflanzlichen Zellen zur Synthese einer Stärke mit reduziertem Phosphatgehalt führt oder das bei Expression in E. coli zu einer Phosphorylierung des in den Zellen synthetisierten Glycogens führt;
(d) Nucleinsäuremolekülen nach (c), wobei die Hybridisierung unter stringenten Bedingungen erfolgt; und
(e) Nucleinsäuremoleküle, deren Sequenz zu mindestens 60% identisch ist mit der codierenden Region der in SEQ ID NO:1 angegebenen Nucleotidsequenz und die ein Protein codieren, das in pflanzlichen Zellen sowohl an Stärkekörner gebunden vorliegt, als auch in löslicher Form und dessen Verringerung in pflanzlichen Zellen zur Synthese einer Stärke mit reduziertem Phosphatgehalt führt oder das bei Expression in E. coli zu einer Phosphorylierung des in den Zellen synthetisierten Glycogens führt;
und wobei die transgenen Pflanzenzellen **dadurch gekennzeichnet sind, dass** sie
(B)
(i) ein DNA-Molekül enthalten, das exprimiert wird und eine antisense-RNA codiert, die die Expression eines Nucleinsäuremoleküls nach (a) bis (e) in der Zelle inhibiert; oder
(ii) ein DNA-Molekül enthalten, das exprimiert wird und ein Ribozym codiert, das spezifisch Transkripte eines Nucleinsäuremoleküls nach (a) bis (e) spaltet; oder
(iii) ein DNA-Molekül enthalten, das exprimiert wird und eine RNA codiert, die bei Expression in einer pflanzlichen Zelle aufgrund eines Cosuppressions-Effektes zur Verringerung der Expression eines Nucleinsäuremoleküls nach (a) bis (e) führt und die einen hohen Grad an Homologie zu Transkripten dieses Nucleinsäuremoleküls aufweist.

4. Verfahren nach Anspruch 3, wobei die transgenen Pflanzenzellen weiterhin **dadurch gekennzeichnet sind, dass** sie Kartoffel-Pflanzenzellen sind.

## Claims

1. A method for the production of a starch, **characterized in that** starch is isolated from transgenic plant cells or from transgenic plants containing such transgenic plant cells, wherein the transgenic plant cells are **characterized in that** they are transformed with a nucleic acid molecule encoding a protein which is present in plant cells in starch granule-bound form as well as in soluble form, and the reduction of which in plant cells leads to the synthesis of a starch exhibiting reduced phosphate content, or which leads upon expression in E. *coli* to a phosphorylation of glycogen synthesized within the cells, wherein the nucleic acid molecule is selected from the group consisting of:
(a) nucleic acid molecules encoding a protein with the amino acid sequence indicated in SEQ ID NO:2;
(b) nucleic acid molecules comprising the coding region of the nucleotide sequence indicated in SEQ ID NO:1;
(c) nucleic acid molecules hybridizing to the nucleic acid molecules mentioned under (a) or (b) and encoding a protein which is present in plant cells in starch granule-bound form as well as in soluble form, and the reduction of which in plant cells leads to the synthesis of a starch exhibiting reduced phosphate content, or which leads upon expression in *E. coli* to a phosphorylation of glycogen synthesized within the cells;
(d) nucleic acid molecules of (c), wherein the hybridization takes place under stringent conditions;
(e) nucleic acid molecules the sequence of which is at least 60% identical to the coding region of the nucleotide sequence indicated in SEQ ID NO:1 and which encode a protein which is present in cells in starch granule-bound form as well as in soluble form, and the reduction of which in plant cells leads to the synthesis of a starch exhibiting reduced phosphate content, or which leads upon expression in E. *coli* to a phosphorylation of glycogen synthesized within the cells; and
(f) fragments of the nucleic acid molecules mentioned under (a) to (e), wherein the fragments have a length sufficient for encoding a protein which is present in plant cells in starch granule-bound form as well as in soluble form, and the reduction of which in plant cells leads to the synthesis of a starch exhibiting reduced phosphate content, or which leads upon expression in *E. coli* to the phosphorylation of glycogen synthesized within the cells;
wherein said nucleic acid molecule is linked to regulatory DNA elements ensuring the transcription and translation in plant cells.

2. The method of claim 1, wherein the transgenic plant cells are further **characterized in that** they are maize, rice or wheat plant cells.

3. A method for the production of a starch, **characterized in that** starch is isolated from transgenic plant cells or from transgenic plants containing such transgenic plant cells, wherein the transgenic plant cells are **characterized in that**
(A) in said transgenic plant cells, the expression of a protein is inhibited which is present in plant cells in starch granule-bound form as well as in soluble form, and the reduction of which in plant cells leads to the synthesis of a starch exhibiting reduced phosphate content, or which leads upon expression in *E*. *coli* to the phosphorylation of glycogen synthesized within the cells and which is encoded by a nucleic acid molecule which is selected from the group consisting of:
(a) nucleic acid molecules encoding a protein with the amino acid sequence indicated in SEQ ID NO:2;
(b) nucleic acid molecules comprising the coding region of the nucleotide sequence indicated in SEQ ID NO:1;
(c) nucleic acid molecules hybridizing to the nucleic acid molecules mentioned under (a) or (b) and encoding a protein which is present in plant cells in starch granule-bound form as well as in soluble form, and the reduction of which in plant cells leads to the synthesis of a starch exhibiting reduced phosphate content, or which leads upon expression in *E*. *coli* to a phosphorylation of glycogen synthesized within the cells;
(d) nucleic acid molecules of (c), wherein the hybridization takes place under stringent conditions; and
(e) nucleic acid molecules the sequence of which is at least 60% identical to the coding region of the nucleotide sequence indicated in SEQ ID NO:1 and which encode a protein which is present in plant cells in starch granule-bound form as well as in soluble form, and the reduction of which in plant cells leads to the synthesis of a starch exhibiting reduced phosphate content, or which leads upon expression in *E. coli* to a phosphorylation of glycogen synthesized within the cells;
and wherein the transgenic plant cells are **characterized in that**
(B) (i) they contain a DNA molecule being expressed and encoding an anti-sense RNA which inhibits the expression of a nucleic acid molecule under (a) to (e) in the cell; or
(ii) they contain a DNA molecule being expressed and encoding a ribozyme which specifically cleaves transcripts of a nucleic acid molecule under (a) to (e); or
(iii) they contain a DNA molecule being expressed and encoding an RNA which upon expression in a plant cell leads to the reduction of the expression of a nucleic acid molecule under (a) to (e) due to a cosuppression effect, and which exhibits a high degree of homology to the transcripts of said nucleic acid molecule.

4. The method of claim 3, wherein the transgenic plant cells are further **characterized in that** they are potato plant cells.

## Revendications

1. Procédé de fabrication d'un amidon, **caractérisé en ce que** l'on isole l'amidon à partir de cellules végétales transgéniques ou de plantes transgéniques contenant de telles cellules végétales transgéniques, dans lequel les cellules végétales transgéniques sont **caractérisées en ce qu'**elles sont transformées avec une molécule d'acide nucléique, qui code pour une protéine, qui se présente liée dans des cellules végétales non seulement sur des grains d'amidon mais aussi sous forme soluble, et dont la diminution dans les cellules végétales conduit à la synthèse d'un amidon avec une teneur en phosphates réduite ou qui conduit par l'expression dans E. coli à une phosphorylation du glycogène synthétisé dans les cellules, dans lequel la molécule d'acide nucléique est choisie dans le groupe constitué de :
(a) molécules d'acide nucléique, qui codent pour une protéine avec la séquence d'aminoacides indiquée dans SEQ ID NO : 2 ;
(b) molécules d'acide nucléique, qui comprennent la région codant pour la séquence de nucléotides indiquée dans SEQ ID No : 1 ;
(c) molécules d'acide nucléique, qui s'hybrident avec les molécules d'acide nucléique indiquées en (a) ou (b) et qui codent pour une protéine, qui se présente liée dans les cellules végétales non seulement sur des grains d'amidon, mais aussi sous forme soluble, et dont la diminution dans les cellules végétales conduit à la synthèse d'un amidon avec une teneur en phosphates réduite ou qui conduit par l'expression dans E. coli à une phosphorylation du glycogène synthétisé dans les cellules ;
(d) molécules d'acide nucléique selon (c), dans lesquelles on effectue l'hybridation dans des conditions stringentes ;
(e) molécules d'acide nucléique, dont la séquence est identique au moins à 60 % avec la région codant pour la séquence de nucléotides indiquée dans SEQ ID NO : 1 et qui codent pour une protéine, qui se présente liée dans les cellules végétales non seulement sur des grains d'amidon, mais aussi sous forme soluble, et dont la diminution dans les cellules végétales conduit à la synthèse d'un amidon avec une teneur en phosphates réduite ou qui conduit par l'expression dans E. coli à une phosphorylation du glycogène synthétisé dans les cellules ; et
(f) fragments des molécules indiquées en (a) à (e) , dans lesquels les fragments possèdent une longueur suffisante, pour coder pour une protéine, qui se présente liée dans les cellules végétales non seulement sur des grains d'amidon, mais aussi sous forme soluble, et dont la diminution dans les cellules végétales conduit à la synthèse d'un amidon avec une teneur en phosphates réduite ou qui conduit par l'expression dans E. coli à une phosphorylation du glycogène synthétisé dans les cellules ;
dans lequel cette molécule d'acide nucléique est reliée avec des éléments régulateurs d'ADN, qui garantissent la transcription et la traduction dans des cellules végétales.

2. Procédé selon la revendication 1, dans lequel les cellules végétales transgéniques sont de plus **caractérisées en ce qu'**elles sont des cellules végétales de maïs, de riz ou de froment.

3. Procédé pour la fabrication d'un amidon, **caractérisé en ce que** l'on isole l'amidon à partir de cellules végétales transgéniques ou de plantes transgéniques contenant de telles cellules végétales transgéniques, dans lequel les cellules végétales transgéniques sont **caractérisées en ce que**
(A) dans celles-ci est inhibée l'expression d'une protéine, qui se présente liée dans les cellules végétales non seulement sur des grains d'amidon, mais aussi sous forme soluble, et dont la diminution dans les cellules végétales conduit à la synthèse d'un amidon avec une teneur en phosphates réduite ou qui conduit par l'expression dans E. coli à une phosphorylation du glycogène synthétisé dans les cellules ; et qui est codée par une molécule d'acide nucléique choisie dans le groupe constitué de :
(a) molécules d'acide nucléique, qui codent pour une protéine avec la séquence d'aminoacides indiquée dans SEQ ID NO : 2 ;
(b) molécules d'acide nucléique, qui comprennent la région codant pour la séquence de nucléotides indiquée dans SEQ ID No : 1 ;
(c) molécules d'acide nucléique, qui s'hybrident avec les molécules d'acide nucléique indiquées en (a) ou (b) et qui codent pour une protéine, qui se présente liée dans les cellules végétales non seulement sur des grains d'amidon, mais aussi sous forme soluble, et dont la diminution dans les cellules végétales conduit à la synthèse d'un amidon avec une teneur en phosphates réduite ou qui conduit par l'expression dans E. coli à une phosphorylation du glycogène synthétisé dans les cellules ;
(d) molécules d'acide nucléique selon (c), dans lesquelles on effectue l'hybridation dans des conditions stringentes ;
(e) molécules d'acide nucléique, dont la séquence est identique au moins à 60 % avec la région codant pour la séquence de nucléotides indiquée dans SEQ ID NO : 1 et qui codent pour une protéine, qui se présente liée dans les cellules végétales non seulement sur des grains d'amidon, mais aussi sous forme soluble, et dont la diminution dans les cellules végétales conduit à la synthèse d'un amidon avec une teneur en phosphates réduite ou qui conduit par l'expression dans E. coli à une phosphorylation du glycogène synthétisé dans les cellules ;
et dans lequel les cellules végétales transgéniques sont **caractérisées en ce qu'**elles
(B)
(i) contiennent une molécule d'ADN, qui est exprimée et code pour un ARN antisens, qui inhibe l'expression d'une molécule d'acide nucléique selon (a) à (e) dans la cellule ; ou
(ii) contiennent une molécule d'ADN, qui est exprimée et code pour un ribozyme, qui clive spécifiquement des transcrits d'une molécule d'acide nucléique selon (a) à (e) ; ou
(iii) contiennent une molécule d'ADN, qui est exprimée et code pour un ARN, qui conduit par son expression dans une cellule végétale par suite d'un effet de co-suppression à la diminution de l'expression d'une molécule d'acide nucléique selon (a) à (e) et qui présente un haut degré d'homologie avec les transcrits de cette molécule d'acide nucléique.

4. Procédé selon la revendication 3, dans lequel les cellules végétales transgéniques sont de plus
**caractérisées en ce qu'**elles sont des cellules végétales de pomme de terre.
